# EUROPÄISCHE PATENTSCHRIFT

(11) **EP 3 752 201 B1**
(45) Veröffentlichungstag und Bekanntmachung des Hinweises auf die Patenterteilung: **22.11.2023**
(21) Anmeldenummer: 19706420.7
(22) Anmeldetag: 12.02.2019
(51) Int. Cl.: A61K 47/02, A61K 9/10, A61K 9/14

(54) **SUSPENSION NANOSKALIGER, ORGANISCHER PARTIKEL UND VERFAHREN ZU IHRER HERSTELLUNG**
SUSPENSION OF NANOSCALE, ORGANIC PARTICLES AND PROCESS FOR PRODUCTION THEREOF
SUSPENSION DE PARTICULES ORGANIQUES NANOMÉTRIQUES ET PROCÉDÉ POUR SA PRÉPARATION

(30) Priorität: 16.02.2018 DE 102018103528
(43) Veröffentlichungstag der Anmeldung: 23.12.2020
(73) Patentinhaber: Friedrich-Alexander-Universität Erlangen-Nürnberg, 91054 Erlangen (DE)
(72) Erfinder: DISTASO, Monika, 91080 Uttenreuth (DE); PEUKERT, Wolfgang, 85570 Markt Schwaben (DE); TRZENSCHIOK, Holger, 96364 Unterrodach (DE)
(74) Vertreter: Louis Pöhlau Lohrentz
(86) Internationale Anmeldenummer: PCT/EP2019/053394
(87) Internationale Veröffentlichungsnummer: WO 2019/158509

(56) Entgegenhaltungen:
- MD. SHARIF HASAN ET AL: "Transition Metal Complexes of Naproxen: Synthesis, Characterization, Forced Degradation Studies, and Analytical Method Verification", JOURNAL OF ANALYTICAL METHODS IN CHEMISTRY, Bd. 2016, 1. Januar 2016 (2016-01-01), Seiten 1-10, XP055582699, ISSN: 2090-8865, DOI: 10.1155/2016/3560695
- Alireza Badiei ET AL: "Dynamic Biochemistry, Process Biotechnology and Molecular Biology 2009 Global Science Books Incorporation of Ibuprofen into SBA-15; Drug Loading and Release Properties", , 31. Dezember 2009 (2009-12-31), XP055582911, Gefunden im Internet: URL:http://www.globalsciencebooks.info/Onl ine/GSBOnline/images/0906/DBPBMB_3(SI2)/DB PBMB_3(SI2)48-50o.pdf [gefunden am 2019-04-24]
- HOLGER TRZENSCHIOK ET AL: "A new approach for the stabilization of amorphous drug nanoparticles during continuous antisolvent precipitation", CHEMICAL ENGINEERING JOURNAL, Bd. 361, 1. April 2019 (2019-04-01), Seiten 428-438, XP055582692, AMSTERDAM, NL ISSN: 1385-8947, DOI: 10.1016/j.cej.2018.12.075

## Beschreibung

Die Erfindung betrifft eine Suspension nanoskaliger, organischer Partikel, ein Verfahren zu ihrer Herstellung, die Verwendung eines wasserlöslichen Übergangsmetallsalzes zur Stabilisierung einer Suspension nanoskaliger, organischer Partikel und die Verwendung der Suspension sowie eine trockene Zubereitung umfassend nanoskalige, organische Partikel.

Etwa bis zu 30% neu entwickelter Arzneistoffe weisen eine geringe Löslichkeit in wässrigen Medien auf.

Gerade eine schlechte Löslichkeit in wässrigen Medien stellt für pharmazeutische Wirkstoffe einen limitierenden Faktor in Bezug auf die Bioverfügbarkeit dar. Die Bioverfügbarkeit ist eine pharmakologische Messgröße für den Anteil eines Wirkstoffes, der unverändert im systemischen Kreislauf, beispielsweise im Blutkreislauf, zur Verfügung steht. Die Bioverfügbarkeit gibt an, wie schnell und in welchem Umfang der Wirkstoff nach Applikation aufgenommen wird und beispielsweise am Wirkort zur Verfügung steht.

Im Stand der Technik sind verschiedene Methoden bekannt, mit denen die Löslichkeit von schwer löslichen Substanzen verbessert werden kann, beispielsweise die Verwendung von Sprüh- oder Gefriertrocknung, der Zusatz von Lösungsvermittlern, beispielsweise in Form von Detergenzien oder Lipidformulierungen, oder die Partikelgrößenreduktion.

Eine Reduzierung der Partikelgröße führt beispielsweise zu einer Vergrößerung des Verhältnisses von Oberfläche zu Volumen des Partikels und dadurch zu einer höheren Auflösungsrate des Wirkstoffes. Dadurch kann eine signifikante Erhöhung der Bioverfügbarkeit erreicht werden.

Beispielsweise beschreibt die DE 692 31 345 T2 die Bereitstellung von oberflächenmodifizierten Arzneimittel-Nanopartikel durch Vermahlung eines Arzneimittels zusammen mit einer Tensidlösung in einer Perlmühle, wobei durch die mechanische Vermahlung Partikel mit einer effektiven mittleren Korngröße von weniger als 400 nm erhalten werden, an deren Oberfläche der oberflächenmodifizierende Stoff gebunden wurde.

Bei einer Reduzierung der Partikelgröße erhöht sich jedoch die Neigung der Partikel zur Agglomeration, was letztendlich zu einer Sedimentation der dispergierten Partikel führt.

Bei einer Suspension handelt es sich vorzugsweise um ein heterogenes Stoffgemisch aus wenigstens einer Flüssigkeit und darin fein verteilten Partikeln, vorzugsweise Festkörpern.

Durch Zugabe von beispielsweise Tensiden oder Thixotropiermitteln kann eine Phasentrennung, beispielsweise eine Sedimentation und/oder eine Aufschwimmen von in der Suspension fein verteilten Partikeln, vorzugsweise Festkörpern, verlangsamt werden.

Bei der Herstellung von Suspensionen sind Partikel bevorzugt, die eine Partikelgröße im Nanometer-Bereich aufweisen, da diese Partikel vorzugsweise eine erhöhte Auflöserate (Gibbs-Thompson-Effekt) aufweisen. Trotzdem kann bei der Herstellung herkömmlicher Suspensionen nicht auf die Verwendung von Stabilisatoren oder Dispergiermitteln, wie beispielsweise Tensiden und/ oder Emulgatoren, verzichtet werden.

Stabilisatoren oder Dispergiermittel können an suspendierte Partikel binden und so die gegenseitige Anziehung der Partikel zumindest teilweise unterbinden. Dadurch kann eine Reifung der Partikel verzögert bzw. verhindert werden.

Die im Stand der Technik verwendeten Stabilisatoren oder Dispergiermittel sind meistens organische Moleküle, die an suspendierte Partikel binden und beispielsweise durch sterische Stabilisierung und/oder elektrostatische Stabilisierung eine Reifung der Partikel unterdrücken.

Als Reifung wird vorzugsweise eine Veränderung der Verteilung der Partikelgröße von suspendierten, festen Partikeln in einer Suspension bezeichnet. Dabei wachsen vorzugsweise größere Partikel an, wogegen kleinere Partikel gelöst werden. Vorzugsweise verringert sich dadurch die Zahl der suspendierten Partikel in der Suspension mit fortschreitender Reifung. Weiterhin kann es zu einer Phasentrennung kommen, wenn die Dichtedifferenz zwischen suspendierten Partikeln und dem flüssigem Suspensionsmedium groß genug ist.

Bei einer sterischen Stabilisierung sind Moleküle des Dispergieradditivs an die Oberfläche der suspendierten Partikel gebunden, wobei beispielsweise lange organische Molekülketten der Moleküle in das Dispergiermedium hineinragen. Die suspendierten Partikel weisen eine Hülle aus solvatisierten Polymermolekülen auf, die ein Aneinanderlagern von suspendierten Partikeln sterisch verhindern kann.

Bei der elektrostatischen Stabilisierung trägt das Dispergieradditiv eine elektrische Ladung, die die Oberflächenladung der suspendierten Partikel beeinflusst. Wenn beispielsweise negativ geladene Polymere, beispielsweise Salze von Polyacrylaten, an die Oberfläche der suspendierten Partikel gebunden sind, weisen die Partikel eine negative Ladung auf.

Dadurch kann eine hohe Abstoßung zwischen gleich geladenen Partikeln erzielt werden, welches die Agglomeration der Partikel unterdrückt.

Alternativ kann beispielsweise die Größe der Partikel durch Präzipitation oder Kristallisierung eines in Wasser schwer löslichen Wirkstoffes, der in einem organischen Lösungsmittel gelöst wurde, oder durch Sprühtrocknung reduziert werden, wobei vorzugsweise nanoskalige Partikel hergestellt werden.

Bei den vorgenannten Verfahren tritt jedoch das Problem auf, dass die Verteilung der Partikelgröße nicht oder nur schwer eingestellt werden kann und beispielsweise eine stabile Partikelgröße von weniger als 100 nm oft nicht möglich ist.

Die Kontrolle der Partikelgrößenverteilung ist jedoch eine wichtige Größe, um die physikalischen Eigenschaften der nanoskaligen Partikel einzustellen, beispielsweise hinsichtlich der Freisetzungscharakteristik oder beispielsweise die optischen Eigenschaften, wie Lichtstreuung oder Lichtabsorption, zu steuern.

Hasan Md. S. et al. Journal of Analytical Methods in Chemistry, Bd. 2016, Artikel-ID 3560695, Seiten 1-10, offenbart Übergangsmetallkomplexe von Naproxen: Synthese; Charakterisierung, Studien zum erzwungenen Abbau und analytischer Verfahrensnachweis.

Badiei A. et al., Dynamic Biochemistry, Process Biotechnology and Molecular Biology 3 (Special Issue 2) 2009, 48-50, offenbart die Aufnahme von Ibuprofen in SBA-15, Arzneimittelbeladung und Abgabeeigenschaften.

Aufgabe der vorliegenden Erfindung ist es daher, eine Suspension nanoskaliger, organischer Partikel bereitzustellen, in der der Festkörper vorzugsweise vollständig in Form von einzelnen organischen Partikeln vorliegt, wobei die Suspension vorzugsweise keine organischen Stabilisator- oder Dispergier-Komponenten umfasst.

Eine weitere Aufgabe der vorliegenden Erfindung ist es, ein Verfahren bereitzustellen, das die Herstellung einer, vorzugsweise stabilisierten, Suspension von nanoskaligen, organischen Partikeln ermöglicht.

Die Aufgabe der vorliegenden Erfindung wird gelöst durch Bereitstellung einer Suspension nach Anspruch 1, wobei die Suspension Wasser, nanoskalige, vorzugsweise amorphe, organische Partikel mit einer volumenbezogenen mittleren Partikelgröße von weniger als 350 nm und wenigstens ein wasserlösliches Salz eines Übergangsmetalls umfasst oder im Wesentlichen daraus besteht, wobei zumindest an der Oberfläche der nanoskaligen, vorzugsweise amorphen, organischen Partikel wenigstens teilweise, vorzugsweise vollständig, Übergangsmetall-Kationen und/oder, vorzugsweise positiv geladene, Hydroxid-Komplexe davon angeordnet, vorzugsweise gebunden, sind, und wobei die nanoskaligen, organischen Partikel wenigstens eine organische Verbindung mit wenigstens 6 C-Atomen und wenigstens einem Säuregruppen-haltigen Rest umfassen.

Die Aufgabe der vorliegenden Erfindung wird weiterhin gelöst durch die Bereitstellung eines Verfahrens nach Anspruch 10 zur Herstellung einer Suspension nach einem der Ansprüche 1 bis 9, wobei das Verfahren folgenden Schritt umfasst:
a) In Kontakt bringen, vorzugsweise Mischen, einer Lösung, die wenigstens eine organische Verbindung mit wenigstens 6 C-Atomen und wenigstens einen Säuregruppen-haltigen Rest aufweist, mit einer wässrigen Lösung, die wenigstens ein wasserlösliches Salz eines Übergangsmetalls umfasst oder daraus besteht.

Die Aufgabe der vorliegenden Erfindung wird ebenfalls gelöst durch die Verwendung eines wasserlöslichen Übergangsmetallsalzes nach Anspruch 14 zur Stabilisierung einer Suspension, die Wasser und nanoskalige, organische Partikel mit einer volumenbezogenen mittleren Primärpartikelgröße von weniger als 350 nm umfasst oder daraus besteht, wobei die organischen Partikel wenigstens eine organische Verbindung mit wenigstens 6 C-Atomen und wenigstens einem Säuregruppen-haltigen Rest umfassen oder daraus bestehen, wobei das wasserlösliche Übergangsmetallsalz Übergangsmetallkationen mit einer formalen Oxidationszahl aus einem Bereich von +1 bis +4 umfasst.

Die Aufgabe der vorliegenden Erfindung wird ebenfalls gelöst durch die Bereitstellung einer Verwendung nach Anspruch 15 einer Suspension nach einem der Ansprüche 1 bis 9 als Additiv in Kosmetika, als Pflanzenschutzmittel oder bei der Beschichtung oder Imprägnierung von anorganischen oder organischen Oberflächen und/oder Gegenständen.

Die Aufgabe der vorliegenden Erfindung wird ebenfalls gelöst durch die Bereitstellung einer trockenen Zusammensetzung nach Anspruch 16, wobei die Zusammensetzung nanoskalige, organische Partikel mit einer volumenbezogenen mittleren Partikelgröße von weniger als 350 nm und wenigstens einen Hilfsstoff, der aus der Gruppe, die aus pharmazeutisch akzeptablen, vorzugsweise wasserlöslichen, Matrixbildnern, anorganischen, vorzugsweise wasserlöslichen, Salzen, Bindemitteln, Füllstoffen, Pigmenten und Mischungen davon besteht, ausgewählt wird, umfasst oder daraus besteht, wobei zumindest an der Oberfläche der nanoskaligen, organischen Partikel wenigstens teilweise, vorzugsweise vollständig, Übergangsmetall-Kationen und/oder, vorzugsweise positiv geladene, Hydroxid-Komplexe davon angeordnet, vorzugsweise gebunden, sind, und wobei die nanoskaligen, organischen Partikel wenigstens eine organische Verbindung mit wenigstens 6 C-Atomen und wenigstens 1 Säuregruppen-haltigen Rest umfassen.

Die Aufgabe der vorliegenden Erfindung wird ebenfalls gelöst durch die Bereitstellung von, vorzugsweise amorphen, nanoskaligen, organischen Partikel nach Anspruch 17 mit einer volumenbezogenen mittleren Partikelgröße von weniger als 350 nm, wobei zumindest an der Oberfläche der organischen Partikel wenigstens teilweise, vorzugsweise vollständig, Übergangsmetall-Kationen und/oder, vorzugsweise positiv geladene, Hydroxid-Komplexe davon angeordnet, vorzugsweise gebunden, sind, und wobei die nanoskaligen, organischen Partikel wenigstens eine organische Verbindung mit wenigstens 6 C-Atomen und wenigstens einem Säuregruppen-haltigen Rest und Übergangsmetall-Kationen und/oder, vorzugsweise positiv geladene, Hydroxid-Komplexe davon umfassen oder daraus bestehen.

Bevorzugte Ausführungsformen der Erfindung sind in den Unteransprüchen angegeben.

Die Erfinder haben festgestellt, dass durch Verwendung einer wässrigen Lösung, die wenigstens ein wasserlösliches Salz eines Übergangsmetalls umfasst oder daraus besteht, wässrige Suspensionen von nanoskaligen, vorzugsweise amorphen, organischen Partikeln bereitgestellt werden können, bei der die nanoskaligen, organischen Partikel zumindest teilweise, vorzugsweise vollständig, in Form von einzelnen Partikeln vorliegen.

Die im Folgenden angegebenen pH-Werte werden, falls nichts anderes angegeben, bei Standardbedingungen (Temperatur von 25°C, Druck von 1013 mbar) gemessen.

Eine erfindungsgemäße Suspension umfasst vorzugsweise eine wässrige Phase und mindestens eine in der wässrigen Phase bei einer Temperatur von 25°C, einem Druck von 1013 mbar und einem pH-Wert aus einem Bereich von 0 bis 7, vorzugsweise von 0,5 bis 4, weiter bevorzugt von 1 bis 3, weiter bevorzugt von 1,15 bis 2,5, weiter bevorzugt von 1,25 bis 2,25, schwerlöslichen, vorzugsweise nicht löslichen, partikulären Komponente, welche weiter bevorzugt nicht als Partikelagglomerat vorliegt, oder besteht daraus.

Vorzugsweise umfasst die wässrige Phase einer erfindungsgemäßen Suspension mindestens 50 Gew.-%, vorzugsweise mindestens 55 Gew.-%, mindestens 70 Gew.-%, Wasser, jeweils bezogen auf das Gesamtgewicht der bei Standardbedingungen (Druck: 1013 mbar, Temperatur: 25°C) flüssigen Bestandteile der wässrigen Phase.

Vorzugsweise umfasst die wässrige Phase einer erfindungsgemäßen Suspension wenigstens ein, in Wasser lösliches oder mischbares organisches Lösungsmittel, das vorzugsweise aus der Gruppe, die aus Alkoholen mit 1 bis 4 C-Atomen, Ketone mit 3 bis 4 C-Atomen, Aldehyde mit 1 bis 4 C-Atomen, Alkannitrilen mit 1 bis 4 C-Atomen, Ethern mit 1 bis 4 C-Aomen, Carbonsäuren mit 1 bis 4 C-Atomen, Carbonsäureamiden mit 1 bis 4 C-tomen, Sulfoxiden mit 1 bis 4 C-Atomen und Mischungen davon, weiter bevorzugt aus Methanol, Ethanol, Propan-1-ol, 2-Methylpropan-1-ol, Propan-2-ol, 2-Methylpropan-2-ol, Propan-1,2,3-triol, Aceton, Butan-2-on, Acetaldehyd, Acetonitril, Tetrahydrofuran, 1,4-Dioxan, Dimethylsulfoxid und Mischungen davon besteht, ausgewählt wird.

Weiter bevorzugt umfasst die wässrige Phase einer erfindungsgemäßen Suspension das wenigstens eine, in Wasser lösliche oder mischbare organische Lösungsmittel in einem Anteil von höchstens 80 Gew.-%, vorzugsweise von höchstens 65 Gew.-%, vorzugsweise von höchstens 50 Gew.-%, vorzugsweise höchstens 45 Gew.-%, weiter bevorzugt höchstens 30 Gew.-%, jeweils bezogen auf das Gesamtgewicht der bei Standardbedingungen (Druck: 1013 mbar, Temperatur: 25°C) flüssigen Bestandteile der wässrigen Phase.

Vorzugsweise wird unter dem Begriff "schwerlösliche, vorzugsweise nicht lösliche, partikuläre Komponente" eine partikuläre Komponente verstanden, die organische Partikel umfasst oder daraus besteht, welche bei einer Temperatur von 25°C, einem Druck von 1013 mbar und einem pH-Wert aus einem Bereich von 0 bis 7, vorzugsweise von 0,5 bis 4, weiter bevorzugt von 1 bis 3, weiter bevorzugt von 1,15 bis 2,5, weiter bevorzugt von 1,25 bis 2,25, eine Löslichkeit in Wasser, die vorzugsweise gemäß DIN EN ISO 787-8 ("Allgemeine Prüfverfahren für Pigmente und Füllstoffe - Teil 8: Bestimmung der wasserlöslichen Anteile; Kaltextraktionsverfahren", Ausgabedatum: 2001-09) bestimmt wird, von 15 g/l oder weniger, vorzugsweise von 10 g/l oder weniger, vorzugsweise von 5 g/l oder weniger, weiter bevorzugt von 900 mg/l oder weniger, weiter bevorzugt von 500 mg/l oder weniger, aufweisen.

Die in der wässrigen Phase bei einer Temperatur von 25°C, einen Druck von 1013 mbar und einem pH-Wert aus einem Bereich von 0 bis 7, vorzugsweise von 0,5 bis 4, weiter bevorzugt von 1 bis 3, weiter bevorzugt von 1,15 bis 2,5, weiter bevorzugt von 1,25 bis 2,25, schwerlösliche, vorzugsweise nicht lösliche, partikuläre Komponente umfasst oder besteht vorzugsweise aus nanoskaligen, vorzugsweise amorphen, organischen Partikeln mit einer, vorzugsweise stabilen, volumenbezogenen mittleren Primärpartikelgröße von weniger als 350 nm, vorzugsweise aus einem Bereich von 5 nm bis 330 nm, von 15 nm bis 250 nm, vorzugsweise aus einem Bereich von 25 nm bis 150 nm, wobei zumindest an der Oberfläche der nanoskaligen, organischen Partikel wenigstens teilweise, vorzugsweise vollständig, Übergangsmetall-Kationen, die vorzugsweise eine formale Oxidationszahl aus einem Bereich von +1 bis +6, vorzugsweise von +2 bis +5, weiter bevorzugt +3 bis +4, weiter bevorzugt von +4, aufweisen und weiter bevorzugt aus der Gruppe, die aus Titan, Zirkonium, Hafnium, Cer, Yttrium und Kombinationen davon, weiter bevorzugt Titan, Zirkonium, Hafnium und Kombinationen davon, weiter bevorzugt Zirkonium, Hafnium und Kombinationen davon, besteht, ausgewählt werden, und/oder, vorzugsweise positiv geladene, Hydroxid-Komplexe davon angeordnet, vorzugsweise gebunden, sind.

Unter dem Begriff "formale Oxidationszahl" wird vorzugsweise die Oxidationszahl oder auch Oxidationsstufe eines Atoms, beispielsweise Übergangsmetalls, innerhalb einer chemischen Verbindung oder eines Ions verstanden. Die Oxidationszahl oder auch Oxidationsstufe ist vorzugsweise die Ladung eines Atoms nach genäherter Darstellung seiner Bindungen als lonenbindung (ionische Näherung).

Die Oxidationszahl eines Atoms in einer Verbindung oder einem Ion kann nach dem Fachmann bekannten Methoden ermittelt werden. Beispielsweise kann die Oxidationszahl gemäß der in Karen, P. et al. ("Comprehensive definition of oxidation state (IUPAC Recommendations 2016)", Pure Appl. Chem. 2016, 88(8), Seiten 831 bis 839, DOI: 10.1515/pac-2015-1204) oder der in Karen, P. ("Die Oxidationsstufe, ein Dauerbrenner!", Angew. Chem. 2015, 127(16), Seiten 4798 bis 4809, DOI: 10.1002/ange.201407561) beschriebenen Verfahren, beispielsweise durch ionische Näherung, ermittelt werden.

Unter dem Begriff "Übergangsmetall-Kationen" werden vorzugsweise Kationen, d.h. positiv geladene Ionen, von chemischen Elementen mit den Ordnungszahlen von 21 bis 30, 39 bis 48, 57 bis 80, 89 bis 112 oder Mischungen davon, vorzugsweise Titan, Zirkonium, Hafnium, Cer, Yttrium oder Mischungen davon, weiter bevorzugt Titan, Zirkonium, Hafnium oder Mischungen davon, weiter bevorzugt Zirkonium, Hafnium oder Mischungen davon, verstanden.

Vorzugsweise können vorgenannte Übergangsmetall-Kationen, vorzugsweise Ti⁴⁺, Zr⁴⁺, Hf⁴⁺ oder Mischungen davon, weiter bevorzugt Zr⁴⁺, Hf⁴⁺ oder Mischungen davon, ebenfalls als, vorzugsweise positiv geladener, Hydroxid-Komplex, beispielsweise der Formel [Zr₃(OH)₄]⁸⁺, [Zr₄(OH)₈]⁸⁺ oder Mischungen davon, vorliegen.

Eine erfindungsgemäße Suspension weist nanoskalige, vorzugsweise amorphe, organische Einzel-Partikel auf, die vorzugsweise für wenigstens 24 Stunden, weiter bevorzugt für wenigstens 36 Stunden, weiter bevorzugt für wenigstens 48 Stunden, im Wesentlichen nicht, vorzugsweise nicht, sedimentieren und/oder reifen.

Nicht-erfindungsgemäße nanoskalige, organische Partikel, d.h. nanoskalige, organische Partikel, an deren Oberfläche keine Übergangsmetall-Kationen, die vorzugsweise eine formale Oxidationszahl aus einem Bereich von +1 bis +6, vorzugsweise von +2 bis +5, weiter bevorzugt +3 bis +4, weiter bevorzugt von +4, aufweisen und weiter bevorzugt aus der Gruppe, die aus Titan, Zirkonium, Hafnium, Cer, Yttrium und Kombinationen davon, weiter bevorzugt Titan, Zirkonium, Hafnium und Kombinationen davon, weiter bevorzugt Zirkonium, Hafnium und Kombinationen davon, besteht, ausgewählt werden, und/oder, vorzugsweise positiv geladene, Hydroxid-Komplexe davon, angeordnet, vorzugsweise gebunden, sind, können sich in wässriger Phase in Abwesenheit von Stabilisatoren oder Dispergiermitteln, wie beispielsweise Tensiden und/ oder Emulgatoren, zu größeren Agglomeraten zusammenschließen und/oder durch Reifung größere Partikel bilden.

Vorzugsweise weist eine nicht erfindungsgemäße Suspension, vorzugsweise in Abwesenheit von Stabilisatoren oder Dispergiermitteln, wie beispielsweise Tensiden und/ oder Emulgatoren, eine Stabilität von weniger als 12 Stunden auf, d.h. in einem Zeitraum von weniger als 12 Stunden kommt es mindestens zu einer wenigstens teilweisen Sedimentation der Partikel und/oder wenigstens teilweisen Ablagerung der Partikel an wenigstens einer Wand eines die nicht-erfindungsgemäße Suspension enthaltenen Gefäßes und/oder auf der Oberfläche der Suspension und/oder zu einer wenigstens teilweisen Flockung/Agglomeration der Partikel.

Gemäß DIN ISO/TS 27687 ("Nanotechnologien -Terminologie und Begriffe für Nanoobjekte, Nanopartikel, Nanofaser und Nanoplättchen", Ausgabedatum: 2010-02) ist ein "Agglomerat" eine Ansammlung von schwach gebundenen Partikeln, in der die resultierende Oberfläche ähnlich der Summe der Oberflächen der einzelnen Bestandteile ist. Die ein Agglomerat zusammenhaltenden Kräfte sind schwache Kräfte, zum Beispiel Van-der-Waals-Kräfte, oder einfache physikalische Verhakungen.

Unter dem Begriff "Partikel" wird vorzugsweise ein sehr kleines Stück einer Substanz mit definierten physikalischen Grenzen verstanden, wobei eine physikalische Grenze auch als Grenzfläche beschrieben werden kann.

Vorzugsweise werden unter dem Begriff "Partikel" im Sinne der Erfindung Primärpartikel verstanden, die eine, vorzugsweise stabile, volumenbezogene mittlere Primärpartikelgröße von weniger als 350 nm, vorzugsweise von weniger als 270 nm, vorzugsweise von weniger als 190 nm, vorzugsweise von weniger als 150 nm, vorzugsweise von weniger als 100 nm, vorzugsweise aus einem Bereich zwischen 1 nm und 350 nm, vorzugsweise aus einem Bereich von 5 nm bis 330 nm, vorzugsweise aus einem Bereich von 15 nm bis 250 nm, vorzugsweise aus einem Bereich von 25 nm bis 150 nm, weiter bevorzugt aus einem Bereich von 30 nm bis 100 nm, aufweisen. Vorzugsweise kann sich ein Partikel als Einheit innerhalb der Suspension bewegen.

Unter dem Begriff "Partikelgröße" wird vorzugsweise die Größe der nanoskaligen, organischen Partikel verstanden, wobei vorzugsweise die Partikelgröße anhand des Durchmessers einer äquivalenten Kugel, die das gleiche Volumen wie das tatsächliche Partikel aufweist, definiert wird. Die volumenbezogene Partikelgröße ist vorzugsweise der volumenbezogene Äquivalentdurchmesser der nanoskaligen, organischen Partikel.

Die volumenbezogene mittlere Partikelgröße bzw. die volumenbezogene mittlere Primärpartikelgröße kann auch als volumenbezogener Äquivalentdurchmesser der nanoskaligen, organischen Partikel bzw. der nanoskaligen, nicht-agglomerierten Einzel-Partikel, weiter bevorzugt Primärpartikel, bezeichnet werden.

Unter dem Begriff "Primärpartikelgröße" wird vorzugsweise die Größe der nicht-agglomerierten Einzel-Partikel, weiter bevorzugt Primärpartikel, verstanden. Vorzugsweise wird die Primärpartikelgröße ebenfalls anhand des Durchmessers einer äquivalenten Kugel definiert, die das gleiche Volumen wie das tatsächliche Primärpartikel aufweist.

Die Bestimmung der Größe und der Größenverteilung der Primärpartikel ist beispielsweise möglich durch Messung der Breite und Dicke der Primärpartikel mit Hilfe von Aufnahmen mit einem Transmissionselektronenmikroskop oder mit einem Rasterelektronenmikroskop, die nach Aufbringen einer Suspension auf einen geeigneten Träger und anschließender Trocknung möglich sind. Die Auszählung einer ausreichenden Anzahl an Partikeln, vorzugsweise wenigstens 500 Partikeln, weiter bevorzugt wenigstens 1000 Partikeln, ermöglicht eine Bestimmung der Primärpartikelgröße und deren Verteilung.

Unter dem Begriff "stabile, volumenbezogene mittlere Primärpartikelgröße" bzw. "stabile, volumenbezogene mittlere Partikelgröße" wird vorzugsweise die volumenbezogene mittlere Primärpartikelgröße bzw. die volumenbezogene mittlere Partikelgröße verstanden, die jeweils in wenigstens einer ersten Messung zu einem Zeitpunkt T1 und wenigstens einer zweiten Messung zu einem späteren Zeitpunkt T2 bestimmt wird, die zeitlich voneinander getrennt durchgeführt werden, wobei vorzugsweise zwischen dem Zeitpunkt T1 und dem späteren Zeitpunkt T2 eine Zeitraum von wenigstens 24 Stunden, vorzugsweise wenigstens 36 Stunden, vorzugsweise wenigstens 48 Stunden liegt und wobei vorzugsweise die beim späteren Zeitpunkt T2 gemessene volumenbezogene mittlere Primärpartikelgröße bzw. die, volumenbezogene mittlere Partikelgröße um höchstens 20 %, vorzugsweise um höchstens 10 %, zunimmt, jeweils bezogen auf die am Zeitpunkt T1 gemessene volumenbezogene mittlere Primärpartikelgröße bzw. die, volumenbezogene mittlere Partikelgröße.

Vorzugsweise liegen die in einer erfindungsgemäßen Suspension bzw. in einer erfindungsgemäßen trockenen Zusammensetzung enthaltenen erfindungsgemäßen nanoskaligen, vorzugsweise amorphen, organischen Partikel als nanoskalige, organische Einzel-Partikel, d.h. organische Primärpartikel, vor, die eine, vorzugsweise stabile, volumenbezogene mittlere Primärpartikelgröße von weniger als 350 nm, vorzugsweise von weniger als 270 nm, vorzugsweise von weniger als 190 nm, vorzugsweise von weniger als 150 nm, vorzugsweise von weniger als 100 nm, vorzugsweise aus einem Bereich zwischen 1 nm und 350 nm, vorzugsweise aus einem Bereich von 5 nm bis 330 nm, vorzugsweise aus einem Bereich von 15 nm bis 250 nm, vorzugsweise aus einem Bereich von 25 nm bis 150 nm, weiter bevorzugt aus einem Bereich von 30 nm bis 100 nm, aufweisen.

Die volumenbezogene mittlere Partikelgröße und die volumenbezogene mittlere Primärpartikelgröße können beispielsweise mittels Laserbeugung bestimmt werden. Ein geeignetes Verfahren ist beispielsweise in der ISO 13320 ("Particle size analysis - Laser diffraction methods", Ausgabedatum: 2009-10) beschrieben.

Bei der Laserbeugung wird vorzugsweise eine intensitätsbezogene Partikelverteilung ermittelt, die dann beispielsweise unter der Annahme sphärischer Partikel in die volumenbezogene Summenverteilung umgerechnet werden kann.

Alternativ können die Partikelgröße und Primärpartikelgröße mit Hilfe der dynamischen Lichtstreuung (DLS) bestimmt werden. Geeignete Verfahren zur Partikelgrößenanalyse mit Hilfe von dynamischer Lichtstreuung sind beispielsweise in der ISO 22412 ("Particle size analysis - Dynamic light scattering (DLS)", Ausgabedatum: 2008-05) angegeben.

Bei der dynamischen Lichtstreuung wird vorzugsweise ebenfalls eine intensitätsbezogene Partikelgrößenverteilung ermittelt, die dann unter der Annahme sphärischer Partikel in eine volumenbezogene Partikelgrößenverteilung umgerechnet werden kann.

Geeignete Methoden zur Bestimmung von Teilchengrößenverteilungen in Suspensionen mittels Lichtstreuung sind im Stand der Technik bekannt und können beispielsweise unter Verwendung der dynamischen Lichtstreuung, z.B. unter Verwendung eines Malvern Zetasizers^{®} Nano ZS der Malvern Instruments Limited (Malvern, Worcestershire, UK,), oder der statischen Lichtstreuung, z.B. unter Verwendung eines HORIBA^{®} LA-910 (Horiba Ltd., Kyoto, JP), durchgeführt werden.

Alternativ können die Partikelgröße und die Partikelgrößenverteilung durch Sedimentationsverfahren im Fliehkraftfeld in Flüssigkeit, vorzugsweise Wasser, vorzugsweise mit Hilfe von Fotofliehkraftsedimentationsverfahren, bestimmt werden. Ein geeignetes Fotofliehkraftsedimentationsverfahren ist beispielsweise in der ISO 13318-2 ("Determination of particle size distribution by centrifugal liquid sedimentation methods - Part 2: Photocentrifuge method", Ausgabedatum: 2007-09) beschrieben.

Ein geeignetes Gerät zur Durchführung von Fotofliehkraftsedimentationsverfahren ist beispielsweise der LUMiSizer^{®}, der kommerziell von der LUM GmbH (Berlin, DE) erhältlich ist.

Alternativ können die Partikelgröße und die Partikelgrößenverteilung durch Sedimentationsverfahren im Fliehkraftfeld in Flüssigkeit, vorzugsweise Wasser, vorzugsweise mit Hilfe einer analytischen Ultrazentrifuge (AUZ) bestimmt werden. Ein geeignetes Verfahren wird beispielsweise in der DE 38 33 974 A1 beschrieben.

Die Messung der "stabilen volumenbezogenen mittleren Primärpartikelgröße" bzw. "stabilen, volumenbezogenen mittleren Partikelgröße" deren jeweiliger Verteilung erfolgt in wenigstens einer ersten Messung zu einem Zeitpunkt T1 und wenigstens einer zweiten Messung zu einem späteren Zeitpunkt T2, die zeitlich voneinander getrennt durchgeführt werden, wobei vorzugsweise zwischen dem Zeitpunkt T1 und dem späteren Zeitpunkt T2 eine Zeitraum von wenigstens 24 Stunden, vorzugsweise wenigstens 36 Stunden, vorzugsweise wenigstens 48 Stunden liegt, mit einem der vorgenannten Verfahren, wobei vorzugsweise die wenigstens eine erste Messung zu einem Zeitpunkt T1 und die wenigstens eine zweite Messung zu einem späteren Zeitpunkt T2 mit dem selben Verfahren weiter bevorzugt unter Verwendung des selben Messgerätes erfolgt.

Erfindungsgemäß werden unter dem Begriff "nanoskalige, organische Partikel" Partikel verstanden, die eine, vorzugsweise stabile, volumenbezogene mittlere Primärpartikelgröße von weniger als 350 nm, vorzugsweise von weniger als 270 nm, vorzugsweise von weniger als 190 nm, vorzugsweise von weniger als 150 nm, vorzugsweise von weniger als 100 nm, vorzugsweise aus einem Bereich zwischen 1 nm und 350 nm, vorzugsweise aus einem Bereich von 5 nm bis 330 nm, vorzugsweise aus einem Bereich von 15 nm bis 250 nm, vorzugsweise aus einem Bereich von 25 nm bis 150 nm, weiter bevorzugt aus einem Bereich von 30 nm bis 100 nm, aufweisen und wenigstens eine organische Verbindung und Übergangsmetall-Kationen und/oder, vorzugsweise positiv geladene, Hydroxid-Komplexe davon umfassen oder daraus bestehen, wobei zumindest an der Oberfläche der nanoskaligen, vorzugsweise amorphen, organischen Partikel wenigstens teilweise, vorzugsweise vollständig, Übergangsmetall-Kationen und/oder, vorzugsweise positiv geladene, Hydroxid-Komplexe davon angeordnet, vorzugsweise gebunden, sind, wobei die wenigstens eine organische Verbindung wenigstens 6 C-Atome, vorzugsweise 6 C-Atome bis 100 C-Atome, vorzugsweise 7 C-Atome bis 70 C-Atome, weiter bevorzugt 8 C-Atome bis 50 C-Atome, weiter bevorzugt 9 C-Atome bis 28 C-Atome, und wenigstens 1, vorzugsweise 1 bis 10, vorzugsweise 1 bis 6, vorzugsweise 2 bis 4, weiter bevorzugt 1, Säuregruppen-haltigen Rest aufweist, wobei vorzugsweise der Anteil der organischen Verbindung an den nanoskaligen, organischen Partikeln mindestens 75 Gew.-%, weiter bevorzugt mindestens 80 Gew.-%, weiter bevorzugt mindestens 85 Gew.-%, weiter bevorzugt mindestens 89 Gew.-%, jeweils bezogen auf das Gesamtgewicht der trockenen nanoskaligen, organischen Partikel, beträgt, und wobei die Übergangsmetall-Kationen vorzugsweise eine formale Oxidationszahl aus einem Bereich von +1 bis +6, vorzugsweise von +2 bis +5, weiter bevorzugt +3 bis +4, weiter bevorzugt von +4, aufweisen und weiter bevorzugt aus der Gruppe, die aus Titan, Zirkonium, Hafnium, Cer, Yttrium und Kombinationen davon, weiter bevorzugt Titan, Zirkonium, Hafnium und Kombinationen davon, weiter bevorzugt Zirkonium, Hafnium und Kombinationen davon, besteht, ausgewählt werden.

Die mittlere Partikelgröße bzw. die mittlere Primärpartikelgröße ist vorzugsweise der Median der jeweiligen kumulativen Partikelgrößenverteilung, d.h. die Partikelgröße bzw. Primärpartikelgröße, die von 50% der gemessenen Teilchen unter- bzw. überschritten wird.

Vorzugsweise weisen die in einer erfindungsgemäßen Suspension bzw. in einer erfindungsgemäßen trockenen Zusammensetzung enthaltenen erfindungsgemäßen nanoskaligen, vorzugsweise amorphen, organischen Partikel eine, vorzugsweise stabile, Größenverteilung der volumenbezogenen Primärpartikelgröße auf, bei der 90% der Einzel-Partikel, vorzugsweise 95% der Einzel-Partikel, weiter bevorzugt 99% der Einzel-Partikel, jeweils bezogen auf die Gesamtvolumen der in der Suspension enthaltenen nanoskaligen, organischen Partikel, kleiner als 650 nm, vorzugsweise kleiner als 500 nm, vorzugsweise kleiner als 350 nm, vorzugsweise kleiner als 270 nm, vorzugsweise kleiner als 190 nm, weiter bevorzugt kleiner als 150 nm, weiter bevorzugt kleiner als 120 nm, sind.

Weiter bevorzugt weisen die in einer erfindungsgemäßen Suspension bzw. in einer erfindungsgemäßen trockenen Zusammensetzung enthaltenen erfindungsgemäßen nanoskaligen, vorzugsweise amorphen, organischen Partikel eine, vorzugsweise stabile, Größenverteilung der volumenbezogenen Primärpartikelgröße auf, bei der weniger als 15%, vorzugsweise weniger als 10%, weiter bevorzugt weniger als 5%, der Einzel-Partikel kleiner als 1 nm, vorzugsweise kleiner als 5 nm, weiter bevorzugt kleiner als 10 nm, sind.

Die in einer erfindungsgemäßen Suspension bzw. in einer erfindungsgemäßen trockenen Zusammensetzung enthaltenen erfindungsgemäßen nanoskaligen, organischen Partikel können eine beliebige Form, beispielsweise sphärisch, rund, kubisch, nadelförmig, anisotrop oder einer Kombination davon, aufweisen.

Weiter bevorzugt sind die in einer erfindungsgemäßen Suspension bzw. in einer erfindungsgemäßen trockenen Zusammensetzung enthaltenen erfindungsgemäßen nanoskaligen, organischen Partikel kristallin, amorph, oder eine Mischung davon, vorzugsweise amorph.

Die in einer erfindungsgemäßen Suspension enthaltenen erfindungsgemäßen nanoskaligen, organischen Partikel sind vorzugsweise bei einer Temperatur von 25°C, einen Druck von 1013 mbar und einem pH-Wert aus einem Bereich von 0 bis 7, vorzugsweise von 0,5 bis 4, weiter bevorzugt von1 bis 3, weiter bevorzugt von 1,15 bis 2,5 weiter bevorzugt von 1,25 bis 2,25, in der wässrigen Phase der erfindungsgemäßen Suspension schwerlöslich, vorzugsweise nicht löslich.

Vorzugsweise weisen die in einer erfindungsgemäßen Suspension enthaltenen erfindungsgemäßen nanoskaligen, organischen Partikel bei einer Temperatur von 25°C, einem Druck von 1013 mbar und einem pH-Wert von 1 eine Löslichkeit in Wasser, die vorzugsweise gemäß DIN EN ISO 787-8 bestimmt wird, von 15 g/l oder weniger, vorzugsweise von 10 g/l oder weniger, vorzugsweise von 5 g/l oder weniger, weiter bevorzugt von 900 mg/l oder weniger, weiter bevorzugt von 500 mg/l oder weniger, auf.

Die in einer erfindungsgemäßen Suspension bzw. in einer erfindungsgemäßen trockenen Zusammensetzung enthaltenen erfindungsgemäßen nanoskaligen, organischen Partikel umfassen erfindungsgemäß wenigstens eine organische Verbindung mit wenigstens 6 C-Atomen, vorzugsweise 6 C-Atomen bis 100 C-Atomen, vorzugsweise 7 C-Atomen bis 70 C-Atomen, weiter bevorzugt 8 C-Atomen bis 50 C-Atomen, weiter bevorzugt 9 C-Atomen bis 28 C-Atomen, und wenigstens 1 Säuregruppen-haltigen Rest, vorzugsweise 1 bis 10 Säuregruppen-haltige Reste, vorzugsweise1 bis 6 Säuregruppen-haltige Reste, vorzugsweise 2 bis 4 Säuregruppen-haltige Reste, weiter bevorzugt 1 Säuregruppen-haltigen Rest, der vorzugsweise jeweils unabhängig voneinander aus der Gruppe, die aus Carboxygruppe, vinyloge? Carboxygruppe, Enolgruppe, Sulfonsäuregruppe, Sulfatgruppe, Phosphonsäuregruppe, Phosphatgruppe, acider NH-Gruppe, acider CH-Gruppe, acider OH-Gruppe und Kombinationen davon, vorzugsweise Carboxygruppe, vinyloge? Carboxygruppe, Enolgruppe, Sulfonsäuregruppe, Sulfatgruppe, Phosphonsäuregruppe, Phosphatgruppe und Kombinationen davon, vorzugsweise Carboxygruppe, vinyloge Carboxygruppe, Enolgruppe, Sulfonsäuregruppe, Phosphonsäuregruppe und Kombinationen davon, weiter bevorzugt Carboxygruppe, Sulfonsäuregruppe, Phosphonsäuregruppe und Kombinationen davon, weiter bevorzugt Carboxygruppe, besteht, ausgewählt wird.

Die wenigstens eine organische Verbindung kann daher als Brønsted-Säure bezeichnet werden, die wenigstens ein Proton abgeben kann.

Weiter bevorzugt weist die wenigstens eine organische Verbindung bei einer Temperatur von 25°C, einem Druck von 1013 mbar und einem pH-Wert aus einem von Bereich zwischen 1 und 3 vorzugsweise eine Löslichkeit in Wasser, die vorzugsweise gemäß DIN EN ISO 787-8 bestimmt wird, von 15 g/l oder weniger, vorzugsweise von 10 g/l oder weniger, vorzugsweise von 5 g/l oder weniger, weiter bevorzugt von 900 mg/l oder weniger, weiter bevorzugt von 500 mg/l oder weniger, auf.

Beispielsweise kann die Löslichkeit von für die vorliegende Erfindung geeigneten organischen Verbindungen in Wasser bei den oben angegebenen Bedingungen ebenfalls dem "CRC Handbook of Chemistry and Physics" (98th Edition, CRC Press, Boca Raton, Florida, USA, Herausgeber: Lide, D.R. et al. (2008), Seiten 3-1 bis 3-522 und Seiten 8-85 bis 8-115) entnommen werden.

Vorzugsweise weist die wenigstens eine organische Verbindung einen pKs-Wert, gemessen in Wasser bei 25°C und einem Druck von 1013 mbar, von 12,5 oder kleiner, vorzugsweise von 9,25 oder kleiner, vorzugsweise in einem Bereich zwischen 8,2 und 1,0, vorzugsweise in einem Bereich zwischen 7,9 und 2,5, vorzugsweise in einem Bereich von 7,25 bis 3,5, auf.

Der pKs-Wert bezeichnet den negativen dekadischen Logarithmus der Säurekonstante Ks.

Geeignete Verfahren zur Bestimmung des pKs-Wertes sind im Stand der Technik bekannt und werden beispielsweise in der OECD-Richtlinie zur Testung von Chemikalien Nr. 112 ("Dissociation Constants in Water: titration method - spectrophotometric method - conductometric method"; angenommen: 12. Mai 1981) beschrieben.

Eine geeignete Methode zur Bestimmung des pKs-Wertes ist beispielsweise die potentiometrische Titration oder die spektrophotometrische Titration. Beispielsweise kann die Bestimmung des pKs-Wertes mit Hilfe des Gerätes Sirius T3, das kommerziell erhältlich ist von Sirius Analytical Ltd. (East Sussex, UK), erfolgen.

Vorzugsweise wird der pKs-Wert durch potentiometrische Titration unter Verwendung eines Sirius T3-Geräts mit einer Ag/AgCl Referenz-pH Elektrode in einer 0,15 M KCI-Lösung bei einer Temperatur von 25°C und unter Argon Atmosphäre mit einem Druck von 1013 mbar durch Titration mit einer standardisierten 0,5 M KOH-Lösung als Titrationsreagenz bestimmt.

Eine geeignete organische Verbindung kann eine einprotonige Säure oder eine mehrprotonige Säure sein. Beispielsweise tritt bei einer mehrprotonigen Säure eine schrittweise Protolyse, d.h. Abspaltung von sauren Protonen auf. Für jede Protolysationsstufe kann eine eigene Säurekonstante bzw. pKs-Wert ermittelt werden.

Vorzugsweise weist eine organische Verbindung, die eine mehrprotonige Säure ist, zwei oder mehr pKs-Werte auf, wobei die entsprechenden pKs-Werte, jeweils gemessen in Wasser bei 25°C und einem Druck von 1013 mbar, 12,5 oder kleiner, vorzugsweise 9,25 oder kleiner, vorzugsweise in einem Bereich zwischen 8,2 und 1,0, vorzugsweise in einem Bereich zwischen 7,9 und 2,5, vorzugsweise in einem Bereich von 7,25 bis 3,5, sind.

Beispielsweise weist Adipinsäure (Hexandicarbonsäure) zwei Protolysationsstufen auf, wobei die erste Protolysationsstufe einen pKs-Wert von 4,43 und die zweite Protolysationsstufe einen pKs-Wert von 5,42 aufweist.

Beispielsweise können pKs-Werte von für die vorliegende Erfindung geeigneten organischen Verbindungen bei den oben angegebenen Bedingungen ebenfalls dem "CRC Handbook of Chemistry and Physics" (98th Edition, CRC Press, Boca Raton, Florida, USA, Herausgeber: Lide, D.R. et al. (2008), Seiten 8-42 bis 8-51) entnommen werden.

Geeignete organische Verbindungen mit wenigstens 6 C-Atomen und wenigstens 1 der vorgenannten Säuregruppen-haltigen Reste sind beispielsweise pharmazeutische Wirkstoffe, Pestizide, Säurefarbstoffe, oder Mischungen davon.

Geeignete pharmazeutische Wirkstoffe sind beispielsweise Antidepressiva, Betarezeptorenblocker, Antidiabetika, Analgetika, Antiphlogistika, Antirheumatika, Antihypotonika, Antihypertonika, Psychopharmaka, Tranquilizer, Antiemetika, Muskelrelaxantien, Glucocorticoide, Mittel zur Behandlung von Colitis ulcerosa oder Morbus Crohn, Antiallergika, Antibiotika, Antiepileptika, Antikoagulantia, Antimykotika, Antitussiva, Arteriosklerosemittel, Diuretika, Enzyme, Enzyminhibitoren, Gichtmittel, Hormone und deren Hemmstoffe, Herzglykoside, Immuntherapeutika und Zytokine, Laxantien, Lipidsenker, Magen-Darmtherapeutika, Migränemittel, Otologika, Parkinsonmittel, Schilddrüsentherapeutika, Spasmolytika, Thrombozytenaggregationshemmer, Vitamine, Zytostatika, Metastasenhemmer, Phytopharmaka, Chemotherapeutika, Carotinoide, Aminosäuren oder Mischungen davon, die jeweils wenigstens 1 der vorgenannten Säuregruppen-haltigen Reste, vorzugsweise 1 bis 6 Säuregruppen-haltige Reste, aufweisen, der/die weiter bevorzugt jeweils unabhängig voneinander aus der Gruppe, die aus Carboxygruppe, Sulfonsäuregruppe, Phosphonsäuregruppe und Kombinationen davon, weiter bevorzugt Carboxygruppe, Sulfonsäuregruppe und Kombinationen davon, weiter bevorzugt Carboxygruppe, besteht, ausgewählt werden.

Geeignete pharmazeutische Wirkstoffe mit sauren Gruppen sind beispielsweise: Aceclofenac (INN), Acediasulfon (INN), Acedoben (INN), Aceglutamid (INN), Acemetacin (INN), Acetoxyvalerensäure, Acetylcystein (INN), Acetylleucin (INN), Acetylmethionin (INN), Acetylsalicylsäure, 3,5-Diiodacetylsalicylsäure, Acexaminsäure (INN), Acipimox (INN), Acitretin (INN), Aconiazid (INN), Acrivastin (INN), Actarit (INN), Adapalen (INN), Alacepril (INN), Alatrofloxacin (INN), Alclofenac (INN), Aleplasinin (INN), Alitretinoin (INN), Alminoprofen (INN), Alprostadil (INN), Alvimopan (INN), Ambrisentan (INN), Amfenac (INN), 4-Aminosalicylsäure, Amoxicillin (INN), Ampicillin (INN), Amphotericin B (INN), Abietinsäure, Neoabietinsäure, Levopimarsäure, Palustrinsäure, Dehydroabietinsäure, Pimarsäure, Sandaracopimarsäure, Podocarpinsäure, Copalsäure, Eperuesäure, Labdanolsäure, Pinifolsäure, Dammarolsäure, Ursolsäure, Ursonsäure, β-Boswelliasäure, 11-Keto-β-boswelliasäure, 3-O-Acetyl-β-boswelliasäure, 3-O-Acetyl-11-keto-β-boswelliasäure, Lupeolsäure, Isomasticadienonsäure, Masticadienonsäure, Elemolsäure, Elemonsäure, Oleanonsäure, Oleanolsäure, Moronsäure, α-Boswelliasäure, 3-O-Acetyl-11-keto-α-boswelliasäure, 3-O-Acetyl-α-boswelliasäure, Argatroban (INN), Artesunat (INN), Aspartam, Aspoxicillin (INN), Atorvastatin (INN), Azelainsäure (INN), Azidocillin (INN), Azlocillin (INN), Aztreonam (INN), Baclofen (INN), Balofloxacin (INN), Balsalazid (INN), Benazepril (INN), Bendazac (INN), Bensuldazinsäure (INN), Benoxaprofen (INN), Benzoesäure, 2-Hydroxy-5-octanoylbenzoesäure, 4-Methoxybenzoesäure, 4-Aminobenzoesäure, Benzylpenicillin (INN), Phenoxymethylpenicillin (INN), Bepotastin (INN), Beraprost (INN), Betamipron (INN), Bexaroten (INN), Bezafibrat (INN), Biotin, Bromfenac (INN), Bumadizon (INN), Bumetanid (INN), Butibufen (INN), Canrenonsäure (INN), Capobeninsäure (INN), Captopril (INN), Carbenicillin (INN), Carbenoxolon (INN), Carbidopa (INN), Carboprost (INN), Carfecillin (INN), Carindacillin (INN), Carnosolsäure, Carnosin, Carprofen (INN), Carglumsäure (INN), Carumonam (INN), Carzenid, Cefacetril (INN), Cefaclor (INN), Cefadroxil (INN), Cefalexin (INN), Cefaloglycin (INN), Cefaloridin (INN), Cefalotin (INN), Cefamandol (INN), Cefaparol (INN), Cefapirin (INN), Cefatrizin (INN), Cefazolin (INN), Cefbuperazon (INN), Cefdinir (INN), Cefolitoren (INN), Cefetamet (INN), Cefepim (INN), Cefixim (INN), Cefmenoxim (INN), Cefmetazol (INN), Cefminox (INN), Cefodizim (INN), Cefonicid (INN), Cefoperazon (INN), Ceforanid (INN), Cefoselis (INN), Cefotaxim (INN), Cefotetan (INN), Cefotiam (INN), Cefovecin (INN), Cefoxazol (INN), Cefoxitin (INN), Cefozopran (INN), Cefpimizol (INN), Cefpodoxium (INN), Cefpiramid (INN), Cefprozil (INN), Cefradin (INN), Cefroxadin (INN), Cefsulodin (INN), Ceftazidim (INN), Cefteram (INN), Ceftezol (INN), Ceftibuten (INN), Ceftiofur (INN), Ceftiolen (INN), Ceftizoxim (INN), Ceftriaxon (INN), Ceftrizol (INN), Cefuroxim (INN), Cefuzonam (INN), Cetirizin (INN), Ciprofloxacin (INN), Cholsäure, Chenodeoxycholsäure, Ursodesoxycholsäure, Desoxycholsäure, Lithocholsäure, Taurocholsäure, Dehydrocholsäure, Chlorambucil (INN), Chromocarb (INN), Cromoglicinsäure (INN), Ciclacilin (INN), Cicloxilinsäure, Cilazapril (INN), Cinametinsäure, Cinoxacin (INN), Ciprofibrat (INN), Ciprofloxacin (INN), Clanobutin (INN), Clavulansäure (INN), Clidanac (INN), Clinofibrat (INN), Clobuzarit (INN), Clometazin (INN), Cinmetacin (INN), Clonixin (INN), Clorazepat (INN), Cloxacillin (INN), Cromoglicinsäure (INN), Cyclobutyrol (INN), Cynarin, Danofloxacin (INN), Daptomycin (INN), Delapril (INN), Dexibuprofen (INN), Dexketopropfen (INN), Dextrothyroxin (INN), Diacerein (INN), Dibromotyrosin, 4-(2,4-Dichlorphenoxy)buttersäure, 2,4-Dichlorphenoxyessigsäure, Diclofenac (INN), Dicloxacillin (INN), Difenoxin (INN), Difloxacin (INN), Diflunisal (INN), Diiodotyrosine, Dimecrotinsäure (INN), Dinoprost (INN), Dinoproston (INN), Dipyrocetyl (INN), Divalproex (INN), Doconexent (INN), Doripenem (INN), Droxidopa (INN), Ecabet (INN), Eflornithin (INN), Eltenac (INN), Eltrombopag (INN), Enalapril (INN), Enalaprilat (INN), Enfenaminsäure, Enoxacin (INN), Enoxolon, Enrofloxacin (INN), Epalrestat (INN), Epoprostenol (INN), Eprosartan (INN), Eptifibatid (INN), Erdostein (INN), Ertapenem (INN), Etacrynsäure (INN), Etodolac (INN), Faropenem (INN), Febuxostat (INN), Felbinac (INN), Fenbufen (INN), Fencibutirol (INN), Fenclozinsäure, Fenofibrinsäure, Fenoprofen (INN), Fentiazac (INN), Fexofenadin (INN), Flavodinsäure (INN), Fleroxacin (INN), Flomoxef (INN), Flucloxacillin (INN), Flufenaminsäure (INN), Flumequin (INN), Flunixin (INN), Flunoxaprofen (INN), Fluprostenol (INN), Flurbiprofen (INN), Flutiazin (INN), Folsäure, Folinsäure, Fosinopril (INN), Fudostein (INN), Fumagillin (INN), Furosemid (INN), Fusidinsäure (INN), Gabapentin (INN), Garenoxacin (INN), Gatifloxacin (INN), Gemfibrozil (INN), Gemifloxacin (INN), Gibberellinsäure, Gibberellin A4, Gibberellin A7, Glutaminsäure, Glycyrrhizin, Grepafloxacin (INN), Hetacillin (INN), Hyaluronsäure, Ibafloxacin (INN), Ibuprofen (INN), Icatibant (INN), Icosapent, Iloprost (INN), Imidapril (INN), Imipenem (INN), Indobufen (INN), Indometacin (INN), Indoprofen (INN), Dexindoprofen (INN), locetamsäure, lodipamide (INN), lodoxamsäure, loglicinsäure, lopansäure, lotalamsäure, lotroxinsäure, Isotretinoin (INN), Isoxepac (INN), Ketoprofen (INN), Ketorolac (INN), Lactobionsäure, Lasalocid A (INN), Latamoxef (INN), Letostein (INN), Levocabastin (INN), Levocetirizin (INN), Levofloxacin (INN), Levofolinsäure, Levomefolsäure, Levopropicillin (INN), Levothyroxin (INN), Liponsäure, Limaprost (INN), Liothyronin (INN), Lisinopril (INN), Lobenzarit (INN), Lodoxamid (INN), Lomefloxacin, Lonazolac (INN), Lonidamin (INN), Loracarbef (INN), Lornoxicam (INN), Loxoprofen (INN), Lubiproston (INN), Lumiracoxib (INN), Luprostiol (INN), Lymecyclin (INN), Marbofloxacin (INN), Maslinsäure, Mecillinam (INN), Meclofenaminsäure (INN), Mefenaminsäure (INN), Mefolinsäure, Meglutol (INN), Meloxicam (INN), Melphalan (INN), Meropenem (INN), Mesalazin (INN), Metampicillin (INN), Methotrexat (INN), Methyldopa (INN), Metiazinsäure, Methicillin (INN), Metirosin (INN), Mezlocillin (INN), Miloxacin (INN), Miroprofen (INN), Mitiglinid (INN), Moexipril (INN), Mofezolac (INN), Monensin (INN), Montelukast (INN), Moxifloxacin (INN), Mupirocin (INN), Mycophenolsäure (INN), Nadifloxacin (INN), Nadroparin (INN), Nafcillin (INN), Nalidixinsäure (INN), 1-Naphthoesäure, Naphthalen-1-essigsäure, Naproxen (INN), Narasin (INN), Natamycin (INN), Nateglinid (INN), Nedocromil (INN), Nicotinsäure (INN), Nifluminsäure (INN), Nitrocefin (INN), Norfloxacin (INN), Nystatin (INN), Ofloxacin (INN), Olopatadine (INN), Olsalazin (INN), Orazamid (INN), Orbifloxacin (INN), Orotsäure, Oxaceprol (INN), Oxacillin (INN), Oxaprozin (INN), Oxitriptan (INN), Ozagrel (INN), Pamoasäure, Panipenem (INN), Pantothensäure, Pazufloxacin (INN), Pefloxacin (INN), Pemetrexed (INN), Penicillin G (INN), Peramivir (INN), Perindopril (INN), Phenethicillin (INN), Phenoxymethylpenicillin (INN), Phthalylsulfathiazol (INN), Pidolinsäure, Pidotimod (INN), Pipemidsäure (INN), Piperacillin (INN), Pirenoxin (INN), Piretanid (INN), Piromidinsäure (INN), Piroxicam (INN), Pirprofen (INN), Pitavastatin (INN), Pivagabin (INN), Pralatrexat (INN), Pranoprofen (INN), Pregabalin (INN), Probenecid (INN), Procodazol (INN), Proglumid (INN), Propicillin (INN), Protizinsäure (INN), Prulifloxacin (INN), Quinapril (INN), Raltitrexed (INN), Ramatroban (INN), Ramipril (INN), Rebamipid (INN), Repaglinid (INN), Rhein, Diacerein (INN), Ritiometan (INN), Robenacoxib (INN), Romurtid (INN), Rosmarinsäure, Rosoxacin (INN), Rotraxat (INN), Rosuvastatin (INN), Rufloxacin (INN), Sacubitril (INN), Salicylsäure, 3,5-Diiodsalicylsäure, 5-Hydroxysalicylsäure, Salinomycin (INN), Salsalat (INN), Sarafloxacin (INN), Sarpogrelat (INN), Semduramicin (INN), Seratrodast (INN), Sivelestat (INN), Sofalcon (INN), Sorbinsäure, Spagluminsäure (INN), Sparfloxacin (INN), Spirapril (INN), Stepronin (INN), Sulbactam (INN), Sulbenicillin (INN), Sulfasalazin (INN), Sulfasuccinamid (INN), Sulindac (INN), Suprofen (INN), Suxibuzon (INN), Tamibaroten (INN), Tarenflurbil (INN), Tazobactam (INN), Teicoplanin (INN), Telmestein (INN), Telmisartan (INN), Temocapril (INN), Temocillin (INN), Tenoxicam (INN), Tiagabin (INN), Tianeptin (INN), Tiaprofensäure (INN), Tiaprost (INN), Ticarcillin (INN), Tiopinac (INN), Tiratricol (INN), Tirofiban (INN), Tolfenaminsäure (INN), Tolmetin (INN), Tosufloxacin (INN), Trandolapril (INN), Tranilast (INN), Trepibuton (INN), Treprostinil (INN), Tretinoin (INN), Triflusal (INN), Tropesin (INN), Trovafloxacin (INN), Ubenimex (INN), Undecylensäure, Ursodeoxycholsäure, Vanillinsäure, Isovanillinsäure, Valerensäure, Valproinsäure (INN), Valsartan (INN), Vancomycin (INN), Vebufloxacin (INN), Vedaprofen (INN), Vigabatrin (INN), Zaltoprofen (INN), Zanamivir (INN), Zofenopril (INN), Zimtsäure, 4-Hydroxyzimtsäure, 3,4-Dihydroxyzimtsäure, Zomepirac (INN) oder Mischungen davon.

Bei einer bevorzugten Ausführungsform wird die wenigstens eine organische Verbindung aus der Gruppe der sauren antiphlogistischen antipyretischen Analgetika ausgewählt, die auch als nichtsteroidale Antiphlogistika (NSAP) oder Säureantiphlogistika bezeichnet werden können.

Vorzugsweise wird die wenigstens eine organische Verbindung aus der Gruppe, die aus Anthracen-Derivaten, Salicylsäure-Derivaten, Anthranilsäure-Derivaten, Arylessigsäure-Derivaten, Arylpropionsäure-Derivaten, Enolsäure-Derivaten und Kombinationen davon, vorzugsweise Salicylsäure-Derivaten, Anthranilsäure-Derivaten, Arylessigsäure-Derivaten, Arylpropionsäure-Derivaten und Mischungen davon, weiter bevorzugt Arylessigsäure-Derivaten, Arylpropionsäure-Derivaten und Mischungen davon, weiter bevorzugt Arylpropionsäure-Derivaten, besteht, ausgewählt.

Geeignete Anthracen-Derivaten sind beispielsweise Rhein (CAS-Nummer: 478-43-3), Diacerein (INN) (CAS-Nummer: 13739-02-1) oder Mischungen davon.

Geeignete Salicylsäure-Derivate sind beispielsweise Salicylsäure (CAS-Nummer: 69-72-7), 3,5-Diiodsalicylsäure (CAS-Nummer: 133-91-5), 5-Hydroxysalicylsäure (CAS-Nummer: 490-79-9), Acetylsalicylsäure (CAS-Nummer: 50-78-2), 3,5-Diiodacetylsalicylsäure (CAS-Nummer: 36415-60-8), Mesalazin (INN) (CAS-Nummer: 89-57-6), Balsalazid (INN) (CAS-Nummer: 80573-04-2), Olsalazin (INN) (CAS-Nummer: 15722-48-2), Sulfasalazin (INN) (CAS-Nummer: 599-79-1), Salsalat (INN) (CAS-Nummer: 552-94-3), Dipyrocetyl (INN) (CAS-Nummer: 486-79-3), Diflunisal (INN) (CAS-Nummer: 22494-42-4), Triflusal (INN) (CAS-Nummer: 322-79-2) oder Mischungen davon.

Geeignete Anthranilsäure-Derivate sind beispielsweise Mefenaminsäure (INN) (CAS-Nummer: 61-68-7), Flufenaminsäure (INN) (CAS-Nummer: 530-78-9), Tolfenaminsäure (INN) (CAS-Nummer: 13710-19-5), Meclofenaminsäure (INN) (CAS-Nummer: 644-62-2), Nifluminsäure (INN) (CAS-Nummer: 4394-00-7), Flutiazin (INN) (CAS-Nummer: 7220-56-6), Flunixin (INN) (CAS-Nummer: 38677-85-9), Clonixin (INN) (CAS-Nummer: 17737-65-4) oder Mischungen davon.

Geeignete Arylessigsäure-Derivate sind beispielsweise Aceclofenac (INN) (CAS-Nummer: 89796-99-6), Acemetacin (INN) (CAS-Nummer: 53164-05-9), Alclofenac (INN) (CAS-Nummer: 22131-79-9), Amfenac (INN) (CAS-Nummer: 51579-82-9), Bendazac (INN) (CAS-Nummer: 20187-55-7), Bromfenac (INN) (CAS-Nummer: 91714-94-2), Bumadizon (INN) (CAS-Nummer: 91714-94-2), Clometazin (INN) (CAS-Nummer: 25803-14-9), Clobuzarit (INN) (CAS-Nummer: 22494-47-9), Cinmetacin (INN) (CAS-Nummer: 20168-99-4), Diclofenac (INN) (CAS-Nummer: 15307-86-5), Eltenac [INN] (CAS-Nummer: 72895-88-6), Etodolac (INN) (CAS-Nummer: 41340-25-4), Felbinac (INN) (CAS-Nummer: 5728-52-9), Fenclozinsäure (CAS-Nummer: 17969-20-9), Fentiazac (INN) (CAS-Nummer: 18046-21-4), Indometacin (INN) (CAS-Nummer: 53-86-1), Isoxepac (INN) (CAS-Nummer: 55453-87-7), Ketorolac (INN) (CAS-Nummer: 74103-06-3), Lonazolac (INN) (CAS-Nummer: 53808-88-1), Mofezolac (INN) (CAS-Nummer: 78967-07-4), Sulindac (INN) (CAS-Nummer: 38194-50-2), Tiopinac (INN) (CAS-Nummer: 61220-69-7), Tolmetin (INN) (CAS-Nummer: 26171-23-3), Zomepirac (INN) (CAS-Nummer: 33369-31-2), Lumiracoxib (INN) (CAS-Nummer: 220991-20-8), Robenacoxib (INN) (CAS-Nummer: 220991-32-2), Metiazinsäure (CAS-Nummer: 13993-65-2) oder Mischungen davon.

Geeignete Arylpropionsäure-Derivate sind beispielsweise Alminoprofen (INN) (CAS-Nummer: 39718-89-3), Benoxaprofen (INN) (CAS-Nummer: 51234-28-7), Carprofen (INN) (CAS-Nummer: 53716-49-7), Fenbufen (INN) (CAS-Nummer: 36330-85-5), Fenoprofen (INN) (CAS-Nummer: 29679-58-1), Flunoxaprofen (INN) (CAS-Nummer: 66934-18-7), Flurbiprofen (INN) (CAS-Nummer: 5104-49-4), Tarenflurbil (INN) (CAS-Nummer: 51543-40-9), Esflurbiprofen (INN) (CAS-Nummer: 51543-39-6), Ibuprofen (INN) (CAS-Nummer: 15687-27-1), Dexibuprofen (INN) (CAS-Nummer: 51146-56-6), Indoprofen (INN) (CAS-Nummer: 31842-01-0), Dexindoprofen (INN) (CAS-Nummer: 53086-13-8), Ketoprofen (INN) (CAS-Nummer: 22071-15-4), Dexketoprofen (INN) (CAS-Nummer: 22161-81-5), Loxoprofen (INN) (CAS-Nummer: 68767-14-6), Miroprofen (INN) (CAS-Nummer: 55843-86-2), Naproxen (INN) (CAS-Nummer: 22204-53-1), Oxaprozin (INN) (CAS-Nummer: 21256-18-8), Pranoprofen (INN) (CAS-Nummer: 52549-17-4), Protizinsäure (INN) (CAS-Nummer: 13799-03-6), Pirprofen (INN) (CAS-Nummer: 31793-07-4), Suprofen (INN) (CAS-Nummer: 40828-46-4), Tiaprofensäure (CAS-Nummer: 33005-95-7), Vedaprofen (INN) (CAS-Nummer: 71109-09-6), Zaltoprofen (INN) (CAS-Nummer: 74711-43-6) oder Mischungen davon.

Geeignete Enolsäure-Derivate, die auch als Oxicame bezeichnet werden können, sind beispielsweise Piroxicam (INN) (CAS-Nummer: 36322-90-4), Meloxicam (INN) (CAS-Nummer: 71125-38-7), Tenoxicam (INN) (CAS-Nummer: 59804-37-4), Lornoxicam (INN) (CAS-Nummer: 70374-39-9), Isoxicam (INN) (CAS-Nummer: 34552-84-6) oder Mischungen davon.

Bei einer weiter bevorzugten Ausführungsform wird die wenigstens eine organische Verbindung aus der Gruppe, die aus Rhein, Diacerein, Salicylsäure, 3,5-Diiodsalicylsäure, 5-Hydroxysalicylsäure, Acetylsalicylsäure, 3,5-Diiodacetylsalicylsäure, Mesalazin, Balsalazid, Olsalazin, Sulfasalazin, Salsalat, Dipyrocetyl, Diflunisal, Triflusal, Mefenaminsäure, Flufenaminsäure, Tolfenaminsäure, Meclofenaminsäure, Nifluminsäure, Flutiazin, Flunixin, Clonixin, Clometazin, Clobuzarit, Cinmetacin, Diclofenac, Indometacin, Aceclofenac, Acemetacin, Lonazolac, Tolmetin, Ketorolac, Lumiracoxib, Felbinac, Fenclozinsäure, Alclofenac, Amfenac, Bromfenac, Eltenac, Etodolac, Mofezolac, Sulindac, Bendazac, Bumadizon, Fentiazac, Isoxepac, Metiazinsäure, Zomepirac, Tiopinac, Robenacoxib, Benoxaprofen, Carprofen, Indoprofen, Ibuprofen, Ketoprofen, Naproxen, Dexketoprofen, Flurbiprofen, Tarenflurbil, Dexibuprofen, Tiaprofensäure, Alminoprofen, Fenbufen, Fenoprofen, Flunoxaprofen, Loxoprofen, Pirprofen, Pranoprofen, Protizinsäure, Suprofen, Vedaprofen, Zaltoprofen, Miroprofen, Oxaprozin, Piroxicam, Meloxicam, Tenoxicam, Lornoxicam, Isoxicam und Mischungen davon, vorzugsweise Benoxaprofen, Indoprofen, Ibuprofen, Ketoprofen, Naproxen, Dexketoprofen, Flurbiprofen, Tarenflurbil, Dexibuprofen, Tiaprofensäure, Alminoprofen, Carprofen, Fenbufen, Fenoprofen, Flunoxaprofen, Loxoprofen, Pirprofen, Pranoprofen, Protizinsäure, Suprofen, Vedaprofen, Zaltoprofen, Miroprofen, Oxaprozin und Mischungen davon, weiter bevorzugt Naproxen, Ibuprofen und Mischungen davon, besteht, ausgewählt.

Die vorstehend aufgeführten organischen Verbindungen sind teilweise in Form ihres internationalen Freinamens (INN, "International Nonproprietary Name") angegeben, der von der Weltgesundheitsorganisation vergeben wird und den gemeinfreien Namen für einen Arzneimittelwirkstoff bezeichnet.

Geeignete Pestizide sind beispielsweise Herbizide, Fungizide, pflanzliche Wachstumsregulatoren, Insektizide, Nematizide, Akarizide oder Mischungen davon, die jeweils wenigstens 1 der vorgenannten Säuregruppen-haltigen Reste, vorzugsweise 1 bis 6 Säuregruppen-haltige Reste, aufweisen, der/die weiter bevorzugt jeweils unabhängig voneinander aus der Gruppe, die aus Carboxygruppe, Sulfonsäuregruppe, Phosphonsäuregruppe und Kombinationen davon besteht, ausgewählt werden.

Geeignete Pestizide mit sauren Gruppen sind beispielsweise Abscisinsäure, 5-[2-Chlor-4-(trifluormethyl)phenoxy]-2-nitrobenzoesäure (Acifluorfen), 6-Amino-5-chlor-2-cyclopropylpyrimidin-4-carbonsäure (Aminocyclopyrachlor), 4-Chlor-2-oxo-3(2H)-benzothiazolessigsäure (Benazolin), α-[(4,6-Dimethoxypyrimidin-2-ylcarbamoyl)sulfamoyl]-o-toluensäure (Bensulfuron), Indol-3-essigsäure, 4-(Indol-3-yl)buttersäure, 3-Amino-2,5-dichlorbenzoesäure (Chloramben), 3,6-Dichlor-2-methoxybenzoesäure (Dicamba), 2,3,6-Trichlorbenzoesäure (2,3,6-TBA), 2,3,5-Trichlor-6-methoxybenzoesäure (Tricamba), 2,6-bis(4,6-Dimethoxypyrimidin-2-yloxy)benzoesäure (Bispyribac), 2-Chlor-6-(4,6-dimethoxypyrimidin-2-ylthio)benzoesäure (Pyrithiobac), Tetrachlorterephthalsäure (Chlorthal), 4-Amino-3,6-dichlorpicolinsäure (Aminopyralid), 3,6-Dichlorpicolinsäure (Clopyralid), 4-Amino-3-chlor-6-(4-chlor-2-fluor-3-methoxyphenyl)-5-fluorpicolinsäure (Florpyrauxifen), 4-Amino-3-chlor-6-(4-chlor-2-fluor-3- methoxyphenyl)picolinsäure (Halauxifen), 4-Amino-3,5,6-trichlorpicolinsäure (Picloram), 2-Methyl-4-chlorphenoxyessigsäure (MCPA), 4-(4-Chlorphenoxy)buttersäure (4-CPB), 4-(2,4-Dichlorphenoxy)buttersäure (2,4-DB), 4-(2,4,5-Trichlorphenoxy)buttersäure (2,4,5-TB), 4-(3,4-Dichlorphenoxy)buttersäure (3,4-DB), 4-(4-Chlor-2-methylphenoxy)buttersäure (MCPB), 2-(4-Chlorphenyl)-3-ethyl-2,5-dihydro-5-oxopyridazin-4-carbonsäure (Clofencet), 2-(3-Chlorphenoxy)propionsäure (Cloprop), 2-(4-Chlorphenoxy)propionsäure (4-CPP), 2-(2,4-Dichlorphenoxy)propionsäure (Dichlorprop), 2-(3,4-Dichlorphenoxy)propionsäure (3,4-DP), 2-(2,4,5-Trichlorphenoxy)propionsäure (Fenoprop), 2-(4-Chlor-2-methylphenoxy)propionsäure (Mecoprop), 2-[4-[(3,5-Dichlor-2-pyridinyl)oxy]phenoxy]propionsäure (Chlorazifop), 2-[4-[(5-Chlor-3-fluor-2-pyridinyl)oxy]phenoxy]propionsäure, 2-[4-(4-Chlorphenoxy)phenoxy]propionsäure (Clofop), 2-[4-(4-Cyano-2-fluorphenoxy)phenoxy]propionsäure, 2-[4-(2,4-Dichlorphenoxy)phenoxy]propionsäure (Diclofop), 2-[4-(6-Chlor-1,3-benzoxazol-2-yloxy)phenoxy]propionsäure (Fenoxaprop), 2-[4-(6-Chlor-1,3-benzothiazol-2-yloxy)phenoxy]propionsäure (Fenthiaprop), 2-{4-[5-(Trifluormethyl)-2-pyridyloxy]phenoxy}propionsäure (Fluazifop), 2-{4-[3-Chlor-5-(trifluormethyl)-2-pyridyloxy]phenoxy}propionsäure (Haloxyfop), 2-[4-(6-Chlorquinoxalin-2-yloxy)phenoxy]propionsäure (Quizalofop), 2-[4-(α,α,α-Trifluor-p-tolyloxy)phenoxy]propionsäure (Trifop), 4-Chlor-α-hydroxy-o-tolyloxyessigsäure (Cloxyfonac), 1-(2,4-Dichloranilinocarbonyl)cyclopropancarbonsäure (Cyclanilid), [2-Chlor-5-(cyclohex-1-ene-1,2-dicarboximido)-4-fluorrophenoxy]essigsäure (Flumiclorac), 4-Amino-3,5-dichlor-6-fluor-2-pyridyloxyessigsäure (Fluroxypyr), 2-[(4,6-Dimethoxypyrimidin-2-ylcarbamoyl)sulfamoyl]-6-(trifluormethyl)nicotinsäure (Flupyrsulfuron), 2-[4,5-Dihydro-4-methyl-4-(1-methylethyl)-5-oxo-1H-imidazol-2-yl]-5-(methoxymethyl)-3-pyridincarbonsäure (Imazamox), 2-[4,5-Dihydro-4-methyl-4-(1-methylethyl)-5-oxo-1H-imidazol-2-yl]-5-methyl-3-pyridincarbonsäure (Imazapic), 2-[4,5-Dihydro-4-methyl-4-(1-methylethyl)-5-oxo-1H-imidazol-2-yl]-3-pyridincarbonsäure (Imazapyr), 2-[4,5-Dihydro-4-methyl-4-(1-methylethyl)-5-oxo-1H-imidazol-2-yl]-3-quinolincarbonsäure (Imazaquin), 2-[4,5-Dihydro-4-methyl-4-(1-methylethyl)-5-oxo-1H-imidazol-2-yl]-5-ethyl-3-pyridincarbonsäure (Imazethapyr), 2-(4-Chlorphenyl)-1-ethyl-1,4-dihydro-6-methyl-4-oxonicotinisäure (Karetazan), N-(1-Naphthyl)phthalsäuremonoamid (Naptalam), 2-[(4-Methoxy-6-methyl-1,3,5-triazin-2-yl)carbamoylsulfamoyl]benzoesäure (Metsulfuron), Oxolinsäure, Pelargonsäure, 3,5-Dioxo-4-propionyl-cyclohexancarbonsäure, 3,7-Dichlorquinolin-8-carbonsäure (Quinclorac), 7-Chlor-3-methylquinolin-8-carbonsäure (Quinmerac), 2',3,3',4,5,6-Hexachlorphthalanilinsäure (Tecloftalam), Tolylphthalaminsäure, 2-[4-Methoxy-6-methyl-1,3,5-triazin-2-yl(methyl)carbamoylsulfamoyl]benzoesäure (Tribenuron), 2,4,5-Trichlorphenoxyessigsäure (2,4,5-T), 3,5,6-Trichlor-2-pyridyloxyessigsäure (Triclopyr) oder Mischungen davon.

Vorzugsweise werden geeignete Säurefarbstoffe auch als anionische Farbstoffe bezeichnet.

Geeignete Säurefarbstoffe sind vorzugsweise Anthrachinonfarbstoffen, Azofarbstoffen, Triphenylmethanfarbstoffen, Xanthenfarbstoffen oder Mischungen davon, die jeweils wenigstens 1 der vorgenannten Säuregruppen-haltigen Reste, vorzugsweise 1 bis 6 Säuregruppen-haltige Reste, aufweisen, der/die weiter bevorzugt jeweils unabhängig voneinander aus der Gruppe, die aus Carboxygruppe, Sulfonsäuregruppe, Phosphonsäuregruppe und Kombinationen davon, vorzugsweise Carboxygruppe, Sulfonsäuregruppe und Kombinationen davon, vorzugsweise Carboxygruppe, besteht, ausgewählt werden.

Geeignete Säurefarbstoffe aus der Gruppe der Xanthenfarbstoffe sind beispielsweise Fluorescein, 6-Carboxyfluorescein, 2,7-Dichlorfluorescein, 2,4,5,7-Tetrabromfluorescein, 2,4,5,7-Tetraiodfluorescein, 2,7-Dinitro-4,5-dibromfluorescein, 4,5,6,7-Tetrachlor-2',4',5',7'-tetraiodfluorescein, 4',5'-Bis(1,3,2-dithioarsolan-2-yl)fluorescein oder Mischungen davon.

Geeignete Säurefarbstoffe aus der Gruppe der Anthrachinonfarbstoffe sind beispielsweise 1,4-Dihydroxyanthrachinon-2-sulfonsäure, Karminsäure, 4,5-Dihydroxyanthrachinon-2-carbonsäure, oder Mischungen davon.

Geeignete Säurefarbstoffe aus der Gruppe der Azofarbstoffe sind beispielsweise 3-Hydroxy-4-(2-hydroxy-4-sulfo-1-naphthylazo)-2-naphthalencarbonsäure (Calconcarbonsäure), 1-(2-Hydroxy-1-naphthylazo)-2-naphthol-4-sulfonsäure (Eriochromblauschwarz R), (3E)-5-Hydroxy-3-[(4-nitrophenyl)hydrazinyliden]-4-oxonaphthalen-2,7-disulfonsäure (Chromotrop 2B), (3E)-5-Hydroxy-4-oxo-3-(phenylhydrazinyliden)naphthalen-2,7-disulfonsäure (Chromotrope 2R), (3Z)-3-[(4-Acetamidophenyl)hydrazinyliden]-5-hydroxy-4-oxonaphthalen-2,7-disulfonsäure (Chromotrop 6B), (3Z)-5-Hydroxy-3-(naphthalen-1-ylhydrazinyliden)-4-oxonaphthalen-2,7-disulfonsäure (Chromotrop 10B), 5-[(p-Nitrophenyl)azo]salicylsäure (Alizaringelb R), 2-[(2Z)-2-(2-Oxonaphthalin-1-yliden)hydrazinyl]-5-(4-sulfophenyl)diazenylbenzolsulfonsäure (Biebricher Scharlach), 2-Hydroxy-1-(1-hydroxynaphthyl-2-azo)-6-nitronaphthalin-4-sulfonsäure, 6-Hydroxy-5-(4-sulfophenylazo)-naphthalin-2-sulfonsäure (Eriochromschwarz T), 1-Phenylazo-2-naphthol-6,8-disulfonsäure (Acid Orange 10), 4-(2-Hydroxy-1-naphthyl-azo)-benzolsulfonsäure (Acid Orange 7), 4-[(2,4-Dihydroxyphenyl)diazenyl]benzolsulfosäure, (3Z)-5-Acetamido-4-oxo-3-(phenylhydrazinyliden)naphthalin-2,7-disulfonsäure (Acid Red 1), (4Z)-4-[(2,4-Dimethylphenyl)hydrazinyliden]-3-oxonaphthalen-2,7-disulfonsäure (Xylidin-Ponceau), 7-Hydroxy-8-((4-sulfo-1-naphthalenyl)-azo)-1,3-naphthalendisulfonsäure (Cochenillerot A), (4E)-4-[(4-Methyl-2-sulfonatophenyl)hydrazinyliden]-3-oxonaphthalen-2-carbonsäure (Litholrubin BK), (8Z)-8-(Naphthalen-1-ylhydrazinyliden)-7-oxonaphthalen-1,3-disulfonsäure (Ponceau 6R), (3E)-4-Oxo-3-[(4-sulfonatonaphthalen-1-yl)hydrazinyliden]naphthalen-1-sulfonsäure (Azorubin) oder Mischungen davon.

Chiralitätszentren können, wenn nicht anders angegeben, in der R- oder in der S-Konfiguration vorliegen. Die Erfindung betrifft sowohl die Verwendung von optisch reinen Verbindungen, als auch Stereoisomeren-Gemische, wie Enantiomeren-Gemische und Diastereomeren-Gemische, in jedem Verhältnis.

Beispielsweise ist Flurbiprofen (INN) ein, vorzugsweise 1:1, Gemisch (Racemat) der (R)-Form und der (S)-Form von 2-(2-Fluor-4-biphenylyl)propionsäure. Tarenflurbil (INN) bezeichnet das (R)-Enantiomer und Esflurbiprofen (INN) das (S)-Enantiomer von 2-(2-Fluor-4-biphenylyl)propionsäure.

Beispielsweise ist Ibuprofen (INN) ein, vorzugsweise 1:1, Gemisch der (R)-Form und der (S)-Form von 2-(4-Isobutylphenyl)propionsäure. Dexibuprofen (INN) bezeichnet das (S)-Enantiomer von 2-(4-Isobutylphenyl)propionsäure.

Beispielsweise bezeichnet Naproxen (INN) das (S)-Enantiomer von 2-(6-Methoxy-2-naphthyl)propionsäure.

Eine erfindungsgemäße Suspension umfasst weiterhin ein wasserlösliches Salz eines Übergangsmetalls.

Vorzugsweise umfasst das wasserlösliche Salz eines Übergangsmetalls Anionen, die aus der Gruppe, die aus Halogenid, Nitrat, Sulfat, Hydrogensulfat, Carbonat, Hydrogencarbonat und Mischungen davon, vorzugsweise Nitrat, Halogenid und Mischungen davon, weiter bevorzugt Halogenid, besteht, ausgewählt werden.

Unter dem Begriff "Halogenid" werden vorzugsweise Chlorid, Bromid, lodid, Fluorid oder Mischungen davon, weiter bevorzugt Chlorid, verstanden.

Weiter bevorzugt umfasst das wasserlösliche Salz eines Übergangsmetalls Anionen, die aus der Gruppe, die aus Chlorid, Bromid, lodid, Fluorid und Mischungen davon, weiter bevorzugt Chlorid, Bromid und Mischungen davon, weiter bevorzugt Chlorid, besteht, ausgewählt werden.

Weiter bevorzugt umfasst das wasserlösliche Salz eines Übergangsmetalls Übergangsmetall-Kationen, die eine formale Oxidationszahl aus einem Bereich von +1 bis +6, vorzugsweise von +2 bis +5, weiter bevorzugt +3 bis +4, auf, wobei vorzugsweise vorgenannte Übergangsmetall-Kationen beispielsweise auch als, vorzugsweise positiv geladener, Hydroxid-Komplex vorliegen kann.

Weiter bevorzugt wird das Übergangsmetall aus der Gruppe, die aus Titan, Zirkonium, Hafnium, Cer, Yttrium und Kombinationen davon, weiter bevorzugt Titan, Zirkonium, Hafnium und Kombinationen davon, weiter bevorzugt Zirkonium, Hafnium und Kombinationen davon, besteht, ausgewählt.

Bei einer weiter bevorzugten Ausführungsform umfasst oder besteht das wenigstens eine wasserlösliche Salz eines Übergangsmetalls aus Kationen, die eine formale Oxidationszahl aus einem Bereich von +1 bis +6, vorzugsweise von +2 bis +5, weiter bevorzugt +3 bis +4, weiter bevorzugt von +4, aufweisen und aus der Gruppe, die aus Titan, Zirkonium, Hafnium, Cer, Yttrium und Kombinationen davon, weiter bevorzugt Titan, Zirkonium, Hafnium und Kombinationen davon, weiter bevorzugt Zirkonium, Hafnium und Kombinationen davon, besteht, ausgewählt werden, sowie Anionen, die aus der Gruppe, die aus Halogeniden, die vorzugsweise Chlorid, Bromid, Fluorid, oder Mischungen davon, weiter bevorzugt Chlorid, sind, Nitraten, Sulfaten, Hydrogensulfaten, Carbonaten, Hydrogenkarbonaten, und Mischungen davon, vorzugsweise Halogeniden, die vorzugsweise Chlorid, Bromid, Fluorid, oder Mischungen davon, weiter bevorzugt Chlorid, sind, und Mischungen davon, besteht, ausgewählt werden.

In einer weiter bevorzugten Ausführungsform ist das wenigstens eine wasserlösliche Salz eines Übergangsmetalls wenigstens ein Halogenid, wenigstens ein Oxyhalogenid, Hydrate davon oder eine Mischung davon, weiter bevorzugt wenigstens ein Halogenid, Hydrate davon oder eine Mischung davon, das Übergangsmetallkationen, die aus der Gruppe, die aus Zirkonium, Hafnium und Mischungen davon besteht, ausgewählt werden, mit einer formalen Oxidationszahl aus einem Bereich von +1 bis +4, weiter bevorzugt +3 bis +4, weiter bevorzugt von +4, aufweist und Kationen, die aus der Gruppe, die aus Chlorid, Bromid, lodid, Fluorid und Mischungen davon, weiter bevorzugt Chlorid, besteht, ausgewählt werden.

Weiter bevorzugt wird ein geeignetes wasserlösliches Salz eines Übergangsmetalls aus der Gruppe, die aus ZrO(NO₃)₂, HfCl₄, HfOCl₂, HfBr₄, HfOBr₂, HfI₄, HfOI₂, ZrCl₄, ZrOCl₂, ZrBr₄, ZrOBr₂, ZrI₄, ZrOI₂, Hydraten davon und Mischungen davon, vorzugsweise HfCl₄, HfOCl₂, HfBr₄, HfOBr₂, HfI₄, HfOI₂, ZrCl₄, ZrOCl₂, ZrBr₄, ZrOBr₂, ZrI₄, ZrOI₂, Hydraten davon und Mischungen davon, weiter bevorzugt HfCl₄, HfBr₄, HfI₄, ZrCl₄, ZrBr₄, ZrI₄, Hydraten davon und Mischungen davon, weiter bevorzugt aus HfCl₄, ZrCl₄, ZrOCl₂, Hydraten davon und Mischungen davon, besteht, ausgewählt.

Bei einer weiter bevorzugten Ausführungsform liegt das wenigstens eine wasserlösliche Salz eines Übergangsmetalls und die wenigstens eine organische Verbindung in einem molaren Verhältnis aus einem Bereich von 100:1 bis 1:100, vorzugsweise aus einem Bereich von 20:1 bis 1:20, vorzugsweise aus einem Bereich von 15:1 bis 1:15, vorzugsweise aus einem Bereich von 10:1 bis 1:10, vorzugsweise aus einem Bereich von 8:1 bis 1:8, vorzugsweise aus einem Bereich von 6,5:1 bis 1:6,5, vorzugsweise aus einem Bereich von 5:1 bis 1:5, vorzugsweise aus einem Bereich von 4:1 bis 1:4, weiter bevorzugt aus einem Bereich von 2:1 bis 1:2, vor.

Die Erfinder haben festgestellt, dass sich die Größe, vorzugsweise die mittlere Partikelgröße bzw. die mittlere Primärpartikelgröße, der in einer erfindungsgemäßen Suspension bzw. in einer erfindungsgemäßen trockenen Zusammensetzung enthaltenen organischen Partikel und vorzugsweise ebenfalls deren jeweilige Partikelgrößenverteilung, vorzugsweise die Breite der jeweiligen Partikelgrößenverteilung, durch Variation des molaren Verhältnisses des wenigstens einem wasserlöslichen Salzes eines Übergangsmetalls zu der wenigstens einen organischen Verbindung in den oben angegebenen Bereichen, einstellen lässt.

Vorzugsweise kann die Breite der Partikelgrößenverteilung durch Ermittlung der Breite der Verteilungsfunktion Δx ("Spanne") abgeleitet werden, die aus der folgenden Formel (1) berechnet werden kann: $Δ x = \left({x}_{90 , r}-{x}_{10 , r}\right) / {x}_{50 , r}$ wobei der x_{90,r}-, x_{50,r}- und x_{10,r}-Wert, den jeweiligen Durchmesser bedeutet, den jeweils 90%, 50%, bzw. 10% der untersuchten Partikel aufweisen oder unterschreiten, wobei die jeweils bestimmte Mengenart vorzugsweise durch den Index r gekennzeichnet ist. Beispielsweise bedeutet r = 0, dass die Anzahl der Teilchen bestimmt wurde. Beispielsweise bedeutet r = 3, dass das Volumen der Partikel bestimmt wurde.

Weiter bevorzugt kann der volumenbezogene Äquivalentdurchmesser der untersuchten Partikel bestimmt werden, wobei vorzugsweise die Breite der ermittelten Partikelgrößenverteilung durch Ermittlung der Breite der Verteilungsfunktion Δx abgeleitet werden kann, die aus der folgenden Formel (1a) berechnet werden kann: $Δ x = \left({x}_{90,3}-{x}_{10,3}\right) / {x}_{50,3}$ wobei der x_{90,3}-, x_{50,3}- und x_{10,3}-Wert, den jeweiligen Durchmesser bedeutet, den jeweils 90%, 50%, bzw. 10% der untersuchten Partikel aufweisen oder unterschreiten, wobei durch den Index 3 gekennzeichnet ist, dass jeweils der volumenbezogene Äquivalentdurchmesser der untersuchten Partikel bestimmt wurde.

Beispielsweise bedeutet ein x_{90,3} von 350 nm, dass 90 % der Partikel einen volumenbezogenen Äquivalentdurchmesser von kleiner oder gleich 350 nm aufweisen.

Die Parameter x_{90,3} bzw. x_{50,3} bzw. x_{10,3} der gemessenen Partikelgrößenverteilung können im Folgenden auch als x90,3 bzw. x50,3 bzw. x10,3 bezeichnet werden.

Vorzugsweise bedeutet ein kleiner Span, dass die die jeweilige Partikelgrößenverteilung eng ist.

Vorzugsweise liegt der Span in einem Bereich von, 0,3 bis 1,9, vorzugsweise 0,45 bis 1,7, weiter bevorzugt von 0,5 bis 1,5, weiter bevorzugt von 0,7 bis 1,3.

Vorzugsweise wird durch Verwendung wenigstens eines wasserlöslichen Salzes eines Übergangsmetalls in dem oben angegebenen molaren Verhältnis in Bezug auf die in der wässrigen Suspension enthaltene wenigstens eine organische Verbindung weiterhin die Oberfläche der nanoskaligen, organischen Partikel, die die wenigstens eine organische Verbindung enthalten, zumindest teilweise, vorzugsweise vollständig, mit Übergangsmetall-Kationen und/oder, vorzugsweise positiv geladene, Hydroxid-Komplexen davon bedeckt.

Vorzugsweise wird durch eine Anordnung, vorzugsweise Bindung, von Übergangsmetall-Kationen, die eine formale Oxidationszahl aus einem Bereich von +1 bis +6, vorzugsweise von +2 bis +5, weiter bevorzugt von +3 bis +4, weiter bevorzugt von +4, aufweisen, und/oder, vorzugsweise positiv geladenen, Hydroxid-Komplexen davon auf der Oberfläche der in einer erfindungsgemäßen Suspension enthaltenen nanoskaligen, organischen Partikel die Stabilität der Suspension erhöht.

Eine Anordnung, vorzugsweise Bindung, von Übergangsmetall-Kationen und/oder, vorzugsweise positiv geladenen, Hydroxid-Komplexen davon auf der Oberfläche der nanoskaligen, organischen Partikel kann beispielsweise durch elektrostatische Wechselwirkung, beispielsweise lonenbindung, und/oder kovalente Bindung erfolgen. Durch Anordnung, vorzugsweise Bindung, von positiv geladenen Übergangsmetall-Kationen, die vorzugsweise eine formale Oxidationszahl aus einem Bereich von +1 bis +6, vorzugsweise von +2 bis +5, weiter bevorzugt von +3 bis +4, weiter bevorzugt von +4, aufweisen, und/oder, vorzugsweise positiv geladenen, Hydroxid-Komplexen davon, die vorzuzugsweise eine Ladung von +1 oder mehr, vorzugsweise von +3 oder mehr, vorzugsweise aus einem Bereich von +3 bis +10, vorzugsweise von +4 bis +8, weiter bevorzugt von +8, aufweisen, an der Oberfläche der nanoskaligen, organischen Partikel, wird vorzugsweise die, weiter bevorzugt positive, Ladungsdichte, der Partikel erhöht.

Durch eine Erhöhung der, weiter bevorzugt positiven, Ladungsdichte der Oberfläche der nanoskaligen, organischen Partikel kommt es vorzugsweise zu keiner Agglomeration der Partikel. Weiter bevorzugt stoßen sich die nanoskaligen, organischen Partikel ab, so dass beispielsweise das Entstehen von Agglomeraten und dadurch vorzugsweise eine Sedimentation der Partikel in einer erfindungsgemäßen Suspension, verhindert wird.

Vorzugsweise bilden Übergangsmetall-Kationen, vorzugsweise Zr⁴⁺, Hf⁴⁺ oder Mischungen davon, des wenigstens einen wasserlöslichen Salzes eines Übergangsmetalls in Wasser einen oder mehrere, vorzugsweise positiv geladene(n), Hydroxid-Komplex(e), der/die beispielsweise die Formel [Zr₃(OH)₄]⁸⁺, [Zr₄(OH)₈]⁸⁺ oder Mischungen davon aufweist.

Beispielsweise können der wenigstens eine Säuregruppen-haltige Rest und/oder andere funktionelle Gruppen der vorgenannten wenigstens einen organischen Verbindung, die vorzugsweise an der Oberfläche der nanoskaligen, organischen Partikel angeordnet sind, mit einem, vorzugsweise positiv geladenen, Hydroxid-Komplex interagieren, vorzugsweise reagieren, weiter bevorzugt kondensieren, und/oder elektrostatisch binden.

Durch Anordnung, vorzugsweise Bindung, eines, vorzugsweise positiv geladenen, Hydroxid-Komplexes an die Oberfläche der vorgenannten nanoskaligen, organischen Partikel kommt es vorzugsweise zu einer Erhöhung der positiven Ladungsdichte auf der Oberfläche der vorgenannten nanoskaligen, organischen Partikel.

Weiter bevorzugt sind die auf der Oberfläche der in einer erfindungsgemäßen Suspension bzw. in einer erfindungsgemäßen trockenen Zusammensetzung enthaltenen nanoskaligen, organischen Partikel angeordneten, vorzugsweise gebundenen, Übergangsmetall-Kationen und/oder, vorzugsweise positiv geladenen, Hydroxid-Komplexen davon fest mit der Oberfläche verbunden und können beispielsweise nicht durch Waschen und/oder Verdünnen mit einem wässrigen Lösungsmittel, vorzugsweise Wasser, von der Oberfläche abgelöst werden.

Vorzugsweise liegen in einer erfindungsgemäßen Suspension bzw. in einer erfindungsgemäßen trockenen Zusammensetzung die nanoskaligen, organischen Partikel im Wesentlichen, vorzugsweise vollständig, als Einzel-Partikel vor.

Das Vorliegen von Übergangsmetall-Kationen und/oder, vorzugsweise positiv geladenen, Hydroxid-Komplexen davon auf der Oberfläche der in einer erfindungsgemäßen Suspension bzw. in einer erfindungsgemäßen trockenen Zusammensetzung enthaltenen nanoskaligen, organischen Partikel kann beispielsweise spektroskopisch an isolierten nanoskaligen, organischen Partikel bestimmt werden.

Eine geeignete Methode ist beispielsweise die Fourier-Transformations-Infrarot (FTIR) Spektroskopie, wobei vorzugsweise in einer erfindungsgemäßen Suspension bzw. in einer erfindungsgemäßen trockenen Zusammensetzung enthaltenen nanoskaligen, organischen Partikel zunächst isoliert, beispielsweise durch Zentrifugation, und getrocknet werden.

Beispielsweise werden isolierte und getrocknete nanoskalige, organische Partikel nachfolgend in Kaliumbromid (KBr) Presslinge eingearbeitet und daran die FTIR-Spektren gemessen.

Bei einer weiter bevorzugten Ausführungsform weist die erfindungsgemäße Suspension das wasserlösliche Salz eines Übergangsmetalls in einer Konzentration aus einem Bereich von 0,001 mol/l bis 1,5 mol/l, weiter bevorzugt aus einem Bereich von 0,0025 mol/l bis 0,75 mol/l, weiter bevorzugt aus einem Bereich von 0,005 mol/l bis 0,5 mol/l, weiter bevorzugt aus einem Bereich von 0,0075 mol/l bis 0,35 mol/l, weiter bevorzugt aus einem Bereich von 0,009 mol/l bis 0,29 mol/l, weiter bevorzugt aus einem Bereich von 0,01 mol/l bis 0,20 mol/l, jeweils bezogen auf das Gesamtvolumen der Suspension, auf.

Vorzugsweise enthält eine erfindungsgemäße Suspension bzw. eine erfindungsgemäße trockene Zusammensetzung im Wesentlichen stabilisierte nanoskalige, organische Einzel-Partikel, wobei der Anteil der Einzel-Partikel wenigstens 70%, weiter bevorzugt wenigstens 85%, jeweils bezogen auf die Gesamtzahl der in der Suspension bzw. Zusammensetzung enthaltenen nanoskaligen, organischen Partikel, beträgt.

Das Vorhandensein von Einzel-Partikel kann beispielsweise durch Verwendung eines SMPS-Spektrometers (Scanning Mobility Particle Sizer - Spektrometer) bestimmt werden, beispielsweise unter Verwendung von kommerziell erhältlichen SMPS-Spektrometern der Firma TSI GmbH (Aachen, DE).

Ein SMPS-Spektrometer erlaubt eine Bestimmung des Mobilitätsdurchmessers der nanoskaligen, organischen Partikel, vorzugsweise unabhängig vom Berechnungsindex der jeweiligen Partikel oder der erfindungsgemäßen Suspension bzw. der erfindungsgemäßen Zusammensetzung. Die Größenbestimmung erfolgt vorzugsweise in Analogie zu der in ISO 15900 ("Determination of particle size distribution - Differential electrical mobility analysis for aerosol particles", Ausgabedatum: 2009-05) beschriebenen Methode, vorzugsweise bei einer Temperatur von 20°C, einem Druck von 1013 mbar und einer Wellenlänge von 470 nm.

Die zu messende Probe der Suspension wird vorzugsweise zunächst in ein Aerosol überführt, beispielsweise durch Versprühen und/oder Verdüsen der Probe.

Ein SMPS-Spektrometer verwendet vorzugsweise einen differenziellen Mobilitätsanalysator (DMA, Differential Mobility Analyzer), in dem eine, vorzugsweise enge, Mobilitätsfraktion aus dem Gesamtaerosol herausgegriffen werden kann. Eine Mobilitätsfraktion weist vorzugsweise eine im Wesentlichen ähnliche Mobilität der Partikel im angelegten elektrischen Feld auf und kann daher jeweils als "monomobile Fraktion" bezeichnet werden. Die Mobilität der Partikel im jeweils angelegten elektrischen Feld kann einem Teilchendurchmesser zugeordnet werden. Durch Variation der angelegten Spannung können Partikel, die einen unterschiedlichen Durchmesser aufweisen, ausgewählt werden. Die in der jeweiligen monomobilen Fraktion enthaltenen Partikel werden vorzugsweise jeweils mittels eines Kondensationskernzählers quantifiziert.

Weiter bevorzugt sind Hydroxid-Komplexe der vorgenannten Übergangsmetall-Kationen mehrfach positiv geladen. Dadurch kommt es nach Anordnung, vorzugsweise Bindung, von Hydroxid-Komplexen, beispielsweise der Formel [Zr₃(OH)₄]⁸⁺, [Zr₄(OH)₈]⁸⁺ oder Mischungen davon, an die Oberfläche der vorgenannten nanoskaligen, organischen Partikel vorzugsweise zu einer Erhöhung des, vorzugsweise positiven, Zeta-Potentials.

Eine Erhöhung des, vorzugsweise positiven, Zeta-Potentials bewirkt eine Verstärkung der interpartikulären Abstoßungskräfte, so dass Agglomerationen verringert, vorzugsweise verhindert werden.

Das Zeta-Potential ist vorzugsweise das elektrische Potential an einer Abscherschicht eines bewegten nanoskaligen, organischen Partikels in einer bei Standardbedingungen (Druck: 1013 mbar, Temperatur: 25°C) flüssigen Phase, vorzugsweise wässrigen Phase, einer Suspension.

Befinden sich beispielsweise geladene Partikel in einer Suspension, so lagern sich auf der Partikeloberfläche Ionen des Suspensionsmediums an, die vorzugsweise eine zum geladenen Partikel entgegengesetzte Ladung aufweisen.

Eine Ausbildung einer elektrischen Ladung an Grenzflächen kann beispielsweise durch Reibung oder thermische Bewegung der Partikel im Suspensionsmedium erfolgen. Zu dieser elektrischen Ladung entgegengesetzte Ionen werden an die Grenzfläche angezogen und es entsteht vorzugsweise eine elektrische Doppelschicht. Weitere Ionen können angelagert werden und weisen eine lockere Anordnung auf, die in die flüssige Phase, vorzugsweise wässrige Phase, der Suspension hineinreicht. Vorzugsweise liegt eine diffuse Verteilung positiver und negativer Ionen vor, wobei vorzugsweise die Gesamtladung ausgeglichen ist.

Beispielsweise kann das Zeta-Potential nach im Stand der Technik bekannten Methoden bestimmt werden. Geeignete Methoden werden beispielsweise in der ISO 13099-1 ("Colloidal systems -- Methods for zeta-potential determination -- Part 1: Electroacoustic and electrokinetic phenomena"; Ausgabedatum: 2012-06), der ISO 13099-2 ("Colloidal systemsMethods for zeta-potential determination -- Part 2: Optical methods"; Ausgabedatum: 2012-06) oder der ISO 13099-3 ("Colloidal systems -- Methods for zeta potential determination-Part 3: Acoustic methods"; Ausgabedatum: 2014-07) beschrieben.

Vorzugsweise wird das Zeta-Potential der in einer erfindungsgemäßen Suspension enthaltenen nanoskaligen, organischen Partikel gemäß der in ISO 13099-2:2012-06 beschriebenen Verfahren, vorzugsweise durch Messung der elektrophoretischen Mobilität der zu messsenden Partikel in einem elektrischen Feld, vorzugsweise unter Verwendung der Laser-Doppler-Anemometrie (Laser-Doppler-Geschwindigkeitsmessung), bestimmt.

Vorzugsweise kann das Zeta-Potential von in einer erfindungsgemäßen trockenen Zusammensetzung enthaltenen nanoskaligen, organischen Partikeln nach Einbringen der Zusammensetzung in ein wässriges Lösungsmittel, beispielsweise Wasser, gemäß der in ISO 13099-2:2012-06 beschriebenen Verfahren, vorzugsweise durch Messung der elektrophoretischen Mobilität der zu messsenden Partikel in einem elektrischen Feld, vorzugsweise unter Verwendung der Laser-Doppler-Anemometrie (LDA), bestimmt werden.

Geeignete Messgeräte zur Bestimmung des Zeta-Potentials sind im Stand der Technik bekannt, wobei beispielsweise ein Zetasizer^{®} Nano, beispielsweise der Typen ZSP, ZS, Z oder ZS90, der Malvern Instruments Limited (Malvern, Worcestershire, UK,), verwendet werden kann.

Weiter bevorzugt weisen die in einer erfindungsgemäßen Suspension bzw. in einer erfindungsgemäßen trockenen Zusammensetzung enthaltenen nanoskaligen, organischen Partikel eine Zeta-Potential, vorzugsweise gemessen gemäß ISO 13099-2:2012-06 in Wasser bei Standardbedingungen (Temperatur: 25°C, Druck: 1013 mbar) und einem pH-Wert aus einem Bereich von 1 bis 4, weiter bevorzugt von 2, von mindestens + 20 mV, vorzugsweise von mindestens + 27 mV, weiter bevorzugt von mindestens + 33 mV, vorzugsweise aus einem Bereich von + 20 mV bis + 101 mV, vorzugsweise aus einem Bereich von + 25 mV bis + 95 mV, vorzugsweise aus einem Bereich von + 30 mV bis + 83 mV, vorzugsweise aus einem Bereich von + 33 mV bis + 75 mV, vorzugsweise aus einem Bereich von + 37 mV bis + 65 mV, vorzugsweise aus einem Bereich von + 41 mV bis + 55 mV, auf.

Bei einer weiter bevorzugten Ausführungsform sind die in einer erfindungsgemäßen Suspension bzw. in einer erfindungsgemäßen trockenen Zusammensetzung enthaltenen nanoskaligen, organischen Partikel keine Lipid-basierten Cochleate, keine Lipid Nanopartikel, keine nanostrukturierten Lipid-Carrier, keine Lipid-Arzneistoff-Konjugat-Nanopartikel oder Kombinationen davon.

Vorzugsweise umfassen oder bestehen die in einer erfindungsgemäßen Suspension bzw. in einer erfindungsgemäßen trockenen Zusammensetzung enthaltenen nanoskaligen, organischen Partikel im Wesentlichen aus wenigstens einer der vorgenannten organischen Verbindungen mit wenigstens 6 C-Atomen und wenigstens einem Säuregruppen-haltigen Rest und weiterhin aus Übergangsmetall-Kationen, die vorzugsweise aus der Gruppe, die aus Titan, Zirkonium, Hafnium und Kombinationen davon, weiter bevorzugt Zirkonium, Hafnium und Kombinationen davon, weiter bevorzugt Zirkonium, besteht, ausgewählt werden, und/oder, vorzugsweise positiv geladene, Hydroxid-Komplexe davon, wobei zumindest auf der Oberfläche der nanoskaligen, organischen Partikel wenigstens teilweise, vorzugsweise vollständig Übergangsmetall-Kationen und/oder, vorzugsweise positiv geladene, Hydroxid-Komplexe davon angeordnet, vorzugsweise gebunden, sind.

Vorzugsweise können vorgenannte Übergangsmetall-Kationen und/oder, vorzugsweise positiv geladene, Hydroxid-Komplexe davon ebenfalls innerhalb der nanoskaligen, organischen Partikel angeordnet, vorzugsweise gebunden, sein. Beispielsweise können vorgenannte Übergangsmetall-Kationen und/oder, vorzugsweise positiv geladene, Hydroxid-Komplexe davon an deprotonierte Säuregruppen-haltige Reste der vorgenannten organischen Verbindungen mit wenigstens 6 C-Atomen gebunden, beispielsweise elektrostatisch und/oder kovalent gebunden, vorliegen.

Vorzugsweise umfassen oder bestehen die in einer erfindungsgemäßen Suspension bzw. in einer erfindungsgemäßen trockenen Zusammensetzung enthaltenen nanoskaligen, organischen Partikel mindestens 65 Gew.-%, vorzugsweise mindestens aus 75 Gew.-%, weiter bevorzugt mindestens aus 80 Gew.-%, weiter bevorzugt mindestens aus 85 Gew.-%, weiter bevorzugt mindestens aus 89 Gew.-%, jeweils bezogen auf das Gesamtgewicht der organischen Partikel, aus der wenigstens einen organischen Verbindung mit wenigstens 6 C-Atomen und wenigstens einem Säuregruppen-haltigen Rest.

Weiter bevorzugt umfassen die in einer erfindungsgemäßen Suspension bzw. in einer erfindungsgemäßen trockenen Zusammensetzung enthaltenen nanoskaligen, organischen Partikel höchstens 35 Gew.-%, vorzugsweise höchstens 25 Gew.-%, weiter bevorzugt höchstens 20 Gew.-%, weiter bevorzugt höchstens 15 Gew.-%, weiter bevorzugt höchstens 11 Gew.-%, jeweils bezogen auf das Gesamtgewicht der trockenen nanoskaligen, organischen Partikel, Übergangsmetall-Kationen, die vorzugsweise aus der Gruppe, die aus Titan, Zirkonium, Hafnium und Kombinationen davon, weiter bevorzugt Zirkonium, Hafnium und Kombinationen davon, weiter bevorzugt Zirkonium, besteht, ausgewählt werden, und/oder, vorzugsweise positiv geladene, Hydroxid-Komplex davon, wobei sich der Anteil der Übergangsmetall-Kationen und/oder, vorzugsweise positiv geladener, Hydroxid-Komplexe davon auf die auf der Oberfläche der nanoskaligen, organischen Partikel angeordneten, vorzugsweise gebundenen, Übergangsmetall-Kationen und/oder Hydroxid-Komplexe davon und, optional, auf die innerhalb der nanoskaligen, organischen Partikel angeordneten Übergangsmetall-Kationen und/oder Hydroxid-Komplexe davon bezieht.

Weiter bevorzugt enthalten die in einer erfindungsgemäßen Suspension bzw. in einer erfindungsgemäßen trockenen Zusammensetzung enthaltenen nanoskaligen, organischen Partikel im Wesentlichen keine, vorzugsweise keine Anionen des wasserlöslichen Salzes eines Übergangsmetalls.

Der Gehalt an organischen und anorganischen Bestandteilen der in einer erfindungsgemäßen Suspension bzw. in einer erfindungsgemäßen trockenen Zusammensetzung enthaltenen nanoskaligen, organischen Partikel kann mit im Stand der Technik bekannten Methoden bestimmt werden.

Ein geeignetes Verfahren zur Bestimmung des Gehalts an organischen und anorganischen Bestandteilen von in einer erfindungsgemäßen Suspension bzw. in einer erfindungsgemäßen trockenen Zusammensetzung enthaltenen nanoskaligen, organischen Partikeln ist beispielsweise die thermogravimetrische Analyse (TGA), die beispielsweise gemäß der in DIN EN ISO 11358-1:2014-10 ("Kunststoffe - Thermogravimetrie (TG) von Polymeren - Teil 1: Allgemeine Grundsätze (ISO 11358-1:2014); Ausgabedatum 2014-10) beschriebenen Verfahren durchgeführt werden kann.

Beispielsweise kann die thermogravimetrische Analyse unter Verwendung eines Messsystems der Fa. Perkin Elmer, Inc. (Waltham, MA, USA), beispielsweise einem TGA 8000^{®} TGA 8000^{®}, durchgeführt werden.

Vorzugsweise kann eine Probe, beispielsweise isolierte, vorzugsweise getrocknete, nanoskalige, organische Partikel, mit einer bestimmten Aufheizrate, vorzugsweise mit einer Aufheizrate von 10°C/min, von 25°C auf Temperaturen von bis zu 1200°C, vorzugsweise 800°C, erhitzt werden, vorzugsweise unter Verwendung eines, vorzugsweise inerten, Gases gemäß Herstellerangaben, beispielsweise Stickstoff, Argon und/oder Helium, vorzugsweise mit einem Druck aus einem Bereich von 1 bis 3 bar. Beim Erhitzen kann die Probe durch Reaktion und/oder Verdampfen flüchtige Komponenten an die Umgebung abgeben und/oder aus der Umgebung z. B. durch Oxidation Reaktionspartner aufnehmen. Die Gewichtsabnahme bzw. -zunahme und die Temperatur, bei welcher die Gewichtsänderung stattfindet, kann vorzugsweise spezifisch für eine untersuchte Probe sein. Daraus können vorzugsweise Rückschlüsse auf die Zusammensetzung des Stoffes gezogen werden.

Beispielsweise kann der Gehalt an organischen und anorganischen Bestandteilen der in einer erfindungsgemäßen Suspension bzw. in einer erfindungsgemäßen trockenen Zusammensetzung enthaltenen nanoskaligen, organischen Partikel durch Veraschen von isolierten, vorzugsweise getrockneten, nanoskaligen, organischen Partikeln bestimmt werden, beispielsweise nach den in DIN 38409-1 ("Deutsche Einheitsverfahren zur Wasser-, Abwasser- und Schlammuntersuchung; Summarische Wirkungs- und Stoffgrößen (Gruppe H); Bestimmung des Gesamttrockenrückstandes, des Filtrattrockenrückstandes und des Glührückstandes (H 1)", Ausgabedatum: 1987-01) beschriebenen Verfahren.

Unter Veraschen versteht man vorzugsweise das gezielte Zerstören mehrheitlich organischer Substanzen durch Erhitzen unter Sauerstoffeinfluss zur Bestimmung des Gehalts anorganischer Bestandteile.

Beispielsweise kann aus der Masse der isolierten, vorzugsweise getrockneten, Ausgangsprobe m₀ und der Masse der nach Veraschung verbleibenden Asche m₁ der anorganische Anteil, der auch als Glührückstand bezeichnet werden kann, in Masseprozent als Verhältnis von m₁/m₀ berechnet werden.

Unter Glühverlust (in %) wird nach DIN 18128 ("Baugrund - Untersuchung von Bodenproben - Bestimmung des Glühverlustes", Ausgabedatum: 2002-12) vorzugsweise der Anteil an organischer Substanz einer Probe definiert. Durch die Veraschung erfolgt vorzugsweise eine vollständige Entfernung des organischen Anteils in einer Probe, wobei der enthaltene Kohlenstoff, vorzugsweise vollständig, oxidiert wird und vorzugsweise als Kohlendioxid entweicht.

Beispielsweise können die in einer erfindungsgemäßen Suspension enthaltenen nanoskaligen, organischen Partikel durch Filtration und/oder Zentrifugation isoliert werden, wobei vorzugsweise die isolierten nanoskaligen, organischen Partikel getrocknet werden, weiter bevorzugt bis zur Massekonstanz.

Beispielsweise können die in einer erfindungsgemäßen trockenen Zusammensetzung enthaltenen nanoskaligen, organischen Partikeln zunächst durch Einbringen der Zusammensetzung in eine ausreichende Menge wenigstens eines wässrigen Lösungsmittels, beispielsweise Wasser, resuspendiert werden, wobei vorzugsweise wenigstens ein Hilfsstoff, beispielsweise pharmazeutisch akzeptable Matrixbildner, in dem wässrigen Lösungsmittel, beispielsweise Wasser, gelöst wird. Nachfolgend können die nanoskaligen, organischen Partikel beispielsweise durch Filtration und/oder Zentrifugation isoliert werden, wobei vorzugsweise die isolierten nanoskaligen, organischen Partikel getrocknet werden, weiter bevorzugt bis zur Massekonstanz.

Eine, vorzugsweise vollständige, Veraschung der organischen Bestandteile der isolierten, vorzugsweise getrockneten, nanoskaligen, organischen Partikel kann durch Beaufschlagung mit Energie, vorzugsweise bei einer Temperatur oberhalb des Schmelzpunktes der in den nanoskaligen, organischen Partikeln enthaltenen wenigstens einen organischen Verbindung, weiter bevorzugt bei einer Temperatur von 300°C oder mehr, vorzugsweise in Gegenwart von Sauerstoff, beispielsweise mit einem Druck von 1013 mbar oder mehr, erfolgen.

Vorzugsweise liegen vorgenannte Übergangsmetall-Kationen und/oder Hydroxid-Komplexe davon zumindest nach dem Veraschen als Oxid des entsprechenden Übergangsmetalls, beispielsweise als ZrO₂, HfO₂ oder als Mischung davon, vor.

Bei einer weiteren bevorzugten Ausführungsform weisen die in einer erfindungsgemäßen Suspension bzw. in einer erfindungsgemäßen trockenen Zusammensetzung enthaltenen nanoskaligen, organischen Partikel eine, im Vergleich zu der Dichte der in den organischen Partikeln enthaltenen reinen, vorzugsweise protonierten, wenigstens einen organischen Verbindung, höhere Partikeldichte auf. Die Bestimmung der Partikeldichte der in einer erfindungsgemäßen Suspension bzw. in einer erfindungsgemäßen trockenen Zusammensetzung enthaltenen organischen Partikel kann beispielsweise durch Verwendung eines Pyknometers oder mittels Schwingungsmessgerät (Biegeschwinger) erfolgen.

Beispielsweise kann eine Bestimmung der Partikeldichte in Anlehnung an die in Kratky, O. et al. ("The determination of the partial specific volume of proteins by the mechanical oscillator technique", Methods Enzymol. 27, 1973, Seiten 98 bis 110; DOI: 10.1016/S0076-6879(73)27007-6) beschriebene Methode unter Verwendung eines Dichtemessgerätes, beispielsweise eines Biegeschwingers bekannten Volumens, erfolgen. Der Biegeschwinger wird vorzugsweise mit einer zu analysierenden Flüssigkeit, beispielsweise einer erfindungsgemäßen Suspension oder einer in einem wässrigen Lösungsmittel, vorzugsweise Wasser, resuspendierten erfindungsgemäßen trockenen Zusammensetzung, gefüllt und zum Schwingen angeregt. Nach Kalibrierung der Messzelle mit beispielsweise zwei Flüssigkeiten bekannter Dichte, lässt sich aus der gemessenen Resonanzfrequenz die Dichte der zu analysierenden Flüssigkeit berechnen.

Weiter bevorzugt umfassen oder bestehen die in einer erfindungsgemäßen Suspension bzw. in einer erfindungsgemäßen trockenen Zusammensetzung enthaltenen nanoskaligen, organischen Partikel bzw. erfindungsgemäße nanoskalige, organische Partikel mindestens aus 65 gew.-%, vorzugsweise mindestens aus 75 Gew.-%, weiter bevorzugt mindestens aus 80 Gew.-%, weiter bevorzugt mindestens aus 85 Gew.-%, weiter bevorzugt mindestens aus 89 Gew.-%, jeweils bezogen auf das Gesamtgewicht der trockenen nanoskaligen, organischen Partikel, aus der vorgenannten wenigstens einen organischen Verbindung mit wenigstens 6 C-Atomen und wenigstens einem Säuregruppen-haltigen Rest und höchstens 35 Gew.-%, vorzugsweise höchstens 25 Gew.-%, weiter bevorzugt höchstens 20 Gew.-%, weiter bevorzugt höchstens 15 Gew.-%, weiter bevorzugt höchstens 11 Gew.-%, jeweils bezogen auf das Gesamtgewicht der tockenen nanoskaligen, organischen Partikel, Übergangsmetall-Kationen, die weiter bevorzugt aus der Gruppe, die aus Titan, Zirkonium, Hafnium und Kombinationen davon, weiter bevorzugt Zirkonium, Hafnium und Kombinationen davon, weiter bevorzugt Zirkonium, besteht, ausgewählt werden, und/oder Hydroxid-Komplexe davon.

Weiter bevorzugt beträgt der Anteil der nanoskaligen, organischen Partikel in einer erfindungsgemäßen Suspension höchstens 60 Gew.-%, weiter bevorzugt höchstens 50 Gew.-%, vorzugsweise aus einem Bereich von 0,001 Gew.-% bis 55 Gew.-%, weiter bevorzugt aus einem Bereich von 0,01 Gew.-% bis 45 Gew.-%, jeweils bezogen auf das Gesamtgewicht der Suspension.

Weiter bevorzugt beträgt der Anteil der nanoskaligen, organischen Partikel in einer erfindungsgemäßen trockenen Zusammensetzung höchstens 99,99 Gew.-%, vorzugsweise aus einem Bereich von 0,001 Gew.-% bis 99,99 Gew.-%, weiter bevorzugt aus einem Bereich von 0,01 Gew.-% bis 99,9 Gew.-%, weiter bevorzugt aus einem Bereich von 0,1 Gew.-% bis 99 Gew.-%, jeweils bezogen auf das Gesamtgewicht der trockenen Zusammensetzung.

Bei einer bevorzugten Ausführungsform ist eine erfindungsgemäße Suspension, die vorzugsweise durch das erfindungsgemäße Verfahren erhalten wird, für einen Zeitraum von wenigstens 24 Stunden, vorzugsweise wenigstens 36 Stunden, vorzugsweise wenigstens 48 Stunden, stabil, d.h. in dem angegebenen Zeitraum setzen sich weniger als 1 Gew.-% der eingesetzten nanoskaligen, organischen Partikel, vorzugsweise weniger als 0,5 Gew.-% der eingesetzten nanoskaligen, organischen Partikel, jeweils bezogen auf das Gesamtgewicht der nanoskaligen, organischen Partikel, aus der Suspension ab.

Das erfindungsgemäße Verfahren zur Herstellung einer erfindungsgemäßen Suspension umfasst folgenden Schritt:
a) in Kontakt bringen, vorzugsweise Mischen, einer Lösung, die wenigstens 1 der vorgenannten organischen Verbindung mit wenigstens 6 C-Atomen und wenigstens 1 Säuregruppen-haltigen Rest aufweist, mit einer wässrigen Lösung, die wenigstens ein wasserlösliches Salz eines Übergangsmetalls umfasst, unter Erhalt einer Suspension, die Wasser, nanoskalige, organische Partikel mit einer volumenbezogenen mittleren Primärpartikelgröße von weniger als 350 nm und wenigstens ein wasserlösliches Salz eines Übergangsmetalls umfasst, wobei an der Oberfläche der nanoskaligen, organischen Partikel wenigstens teilweise, vorzugsweise vollständig, Übergangsmetall-Kationen und/oder Hydroxid-Komplexe davon angeordnet, vorzugsweise gebunden, sind, und wobei die organischen Partikel wenigstens eine organische Verbindung mit wenigstens 6 C-Atomen und wenigstens 1 Säuregruppen-haltigen Rest umfassen.

Das erfindungsgemäße Verfahren erlaubt die Bereitstellung einer, vorzugsweise stabilisierten, Suspension nanoskaliger, organischer Partikel. Vorzugsweise weist eine mit dem erfindungsgemäßen Verfahren hergestellte Suspension nanoskaliger, organischer Partikel im Wesentlichen keine, vorzugsweise keine, Partikelagglomerate auf. Weiter bevorzugt weist eine mit dem erfindungsgemäßen Verfahren hergestellte Suspension nanoskalige, organische Partikel auf, die vorzugsweise vollständig in Form von Einzel-Partikeln vorliegen.

Durch Verwendung eines wasserlöslichen Salzes eines Übergangsmetalls ist es möglich, eine erfindungsgemäße Suspension bereitzustellen, die vorzugsweise keine Bestandteile wie organische Stabilisatoren oder Dispergiermitteln, wie beispielsweise Tensiden und/ oder Emulgatoren, enthält.

Durch das erfindungsgemäße Verfahren wird vorzugsweise weiterhin die Bereitstellung einer Suspension nanoskaliger, organischer Partikel ermöglicht, die eine enge Partikelgrößenverteilung aufweisen.

Durch das in Kontakt bringen, vorzugsweise Mischen, einer Lösung, die wenigstens eine der vorgenannten organischen Verbindungen mit wenigstens 6 C-Atomen und wenigstens einem Säuregruppen-haltigen Rest aufweist, mit einer wässrigen Lösung, die wenigstens ein wasserlösliches Salz eines Übergangsmetalls umfasst, ist es weiterhin möglich, eine Suspension nanoskaliger, organischer Partikel bereitzustellen, die auch in einem Bereich der mittleren Partikelgröße von weniger als 150 nm, vorzugsweise von weniger als 100 nm, eine einstellbare, vorzugsweise enge, weiter bevorzugt stabile, Größenverteilung der mittleren Partikelgröße aufweist.

Vorzugsweise kann die Breite der Partikelgrößenverteilung durch Ermittlung der Breite der Verteilungsfunktion ("Spanne"), wie vorstehend erläutert, bestimmt werden.

Vorzugsweise erfolgt Schritt a) des erfindungsgemäßen Verfahrens durch Mischen einer Lösung, die die wenigstens eine vorgenannte organische Verbindung aufweist, mit einer wässrigen Lösung, die wenigstens ein wasserlösliches Salz eines Übergangsmetalls umfasst.

Vorzugsweise kann das in Kontakt bringen, vorzugsweise Mischen, durch Rühren, etwa in einem Rotor-Stator-System, erfolgen. Alternativ kann ein statischer Mischer, beispielsweise ein T-Mischer, ein Y-Mischer oder ein Zyklon-Mischer, oder ein dynamischer Mischer, beispielsweise ein Rührwerk, verwendet werden.

Weiter bevorzugt erfolgt das in Kontakt bringen, vorzugsweise Mischen, unter Verwendung eines statischen Mischers, beispielsweise eines T-Mischers, eines Y-Mischers und/oder eines Zyklon-Mischers.

Beispielsweise kann Schritt a) des erfindungsgemäßen Verfahrens unter Einwirkung von Vibration, Ultraschall, Kavitation oder eine Kombination davon erfolgen.

Bei einer bevorzugten Ausführungsform des erfindungsgemäßen Verfahren wird die im erfindungsgemäßen Verfahren verwendete Lösung, die die vorgenannte wenigstens 1 organische Verbindung mit wenigstens 6 C-Atomen und wenigstens 1 Säuregruppen-haltigen Rest aufweist, durch ein Verfahren bereitgestellt, das folgenden Schritt umfasst:
a1) Lösen der vorgenannten wenigstens 1 organischen Verbindung mit wenigstens 6 C-Atomen und wenigstens 1 Säuregruppen-haltigen Rest in einer wässrigen Lösung, vorzugsweise Wasser, die einen pH-Wert von größer als 6,0, vorzugsweise von größer als 7,0, aufweist, unter Erhalt einer wässrigen, vorzugsweise alkalischen, Lösung oder
a2) Lösen der vorgenannten wenigstens 1 organischen Verbindung mit wenigstens 6 C-Atomen und wenigstens 1 Säuregruppen-haltigen Rest in wenigstens 1 organischen Lösungsmittel unter Erhalt einer organischen Lösung.

Das Lösen der vorgenannten wenigstens 1 organischen Verbindung mit wenigstens 6 C-Atomen und wenigstens 1 Säuregruppen-haltigen Rest in Schritt a1) kann beispielsweise unter Verwendung wenigstens 1 Base, vorzugsweise wenigstens 1 Alkalimetallhydroxids, beispielsweise NaOH, KOH, LiOH oder einer Mischung davon, wenigstens 1 Alkalimetallcarbonats, beispielsweise Na₂CO₃, K₂CO₃ oder einer Mischung davon, und/oder wenigstens 1 Alkalimetallhydrogencarbonats, NaHCOs, KHCO₃ oder einer Mischung davon, weiter bevorzugt wenigstens 1 Alkalimetallhydroxids, beispielsweise NaOH, KOH, LiOH oder einer Mischung davon, durchgeführt werden.

Durch in Kontakt bringen, vorzugsweise Mischen, der in Schritt a1) erhaltenen wässrigen, alkalischen Lösung, die wenigstens 1 der vorgenannten organischen Verbindung mit wenigstens 6 C-Atomen und wenigstens 1 Säuregruppen-haltigen Rest enthält, mit einer, vorzugsweise sauren, Lösung wenigstens 1 wasserlöslichen Übergangsmetallsalzes, die vorzugsweise einen pH-Wert von kleiner als 7,0, vorzugsweise von kleiner als 4,0, aufweist, kommt es vorzugsweise zu einem pH-Wechsel der resultierenden Mischung, die weiter bevorzugt nach dem in Kontakt bringen, vorzugsweise Mischen, einen pH-Wert von kleiner 7,0, vorzugsweise aus einem Bereich von 6,5 bis 0, weiter bevorzugt aus einem Bereich von 4,0 bis 1,0, aufweist.

Durch den pH-Wechsel kommt es vorzugsweise zur Bildung von nanoskaligen, vorzugsweise amorphen, organischen Partikeln, die die vorgenannten wenigstens 1 organische Verbindung mit wenigstens 6 C-Atomen und wenigstens 1 Säuregruppen-haltigen Rest umfassen und an deren Oberfläche zumindest teilweise, vorzugsweise vollständig, Übergangsmetall-Kationen und/oder Hydroxid-Komplexe davon gebunden sind.

Alternativ kann in Schritt a2) die vorgenannte wenigstens 1 organische Verbindung mit wenigstens 6 C-Atomen und wenigstens 1 Säuregruppen-haltigen Rest in wenigstens 1 organischen Lösungsmittel gelöst werden, das vorzugsweise aus der Gruppe, die aus Ethern, Alkanen, Ketonen, Laktonen, Laktamen, Nitrilen, Nitroverbindungen, Sulfoxiden, Sulfonen, aliphatischen Alkoholen, primären Aminen, sekundären Aminen, primären Amiden, sekundären Amiden, und Mischungen davon besteht, ausgewählt werden.

Bevorzugt verwendet werden kurzkettige Alkohole, vorzugsweise mit 1 bis 3 C-Atomen, bzw. Lösungsmittel, die aus kleinen Molekülen, vorzugsweise mit 1 bis 3 C-Atomen, bestehen, da hierbei der Diffusionskoeffizient des Lösemittels in das Wasser größer ist. Dadurch kann es zu einer schnelleren Mischung der Komponenten kommen.

Ungeeignet sind vorzugsweise Lösungsmittel, die mit den Übergangsmetall-Kationen reagieren bzw. in Anwesenheit von Übergangsmetall-Kationen katalytisch mit der wenigstens einen organischen Verbindung mit wenigstens 6 C-Atomen und wenigstens einem Säuregruppen-haltigen Rest umgesetzt werden können.

Vorzugsweise weist die vorgenannte wenigstens 1 organische Verbindung mit wenigstens 6 C-Atomen und wenigstens 1 Säuregruppen-haltigen Rest in dem in Schritt a2) verwendeten wenigstens 1 organischen Lösungsmittel eine Löslichkeit, die vorzugsweise gemäß DIN EN ISO 7579:2010-03 ("Farbstoffe - Bestimmung der Löslichkeit in organischen Lösemitteln-Gravimetrisches und photometrisches Verfahren"; Ausgabedatum: 2010-03) bestimmt wird, bei 25°C und einem Druck von 1013 mbar von 20 g/l oder mehr, vorzugsweise von 50 g/l oder mehr, vorzugsweise von 100 g/l oder mehr, auf.

Nach in Kontakt bringen, vorzugsweise Mischen, der in Schritt a2) erhaltenen organischen Lösung der vorgenannten wenigstens 1 organischen Verbindung mit wenigstens 6 C-Atomen und wenigstens 1 Säuregruppen-haltigen Rest mit einer wässrigen Lösung wenigstens 1 wasserlöslichen Übergangsmetallsalzes, wobei die wässrige Lösung vorzugsweise als Anti-Solvent wirken kann, vermindert sich in der resultierenden Mischung vorzugsweise die Löslichkeit der wenigstens 1 organischen Verbindung mit wenigstens 6 C-Atomen und wenigstens einen Säuregruppen-haltigen Rest.

Dadurch kommt es vorzugsweise zur Bildung von nanoskaligen, vorzugsweise amorphen, organischen Partikeln, die die vorgenannten wenigstens eine organische Verbindung mit wenigstens 6 C-Atomen und wenigstens 1 Säuregruppen-haltigen Rest umfassen und an deren Oberfläche zumindest teilweise, vorzugsweise vollständig, Übergangsmetall-Kationen und/oder Hydroxid-Komplexe davon gebunden sind.

Vorzugsweise können die mit dem erfindungsgemäßen Verfahren erhaltenen nanoskaligen, vorzugsweise amorphen, organischen Partikel isoliert werden, um beispielsweise Spuren des verwendeten organischen Lösungsmittels zu entfernen oder um beispielsweise nanoskalige, organische Partikel für eine spätere Verwendung getrennt von der wässrigen Phase einer erfindungsgemäßen Suspension aufzubewahren.

Beispielsweise können nach Entfernen vorhandener Reste des verwendeten wenigstens 1 organischen Lösungsmittels die nanoskaligen, organischen Partikel erneut in einem, vorzugsweise wässrigen, Lösungsmittel resuspendiert werden.

Beispielsweise können isolierte nanoskalige, organische Partikel kurz vor einer weiteren Verwendung in einem, vorzugsweise pharmazeutisch akzeptablen, wässrigen Lösungsmittel resuspendiert werden.

Geeignete pharmazeutisch akzeptable, wässrige Lösungsmittel sind beispielsweise Wasser oder wenigstens ein, vorzugsweise isotonischer, Puffer.

Das Isolieren der mit dem erfindungsgemäßen Verfahren erhaltenen nanoskaligen, organischen Partikel kann beispielsweise aus einer erfindungsgemäßen Suspension durch Filtration, Zentrifugation, Fliehkraftabscheidung, Dekantation, Trocknung oder einer Kombination davon erfolgen. Geeignete Verfahren der Trocknung sind beispielsweise Sprühtrocknung und/oder Gefriertrocknung.

Isolierte erfindungsgemäße, vorzugsweise amorphe, nanoskalige, organische Partikel weisen eine, vorzugsweise stabile, volumenbezogene mittlere Partikelgröße von weniger als 350 nm auf, wobei an der Oberfläche der nanoskaligen, organischen Partikel wenigstens teilweise, vorzugsweise vollständig, Übergangsmetall-Kationen und/oder Hydroxid-Komplexe davon angeordnet, vorzugsweise gebunden, sind, und wobei die nanoskaligen, organischen Partikel wenigstens 1 der vorgenannten organischen Verbindung mit wenigstens 6 C-Atomen und wenigstens 1 Säuregruppen-haltigen Rest umfassen.

Vorzugsweise können die mit dem erfindungsgemäßen Verfahren erhaltenen nanoskaligen, organischen Partikel, beispielsweise in Form einer erfindungsgemäßen Suspension, zusammen mit wenigstens einem Hilfsstoff, beispielsweise pharmazeutisch akzeptablen Matrixbildner, in eine erfindungsgemäße trockene Zusammensetzung überführt werden.

Beispielsweise kann das Überführen in eine erfindungsgemäße trockene Zusammensetzung durch Trocknung, vorzugsweise Sprühtrocknung und/oder Gefriertrocknung, einer erfindungsgemäßen Suspension in Gegenwart wenigstens eines pharmazeutisch akzeptablen, vorzugsweise wasserlöslichen, Matrixbildners erfolgen.

Eine erfindungsgemäße trockene Zusammensetzung umfasst, nanoskalige, vorzugsweise amorphe, organische Partikel mit einer, vorzugsweise stabilen, volumenbezogenen mittleren Partikelgröße von weniger als 350 nm und wenigstens einen, vorzugsweise bei Standardbedingungen (Druck: 1013 mbar, Temperatur: 25°C) festen, Hilfsstoff, wobei an der Oberfläche der nanoskaligen, organischen Partikel wenigstens teilweise, vorzugsweise vollständig, vorgenannte Übergangsmetall-Kationen und/oder Hydroxid-Komplexe davon angeordnet, vorzugsweise gebunden, sind, und wobei die nanoskaligen, organischen Partikel wenigstens 1 der vorgenannten organischen Verbindung mit wenigstens 6 C-Atomen und wenigstens 1 Säuregruppen-haltigen Rest umfassen.

Vorzugsweise wird der wenigstens eine, vorzugsweise bei Standardbedingungen feste, Hilfsstoff aus der Gruppe, die aus pharmazeutisch akzeptablen, vorzugsweise wasserlöslichen, Matrixbildnern, anorganischen, vorzugsweise wasserlöslichen, Salzen, Bindemitteln, Füllstoffen, Pigmenten und Mischungen davon besteht, ausgewählt.

Weiter bevorzugt beträgt der Anteil des wenigstens eine Hilfsstoffes in einer erfindungsgemäßen trockenen Zusammensetzung wenigstens 0,01 Gew.-%, vorzugsweise aus einem Bereich von 0,01 Gew.-% bis 99,999 Gew.-%, weiter bevorzugt aus einem Bereich von 0,1 Gew.-% bis 99,99 Gew.-%, weiter bevorzugt aus einem Bereich von 1 Gew.-% bis 99,9 Gew.-%, jeweils bezogen auf das Gesamtgewicht der trockenen Zusammensetzung.

Geeignete pharmazeutisch akzeptable, vorzugsweise wasserlösliche, Matrixbildner werden vorzugsweise aus der Gruppe, die aus Carbonsäuren und deren Salze, vorzugsweise Fumarsäure, Citronensäure, Äpfelsäure, Weinsäure, Ascorbinsäure, Asparaginsäure, Adipinsäure, Alginsäure, Benzoesäure, Bernsteinsäure, Isoascorbinsäure, Milchsäure, Äpfelsäure, Maleinsäure, Glutaminsäure, Sorbinsäure, Bernsteinsäure, Salze davon und Mischungen davon, Polysaccharide mit 11 oder mehr Monosaccharid-Einheiten, die über eine glycosidische Bindung verbunden sind, beispielsweise Glykogen, Amylose, Amylopektin, Pektine, Chitin, Callose, Cellulose und Mischungen davon, Oligosaccharide mit zwei bis 10 Monosaccharid-Einheiten, die über eine glycosidische Bindung verbunden sind, beispielsweise Isomaltose, Isomaltulose, Lactose, Maltose, Saccharose, Trehalose und Mischungen davon, Monosaccharide, beispielsweise Fructose, Glucose, Galactose, und Mischungen davon, Carboxyalkylcellulose, vorzugsweise Carboxymethylcellullose, Carboxyethylcellulose und Mischungen davon, Hydroxyalkylcellulose, vorzugsweise Hydroxypropylmethylcellulose, Hydroxyethylmethylcellulose, Hydroxypropylcellulose, Hydroxyethylylcellulose, Hydroxymethylylcellulose und Mischungen davon, Poloxamere, Polyvinylpyrrolidon, und Mischungen davon besteht, ausgewählt.

Geeignete Bindemittel können beispielsweise anorganische Bindemittel, organische Bindemittel oder Mischungen davon sein. Geeignete anorganische Bindemittel sind beispielsweise gebrannter Kalk, Zement, Anhydrit, Ettringit (Satinweiß/Casul), Kaliwasserglas oder Mischungen davon. Geeignete organische Bindemittel sind beispielsweise Polymere und Copolymere auf Basis von Polyurethanen, Poly(meth)acrylaten, Vinylacetaten, Epoxiden, Polyestern, oder Mischungen davon.

Beispielsweise kann eine erfindungsgemäße trockene Zusammensetzung und/oder erfindungsgemäße nanoskalige, vorzugsweise amorphe, organische Partikel kurz vor einer weiteren Verwendung in einem, vorzugsweise pharmazeutisch akzeptablen, wässrigen Lösungsmittel, vorzugsweise Wasser oder wenigstens einem, vorzugsweise isotonischen, Puffer, resuspendiert werden.

Geeignete, vorzugsweise isotonische, Puffer sind wässrige Elektrolytlösungen mit vorzugsweise isotonischer Zusammensetzung, beispielsweise Ringer-Lactat, isotonische Kochsalzlösung, Vollelektrolytlösung, Tyrode-Lösung oder Mischungen davon.

Eine erfindungsgemäße trockene Zusammensetzung weist nanoskalige, vorzugsweise amorphe, organische Einzel-Partikel auf, die nach Resuspension in einem, vorzugsweise pharmazeutisch akzeptablen, wässrigen Lösungsmittel, vorzugsweise Wasser, einem Puffer oder eine Mischungen davon, das vorzugsweise einen pH-Wert aus einem Bereich von 0 bis 7, vorzugsweise von 0,5 bis 4, weiter bevorzugt von 1 bis 3, weiter bevorzugt von 1,15 bis 2,5, weiter bevorzugt von 1,25 bis 2,25, aufweist, vorzugsweise für wenigstens 24 Stunden, weiter bevorzugt für wenigstens 36 Stunden, weiter bevorzugt für wenigstens 48 Stunden, im Wesentlichen nicht, vorzugsweise nicht, sedimentieren und/oder reifen.

Daher kann eine erfindungsgemäße Suspension und/oder eine erfindungsgemäße trockene Zusammensetzung beispielsweise als Medikament verwendet werden, wobei vorzugsweise die jeweils enthaltenen nanoskaligen, organischen Partikel wenigstens 1 der vorgenannten pharmazeutischen Wirkstoffe mit sauren Gruppen umfassen oder daraus bestehen.

Die jeweils enthaltenen nanoskaligen, organischen Partikel agglomerieren bei der Lagerung der Suspension oder trockenen Zusammensetzung im Wesentlichen nicht, vorzugsweise nicht.

Vorzugsweise kommt es nach der Einnahme einer erfindungsgemäßen Suspension und/oder einer erfindungsgemäßen trockene Zusammensetzung und/oder isolierten erfindungsgemäßen, vorzugsweise amorphen, nanoskaligen, organischen Partikeln zu keiner Agglomeration der jeweils enthaltenen nanoskaligen, organischen Partikel.

Weiterhin kann eine erfindungsgemäße Suspension und/oder trockene Zusammensetzung und/oder isolierte erfindungsgemäße, vorzugsweise amorphe, nanoskalige, organische Partikel beispielsweise als Additiv in Kosmetika oder bei der Beschichtung oder Imprägnierung von anorganischen oder organischen Oberflächen und/oder Gegenständen, beispielsweise zur Farbgebung und/oder als UV-Schutz, verwendet werden, wobei vorzugsweise die jeweils enthaltenen nanoskaligen, organischen Partikel wenigstens 1 der vorgenannten Säurefarbstoffe umfassen oder daraus bestehen.

Weiterhin kann eine erfindungsgemäße Suspension und/oder trockene Zusammensetzung und/oder isolierte erfindungsgemäße, vorzugsweise amorphe, nanoskalige, organische Partikel als Pflanzenschutzmittel verwendet werden, wobei vorzugsweise die jeweils enthaltenen nanoskaligen, organischen Partikel wenigstens 1 der vorgenannten Pestizide mit sauren Gruppen umfassen oder daraus bestehen.

Nachfolgend wir die Erfindung durch Beispiele und Zeichnungen beschrieben, ohne hierauf beschränkt zu sein.

In Figur 1a sind die in Beispiels 1 gemessenen Absorptionsspektren dargestellt, wobei jeweils die Konzentration der verwendeten ZrCl₄-Lösung angegeben ist, mit der die entsprechende Suspension hergestellt wurde. Abb. 1b zeigt einen Ausschnitt des in Abb. 1a dargestellten Absorptionsspektrums, wobei ein Vergleich der jeweils gemessenen Extinktion bei 600 nm in Abhängigkeit der eingesetzten ZrCl₄-Konzentration dargestellt ist. In Abb. 1a und Abb. 1b wurden die gemessenen Extinktions-Werte jeweils auf eine Absorbanz bei 333 nm normiert.

Abb. 2 zeigt die in Beispiel 1 gemessenen IR-Spektren nach der Fourier-Transformation des Interferogramms von Naproxen-Partikeln ("Naproxen") und ZrCl₄-stabilisierten Naproxen-Partikeln ("Nap + ZrCl4").

Abb. 3 zeigt die in Beispiel 3 bestimmten Zeta-Potentiale ("ζ-Potential") von Vergleichssuspensionen ohne ZrCl₄-Zugabe in Abhängigkeit des pH-Wertes und das Zeta-Potential von Partikeln einer Suspension, die mit einer ZrCl₄-Lösung (c (ZrCl₄) = 0,075 mol I⁻¹) als Antisolvent hergestellt wurde.

Abb. 4 zeigt die in Beispiel 3 bestimmten Zeta-Potentiale ("ζ-Potential") von ZrCl₄-stabilisierten Naproxen-Suspensionen in Abhängigkeit der eingesetzten ZrCl₄-Konzentration.

In Abb. 5 ist die in Beispiel 3 ermittelte Messkurve zur Bestimmung der Partikeldichte in Abhängigkeit der Naproxen-Konzentration dargestellt.

Abb. 6 zeigt die gemessene Partikelgrößenverteilung der in den in Beispiel 4 hergestellten Suspensionen enthaltenen ZrCl₄-stabilisierten Naproxen-Partikel in Abhängigkeit der eingesetzten ZrCl₄-Konzentration bei Verwendung eines T-Mischers.

Abb. 7 zeigt einen Vergleich der in Beispiel 4 gemessenen Partikelgrößenverteilung, die mittels analytischer Ultrazentrifugation ("AUZ") bzw. unter Verwendung der dynamischen Lichtstreuung mit einem Malvern Zetasizers Nano ZS ("Zetasizer") ermittelt wurde.

Abb. 8 zeigt die gemessene Partikelgrößenverteilung der in den in Beispiel 5 hergestellten Suspensionen enthaltenen ZrCl₄-stabilisierten Ibuprofen-Partikel in Abhängigkeit der eingesetzten ZrCl₄-Konzentration, wobei jeweils die gemessenen Parameter x10,3, x50,3 und x90,3 der Partikelgrößenverteilung nach manuellem Mischen mit Hilfe einer Pipette dargestellt sind.

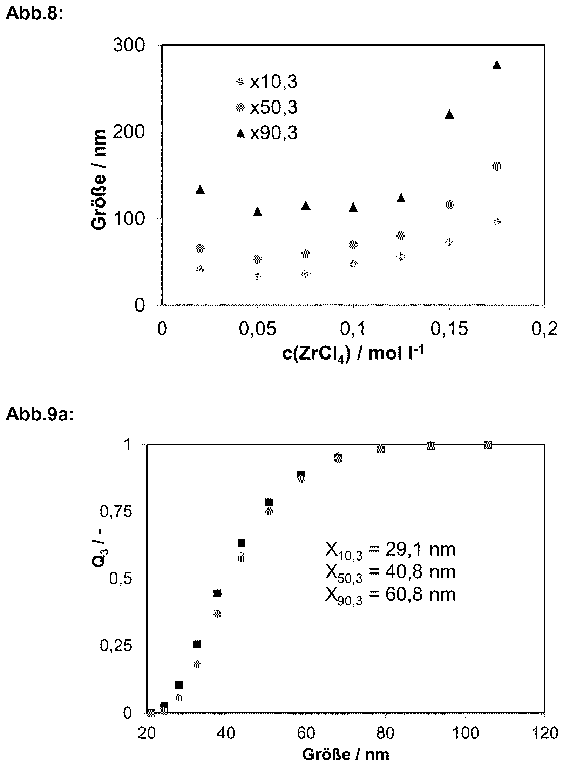

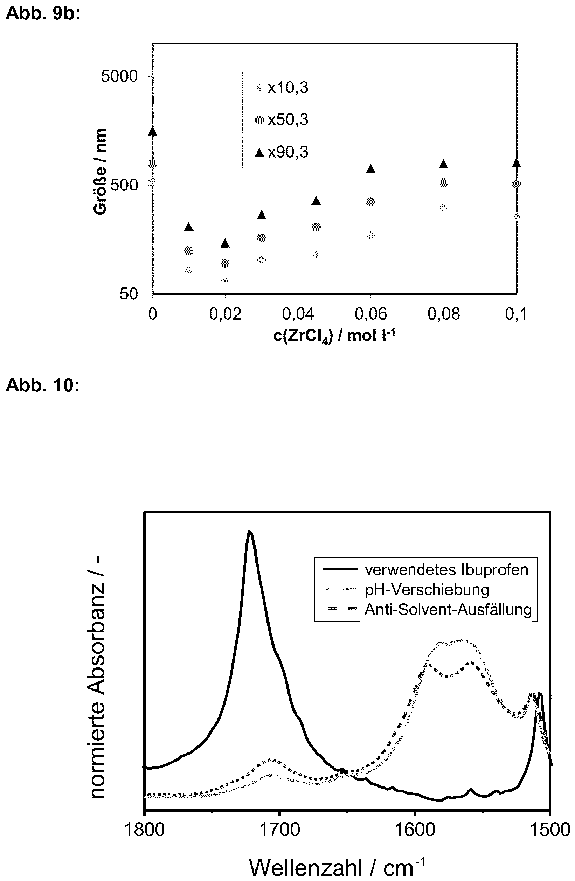

Abb. 9a zeigt die Summenverteilung (Q₃) der volumenbezogenen Äquivalentdurchmesser von 3 unabhängigen Messungen der in den in Beispiel 5 hergestellten Suspensionen enthaltenen ZrCl₄-stabilisierten Ibuprofen-Partikel nach Mischen mit Hilfe eines statischen Mischers. Abb. 9b zeigt die gemessene Partikelgrößenverteilung der in den in Beispiel 5 hergestellten Suspensionen enthaltenen ZrCl₄-stabilisierten Ibuprofen-Partikel in Abhängigkeit der eingesetzten ZrCl₄-Konzentration, wobei jeweils die gemessenen Parameter x10,3, x50,3 und x90,3 der Partikelgrößenverteilung nach Mischen mit Hilfe eines statischen Mischers dargestellt sind.

Abb. 10 zeigt die in Beispiel 5 gemessenen IR-Spektren nach der Fourier-Transformation des Interferogramms von Ibuprofen ("Bulk") und ZrCl₄-stabilisiertem Ibuprofen, das durch Anti-Solvent-Fällung ("Anti-Solvent") bzw. durch pH-Verschiebung ("pH-shift") hergestellt wurde.

Abb. 11 zeigt die gemessene Partikelgrößenverteilung der in den in Beispiel 5 mit verschiedenen organischen Lösungsmittel hergestellten Suspensionen enthaltenen ZrCl₄-stabilisierten Ibuprofen-Partikel, wobei jeweils die gemessenen Parameter x10,3, x50,3 und x90,3 der Partikelgrößenverteilung nach Mischen mit Hilfe eines statischen Mischers dargestellt sind.

Abb. 12 zeigt die gemessene Partikelgrößenverteilung der in den in Beispiel 6 hergestellten Suspensionen enthaltenen ZrOCl₂-stabilisierten Ibuprofen-Partikel in Abhängigkeit der eingesetzten ZrOCl₂-Konzentration, wobei jeweils die gemessenen Größen x10,3, x50,3 und x90,3 der Partikelgrößenverteilung nach Mischen mit Hilfe einem statischen Mischer in Abhängigkeit von der verwendeten ZrOCl₂-Konzentration angegeben sind.

Abb. 13 zeigt das arithmetische Mittel der gemessenen Parameter x10,3, x50,3 und x90,3 der Partikelgrößenverteilung von in Beispiel 7 hergestellten ZrCl₄-stabilisierten Ibuprofen-Suspensionen in Abhängigkeit der verwendeten Ibuprofen-Konzentration.

Abb. 14 zeigt jeweils den gemessenen Parameter x50,3 der Partikelgrößenverteilung von in Beispiel 7 mit unterschiedlichen ZrCl₄-Konzentrationen hergestellten Ibuprofen-Suspensionen in Abhängigkeit der verwendeten ZrCl₄-Konzentration.

Abb. 15 zeigt jeweils die Summenverteilung (Q₃) der volumenbezogenen Äquivalentdurchmesser der in Beispiel 7 hergestellten Suspension enthaltenen ZrCl₄-stabilisierten Ibuprofen-Partikel, die direkt nach der Herstellung ("frisch gefällt") bzw. nach 2 Monaten Lagerung ("nach zwei Monaten") gemessen wurden.

Abb. 16 und 17 zeigen Rasterelektronenmikroskopaufnahmen von ZrCl₄-stabilisierten Ibuprofen-Partikeln, die in Beispiel 7 hergestellt wurden, nach 2 monatiger Lagerung mit einer 26430-fachen Vergrößerung (Mag = 26.43 K X) bzw. 67130-fachen Vergrößerung (Mag =67.13 KX). Die jeweils verwendete Vergrößerung (Mag), Beschleunigungsspannung (EHT) und der Arbeitsabstand (WD) sind für das jeweilige Bild angegeben. Der Detektor (Inlens) ist ebenfalls angegeben.

Abb. 18 zeigt die gemessene Partikelgrößenverteilung der in den in Beispiel 9 hergestellten Suspensionen enthaltenen HfCl₄-stabilisierten Ibuprofen-Partikel in Abhängigkeit von der eingesetzten ZrCl₄-Konzentration. Gezeigt sind jeweils die Parameter x10,3, x50,3 und x90,3 der Partikelgrößenverteilung nach Mischen mit Hilfe eines statischen Mischers, die mit frisch hergestellten Partikeln (gefüllte Symbole) sowie nach 5 Tagen Lagerung der Partikel ("leere Symbole") gemessen wurden.

Abb. 19 zeigt die in Beispiel 9 gemessenen IR-Spektren nach der Fourier-Transformation des Interferogramms von Ibuprofen ("Bulk") und HfCl₄-stabilisiertem Ibuprofen, das durch Anti-Solvent-Fällung ("Anti-Solvent") hergestellt wurde.

Abb. 20 und 21 zeigen SEM-Aufnahmen der in Beispiel 10 hergestellten trockenen Zusammensetzung mit einer 3960-fachen Vergrößerung (Mag = 3.96 K X) bzw. mit einer 51600-fachen Vergrößerung (Mag = 51.60 KX), wobei Abb. 21 eine Vergrößerung des in Abb. 20 markierten Ausschnittes zeigt. Die jeweils verwendete Vergrößerung (Mag), Beschleunigungsspannung (EHT) und der Arbeitsabstand (WD) sind für das jeweilige Bild angegeben. Der Detektor (SE2) ist ebenfalls angegeben.

Abb. 22 und 23 zeigen SEM-Aufnahmen der in Vergleichsbeispiel 11 hergestellten Ibuprofen-Partikel mit einer 3980-fachen Vergrößerung (Mag = 3.98 K X) bzw. 9940-fachen Vergrößerung (Mag =9.94 KX). Die jeweils verwendete Vergrößerung (Mag), Beschleunigungsspannung (EHT) und der Arbeitsabstand (WD) sind für das jeweilige Bild angegeben. Der Detektor (SE2) ist ebenfalls angegeben.

Abb. 24 zeigt die durch Bildauswertung von Abb. 22 und 23 erhaltenen Summenverteilung (Q₃) der volumenbezogenen Äquivalentdurchmesser der in den in Vergleichsbeispiel 11 hergestellten Suspensionen enthaltenen Ibuprofen-Partikel.

Abb. 25 und 26 zeigen SEM-Aufnahmen der in Vergleichsbeispiel 12 hergestellten Ibuprofen-Partikel mit einer 2430-fachen Vergrößerung (Mag = 2.43 K X) bzw. 186000-fachen Vergrößerung (Mag =186 K X). ). Die jeweils verwendete Vergrößerung (Mag), Beschleunigungsspannung (EHT) und der Arbeitsabstand (WD) sind für das jeweilige Bild angegeben. Der Detektor (SE2) ist ebenfalls angegeben.

Die in den folgenden Beispielen jeweils angegebenen Parameter x10,3 bzw. x50,3 bzw. x90,3 der gemessenen Partikelgrößenverteilung geben jeweils an, dass 10 % bzw. 50 % bzw. 90 % der untersuchten Partikel einen Durchmesser aufweisen, der gleich oder kleiner als der angegebene Wert ist, wobei durch den Index 3 gekennzeichnet ist, dass jeweils der volumenbezogene Äquivalentdurchmesser der Partikel bestimmt wurde.

### Beispiel 1: Herstellung von stabilisierten Naproxen-Suspensionen mit ZrCl₄.

Als Ausgangssubstanz wurde kommerziell erhältliches Naproxen [(S)-(+)-6-Methoxy-α-methyl-2-naphthalenessigsäure, CAS-Nr.: 22204-53-1] der Firma Sigma-Aldrich Chemie GmbH (München, DE) (Molare Masse: 230,26 g/mol, Gehalt gemäß Herstellerangabe: ≥ 98%) ohne weitere Reinigung verwendet. Naproxen weist die in Formel (1) dargestellte Struktur auf.

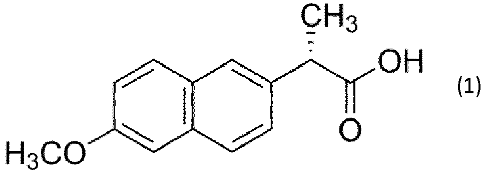

Weiterhin wurde wasserfreies Zirkoniumtetrachlorid [ZrCl₄, CAS-Nr.: 10026-11-6] der Sigma-Aldrich Chemie GmbH (Molare Masse: 233,04 g/mol, Gehalt gemäß Herstellerangabe: ≥ 98%) ohne weitere Reinigung verwendet.

Zur Herstellung einer Naproxen-Stammlösung mit einem Naproxen-Gehalt von 5 mg/mL wurden zunächst 5 g Naproxen in 1 L einer wässrigen NaOH-Lösung (Konzentration: 0,04 mol/L) gelöst. NaOH wurde von Alfa Aesar (Ward Hill, MA, USA) bezogen.

Weiterhin wurden eine ZrCl₄-Stammlösung mit eine Konzentration von 1 mol/L durch Lösen von 233,04 g ZrCl₄ in 1 L Reinstwasser hergestellt. Das verwendete Reinstwasser wurde in einer Reinstwasseranlage PURELAB^{®} Ultra der Firma ELGA LabWater (High Wycombe, UK) hergestellt. Verschiedene ZrCl₄-Lösungen mit den in Tabelle 1 angegebenen Konzentrationen wurden durch Lösen der entsprechenden Menge an ZrCl₄ in 10 mL Reinstwasser hergestellt.

**Tabelle 1: Verwendete ZrCl₄-Lösungen**

| Konzentration ZrCl₄ | Herstellung durch | pH-Wert |
|---|---|---|
| 0,025 mol/L | Verdünnen von 250 µL ZrCl₄-Stammlösung | 1,77 |
| 0,05 mol/L | Verdünnen von 500 µL ZrCl₄-Stammlösung | 1,36 |
| 0,075 mol/L | Verdünnen von 750 µL ZrCl₄-Stammlösung | 1,3 |
| 0,1 mol/L | Verdünnen von 1 mL ZrCl₄-Stammlösung | 1,01 |
| 0,125 mol/L | Verdünnen von 1,25 mL ZrCl₄-Stammlösung | 0,943 |
| 0,15 mol/L | Verdünnen von 1,5 mL ZrCl₄-Stammlösung | 0,827 |
| 0,2 mol/L | Verdünnen von 2 mL ZrCl₄-Stammlösung | 0,723 |
| 0,25 mol/L | Verdünnen von 2,5 mL ZrCl₄-Stammlösung | 0,625 |

Gleiche Volumenanteile der verschiedenen ZrCl₄-Lösungen (je 10 mL) und der Naproxen-Stammlösung (je 10 mL) wurden jeweils miteinander vermischt. Das Vermischen erfolgte manuell durch Pipettieren der Lösungen. Durch den Wechsel des pH-Wertes von basisch zu sauer während des Mischens bildeten sich nanoskalige Naproxen-Partikel, an deren Oberfläche Zirkonium-Ionen und/oder Hydroxid-Komplexe davon gebunden waren.

Von den hergestellten Suspensionen wurden in einem Zeitraum von 5 min nach Herstellung UV-VIS Spektren aufgenommen. Dazu wurden jeweils 10 µl der hergestellten Suspension mit 1 mL Reinstwasser verdünnt und mit einem UV-VIS Spektrometer Varian Cary 100 UV-VIS der Firma Agilent Technologies (Santa Clara, CA, USA) vermessen.

Die zu vermessende Probe wurde mit elektromagnetischer Strahlung im Bereich des sichtbaren (VIS) und ultravioletten (UV) Lichts bestrahlt und die Extinktion der Strahlung bezogen auf die Wellenlänge der verwendet Strahlung gemessen.

Die aufgenommenen Spektren in einem Bereich von 300 nm bis 600 nm wurden auf eine Absorbanz bei 333 nm normiert. Die Absorbanz kann gemäß DIN 1349-1 ("Durchgang optischer Strahlung durch Medien; Optisch klare Stoffe, Größen, Formelzeichen und Einheiten"; Ausgabedatum: 1972-06) als dekadisches Absorptionsmaß bezeichnet werden.

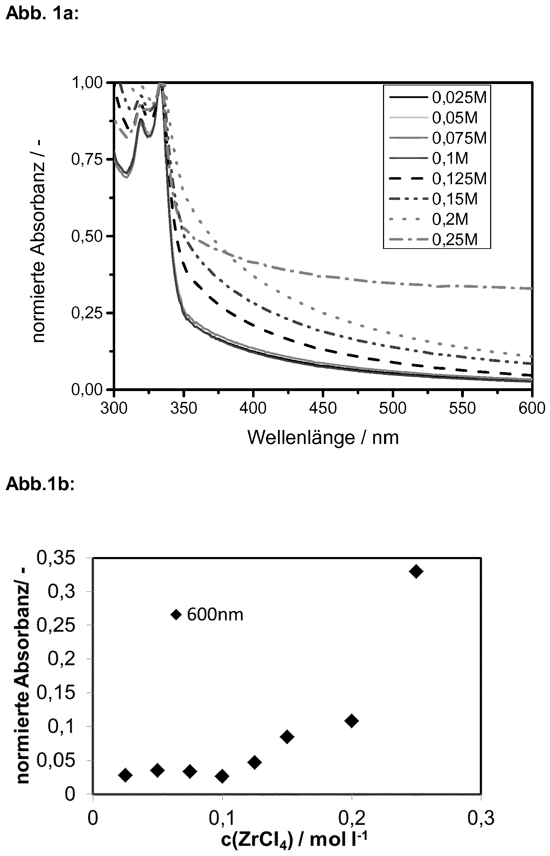

In Abb. 1a sind die gemessenen Absorptionsspektren dargestellt, wobei jeweils die Konzentration der verwendeten ZrCl₄-Lösung angegeben ist, mit der die entsprechenden Suspensionen hergestellt wurde. Wie in Abb. 1a zu sehen ist, weist das Absorptionsspektrum jeweils einen maximalen Absorptionspeak bei 333 nm auf.

Die Extinktion bei 600 nm wurde zur Bewertung eines stabilisierenden Effektes der Übergangsmetall-Kationen herangezogen. Eine Zunahme der Extinktion bei 600 nm kann auf eine verstärkte Streuung des eingestrahlten Lichtes durch agglomerierte bzw. flokkulierte Naproxen-Partikel zurückgeführt werden.

Abb.1b zeigt einen Ausschnitt des in Abb. 1a dargestellten Absorptionsspektrums, wobei ein Vergleich der jeweils gemessenen Extinktion bei 600 nm in Abhängigkeit der eingesetzten ZrCl₄-Konzentration dargestellt ist. Wie in Abb. 1 wurden die gemessenen Extinktions-Werte auf eine Absorbanz bei 333 nm normiert.

Wie Abb. 1b entnommen werden kann, steigt die Absorbanz bei 600 nm ab einer ZrCl₄-Konzentration von 0,1 mol/L an, wobei ab einer Konzentration von 0,25 mol/L die Zunahme der Absorbanz bei 600 nm stärker zunimmt.

Darüber hinaus wurden mit dem zur Herstellung der Suspensionen verwendeten Naproxen sowie mit ZrCl₄-stabilisierten Naproxen-Partikeln Fourier-Transform-Infrarotspektren (FTIR-Spektren) vermessen. Dazu wurden ZrCl₄-stabilisierte Naproxen-Partikel, die unter Verwendung einer ZrCl₄-Lösung mit einer ZrCl₄-Konzentration von 0,075 mol/L hergestellt wurde, durch Zentrifugation isoliert und in einem Trockenschrank bei 37°C bis zur Gewichtskonstanz getrocknet.

2 mg der getrockneten ZrCl₄-stabilisierten Naproxen-Partikel sowie 2 mg des eingesetzten Naproxen wurden jeweils mit 200 mg KBr, kommerziell bezogen von Sigma Aldrich Chemie GmbH (Kaliumbromid für die IR-Spektroskopie Uvasol^{®}, CAS Nummer 7758-02-3, Molmasse: 119.00 g/mol), mithilfe eines Mörsers vermengt. Anschließend wurde die jeweilige Mischung mit 10t Druck unter Verwendung einer Laborpresse MP150 der Firma Maassen GmbH (Reutlingen, DE) zu einem Pellet gepresst und in einem FTIR-Spektrometer Varian Excalibur FTS 3100 der Firma Agilent Technologies vermessen.

Die jeweils gemessenen IR-Spektren nach der Fourier-Transformation des Interferogramms sind in 2 dargestellt. Eine Zuordnung der Banden zeigt Tabelle 2.

**Tabelle 2: Zuordnung der identifizierten FTIR-Banden**

| Wellenzahl / cm⁻¹ | Zuordnung |
|---|---|
| 1728 | Streckschwingung des Carbonyls der Carboxylgruppe von als monomer vorliegenden Naproxen [1] |
| 1683 | Streckschwingung des über H-Brücken gebundenen Carbonyls der Carboxylgruppe von als dimer vorliegenden Naproxen [1] |
| 1550 | Asymetrische Schwingung des Carboxylats [2] |

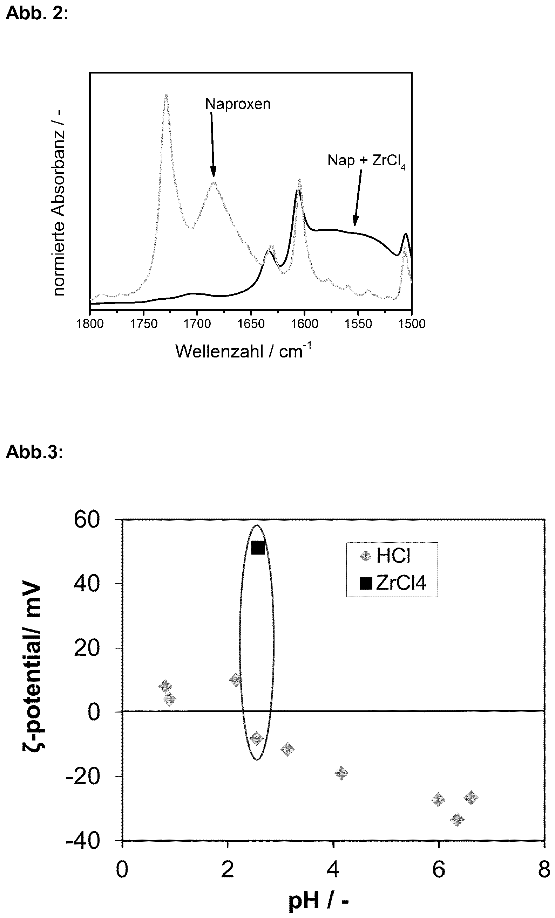

Wie in Abb. 2 zu sehen ist, liegt Naproxen in den ZrCl₄-stabilisierten Partikeln im Wesentlichen als Carboxylat vor.
[1] Paudel, A. und Van den Mooter, G.: "Influence of solvent composition on the miscibility and physical stability of naproxen / PVP K 25 solid dispersions prepared by cosolvent spray-drying", Pharm. Res. 29(1), 2012, Seiten 251 bis 270. DOI: 10.1007/s11095-011-0539-x.
[2] Hesse, M. et al.: "Spektroskopische Methoden in der organischen Chemie", 7. überarbeite Auflage, 2005, Georg Thieme Verlag, ISBN: 978-3-13-576107-7.

### Vergleichsbeispiel 2: Herstellung von Naproxen-Suspensionen mit HCl

Nach Zugabe der entsprechenden ZrCl₄-Lösung zu der Naproxen-Lösung in Beispiel 1 änderte sich der pH-Wert der jeweiligen Suspension. Die Daten der jeweils bei 25°C gemessenen pH-Werte sind in Tabelle 1 dargestellt.

Zur Überprüfung des Einflusses des pH-Wertes wurden daher Vergleichssuspensionen ohne ZrCl₄-Zugabe hergestellt, die einen vergleichbaren pH-Wert aufwiesen. Dazu wurden die in Beispiel 1 beschriebe Naproxen-Lösung verwendet, deren pH durch Zugabe von einer wässrigen HCl-Lösung, die von der Merck Chemicals GmbH bezogen wurde, auf die in Tabelle 3 angegebenen Werte eingestellt wurden.

Gleiche Volumenanteile der verschiedenen HCl-Lösungen (je 10 mL) und der in Beispiel 1 hergestellten Naproxen-Stammlösung (je 10 mL) wurden jeweils miteinander vermischt. Das Vermischen erfolgte manuell durch Pipettieren der Lösungen.

**Tabelle 3: pH-Werte der HCl-Lösungen vor bzw. nach dem Mischen der jeweiligen Lösungen**

| c (HCl) [mol l⁻¹] | pH vor Mischen (HCl) | pH nach Mischen (Naproxen + HCl) |
|---|---|---|
| 0,75 | 0,12 | 0,82 |
| 0,6 | 0,22 | 0,90 |
| 0,055 | 1,26 | 2,16 |
| 0,05 | 1,3 | 2,55 |
| 0,045 | 1,35 | 3,14 |
| 0,04 | 1,4 | 4,15 |
| 0,035 | 1,46 | 5,99 |
| 0,03 | 1,52 | 6,35 |
| 0,025 | 1,6 | 6,61 |

### Beispiel 3: Bestimmung des Zeta-Potentials, der Partikeldicht und des Glührückstands der in Beispiel 1 hergestellten Suspensionen mit ZrCl₄-stabilisierten Naproxen-Partikeln sowie der in Vergleichsbeispiel 2 hergestellten Vergleichssuspensionen.

Mit Aliquots der in Beispiel 1 hergestellten ZrCl₄-Suspensionen (siehe Tabelle 1) sowie der in Vergleichsbeispiel 2 hergestellten Vergleichssuspensionen ohne ZrCl₄-Zugabe (siehe Tabelle 3) wurde jeweils das Zeta-Potential der in den Suspensionen enthaltenen Naproxen-Partikel nach 1:40 Verdünnung gemessen. Dazu wurden jeweils 0,1 ml der zu vermessenden Suspension mit 4 ml Reinstwasser in ein sauberes Gefäß überführt. Vor der Entnahme der Suspension und nach der Mischung, wurde die Suspension für einige Minuten in ein Ultraschalbad gestellt.

Für die Messung wurden DTS 1070 Küvetten (gefaltete Einweg-Kapillar-Zellen), die von Malvern Instruments Ltd. bezogen wurden, verwendet.

Die Messung erfolgte in einem Malvern Zetasizers Nano ZS bei einer Temperatur von 25°C. Die jeweiligen Messungen erfolgten nach einer Standzeit in der temperierten Messkammer ("Equilibrationtime") von 120 s. Zwischen den Messungen wurden die Küvetten mit Reinstwasser und anschließend mit Ethanol gespült und mit Druckluft trockengeblasen.

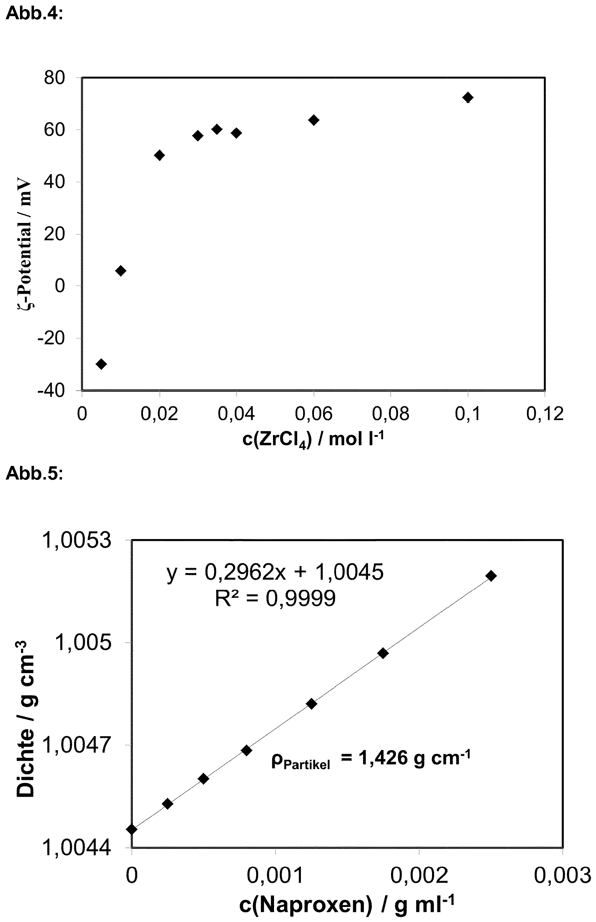

Die Daten sind in Tabelle 4 und 5 sowie in Abb. 3 und in Abb. 4 dargestellt.

Abb. 3 zeigt die in Beispiel 3 bestimmten Zeta-Potentiale ("ζ-Potential") von Naproxen-Partikeln aus Vergleichssuspensionen, die ohne ZrCl₄-Zugabe in Vergleichsbeispiel 2 hergestellt wurden, in Abhängigkeit des pH-Wertes sowie von Partikeln einer Suspension, die mit einer ZrCl₄-Lösung mit einer Konzentration von 0,075 mol l⁻¹ als Antisolvent wie in Beispiel 1 beschrieben hergestellt wurde.

**Tabelle 4: Gemessene pH-Werte nach dem Mischen sowie Zeta-Potentiale der jeweiligen Naproxen-Partikel**

| c (ZrCl₄) [mol l⁻¹] | c(HCl) [mol l⁻¹] | pH nach mischen - | ζ-potential [mV] |
|---|---|---|---|
| - | 0,75 | 0,82 | 8,00 |
| - | 0,6 | 0,90 | 4,00 |
| - | 0,055 | 2,16 | 10,00 |
| - | 0,05 | 2,55 | -8,24 |
| - | 0,045 | 3,14 | -11,57 |
| - | 0,04 | 4,15 | -19,00 |
| - | 0,035 | 5,99 | -27,34 |
| - | 0,03 | 6,35 | -33,57 |
| - | 0,025 | 6,61 | -26,70 |
| 0,075 | - | 2,58 | 51,13 |

Tabelle 4 und Abb. 3 zeigt jeweils die gemessenen pH-Werte der nach dem Mischen der entsprechenden HCl- bzw. ZrCl₄-Lösungen mit der verwendeten Naproxen-Stammlösung gebildeten Suspensionen sowie das gemessene Zeta-Potential der in den Suspensionen enthaltenen Naproxen-Partikel.

Wie in Tabelle 4 und Abb. 3 zu sehen ist, weist die mit einer ZrCl₄-Lösung (c (ZrCl₄) = 0,075 mol l⁻¹) hergestellte Suspension (pH-Wert = 2,58) einen annähernd vergleichbaren pH-Wert wie eine mit einer HCl-Konzentration von 0,05 mol l⁻¹ hergestellte Suspension (pH-Wert= 2,55) auf.

Die mit der entsprechenden ZrCl₄-Lösung hergestellte Suspension weist jedoch ZrCl₄-stabilisierte Naproxen-Partikel (ζ-potential = 51,13 mV) auf, die ein signifikant höheres positives Zeta-Potential aufweisen, als die in der Vergleichssuspension enthaltenen Naproxen-Partikel (ζ-potential = -8,24 mV), die lediglich mit der entsprechenden HCl-Konzentration von 0,05 mol l⁻¹ hergestellt wurden.

**Tabelle 5: Gemessene Zeta-Potentiale von mit der jeweils angegebenen ZrCl₄-Konzentration hergestellten ZrCl₄-stabilisierten Naproxen-Partikeln.**

| c (ZrCl₄) [mol l⁻¹] | ζ-potential [mV] |
|---|---|
| 0,005 | -30,0 |
| 0,01 | 5,7 |
| 0,02 | 50,2 |
| 0,03 | 57,7 |
| 0,035 | 60,1 |
| 0,04 | 58,7 |
| 0,06 | 63,8 |
| 0,1 | 72,4 |

Wie in Tabelle 5 und Abb. 4 zu sehen ist, führt eine Erhöhung der ZrCl₄-Konzentration der verwendeten ZrCl₄-Lösung in einem Bereich von 0,02 mol/L bis 0,1 mol/L zu einer signifikanten Erhöhung des Zeta-Potentials.

Weiterhin wurde das spezifische Volumen von ZrCl₄-stabilisierten Naproxen-Partikeln und von nichtstabilisierte Naproxen-Partikeln in Anlehnung an das in Kratky, O. et al. beschriebenen Verfahren unter Verwendung eines Biegeschwingers bestimmt und daraus jeweils die Dichte der Partikel berechnet,

Das spezifische Volumen der Naproxen-Partikel wurde über das Kratky-Dichte-Gleichgewicht mit Hilfe eines U-förmigen Biegeschwingers bestimmt. Hierbei wurde eine bekannte Masse von ZrCl₄-stabilisierten Naproxen-Partikeln und von nichtstabilisierten Naproxen-Partikeln mit verschiedenen Volumina einer Partikel-freien Stammlösung verdünnt und die Dichte der jeweiligen Suspensionen bestimmt. Aus der sich so ergebenden Gerade ließ sich das spezifische Volumen der Partikeln und hieraus wiederrum die Dichte der Partikel bestimmen.

In Abb. 5 ist die ermittelte Messkurve in Abhängigkeit der Naproxen-Konzentration dargestellt.

Die ermittelte Partikel-Dichte der mit dem erfindungsgemäßen Verfahren hergestellten stabilisierten Naproxen-Partikeln betrug 1,426 g/ml, was einer Erhöhung um 0,229 g/ml im Vergleich zur reinen, protonierten Form von Naproxen (1,197 g/ml) entsprach.

Für eine Bestimmung des Glührückstandes wurden Aliquots der vorliegend hergestellten Naproxen-Suspensionen zentrifugiert und die enthaltenen nanoskaligen Naproxen- Partikel vom Überstand getrennt. Nachfolgend wurden die isolierten Naproxen- Partikel in einem Trockenschrank bis zur Massekonstanz bei 100°C getrocknet.

Die organischen Bestandteile der getrockneten Partikel wurden durch Erhitzen auf 500°C für 2 h im Muffelofen entfernt und der Glührückstand durch Auswägen der zurückbleibenden Aschebestandteile ermittelt. Der Anteil der organischen Bestandteile betrug jeweils etwa 89,8 Gew.-%, jeweils bezogen auf das Gewicht der getrockneten Partikel. Dies entspricht einem Anteil von anorganischen Bestandteilen von etwa 10,2 Gew.-%, jeweils bezogen auf das Gewicht der getrockneten Partikel.

Die signifikant höhere Partikel-Dichte der mit dem erfindungsgemäßen Verfahren hergestellten stabilisierten Naproxen-Partikel sowie der Anteil an anorganischen Bestandteilen im Glührückstand zeigen, dass Zirkonium-Kationen und/oder Hydroxyl-Komplexe davon eine starke Interaktion mit funktionellen Gruppen von Naproxen eingehen und auch nach dem Abtrennen der Partikeln aus der Suspension noch an den Partikeln gebunden vorliegen.

### Beispiel 4: Steuerung der Partikelgröße von stabilisierten Naproxen-Partikeln.

Um den Einfluss der ZrCl₄-Konzentration auf die Partikelgröße der hergestellten Naproxen-Partikel nach Mischen zu bestimmen, wurde, wie vorstehend in Beispiel 1 beschrieben, die Naproxen-Konzentration bei der Herstellung der Suspension konstant gehalten, wobei unterschiedliche ZrCl₄-Konzentration verwendet wurden.

Die Zusammensetzung der verwendeten ZrCl₄-Lösungen ist in Tabelle 6a angegeben.

Die in Tabelle 6a angegebenen ZrCl₄-Lösungen wurden jeweils durch Verdünnung einer 1 molaren ZrCl₄-Stammlösung mit Reinstwasser wie in Beispiel 1 beschrieben hergestellt.

**Tabelle 6a: Verwendete ZrCl₄-Lösungen**

| Konzentration ZrCl₄ | Herstellung durch |
|---|---|
| 0,025 mol/L | Verdünnen von 75 µL ZrCl₄-Stammlösung |
| 0,05 mol/L | Verdünnen von 150 µL ZrCl₄-Stammlösung |
| 0,075 mol/L | Verdünnen von 225 µL ZrCl₄-Stammlösung |
| 0,1 mol/L | Verdünnen von 300 µL ZrCl₄-Stammlösung |
| 0,125 mol/L | Verdünnen von 375 µL ZrCl₄-Stammlösung |

Weiterhin wurde die in Beispiel 1 hergestellte Naproxen-Stammlösung mit einem Naproxen-Gehalt von 5 mg ml⁻¹ verwendet.

Gleiche Volumenanteile der verschiedenen ZrCl₄-Lösungen (je 3 mL) und der Naproxen-Stammlösung (je 3 mL) wurden jeweils miteinander vermischt. Das Vermischen erfolgte unter Verwendung eines statischen Mischers.

Bei dem verwendeten statischen Mischer handelte es sich um einen Drei-Wege-T-Mischer mit zwei gegenüberliegend angeordneten Zuläufen und einem orthogonal dazu angeordneten Auslass. Die Zuläufe bestanden aus 5 mm langen rechteckigen Kanälen mit 1 mm Kantenlänge. Der Auslass bestand aus einem 12 mm langen Kanal, welcher 2 mm breit und 1 mm hoch war. Der verwendete Gesamtvolumenstrom betrug 4 ml s⁻¹.

Die Messung der Partikelgröße sowie deren Verteilung erfolgte unter Verwendung der dynamischen Lichtstreuung (DLS) mittels eines Malvern Zetasizers Nano ZS bei einer Temperatur von 25°C. Die jeweiligen Messungen erfolgten nach einer Standzeit ("Equilibrationtime") von 120 s.

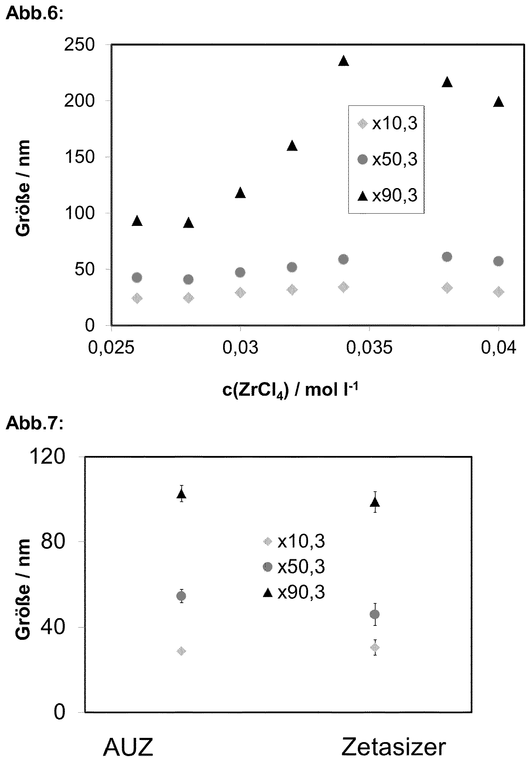

Die Ergebnisse der Partikelgrößen-Bestimmung sind in Tabelle 6b und Abb. 6 dargestellt.

**Tabelle 6b: Ergebnis der Partikelgrößenbestimmung**

| c(ZrCl₄) [mol l⁻¹] | x_{10,3} [nm] | x_{50,3} [nm] | x_{90,3} [nm] |
|---|---|---|---|
| 0,025 | 43 | 76 | 623 |
| 0,05 | 18 | 32 | 98 |
| 0,075 | 33 | 55 | 129 |
| 0,1 | 46 | 79 | 211 |
| 0,125 | 78 | 150 | 378 |

Tabelle 6b und Abb. 6 zeigen die gemessene Partikelgrößenverteilung der in den jeweiligen Suspensionen enthaltenen Naproxen-Partikel in Abhängigkeit der eingesetzten ZrCl₄-Konzentration bei Verwendung eines T-Mischers.

Bei Verwendung von ZrCl₄-Lösungen mit einer ZrCl₄-Konzentration von kleiner als 0,02 mol/L bzw. mehr als 0,15 mol/L war eine Bestimmung der Partikelgröße sowie deren Verteilung durch dynamische Lichtstreuung aufgrund der Instabilität der jeweiligen Suspensionen nicht möglich.

Alternativ erfolge die Messung der Partikelgröße durch analytische Ultrazentrifugation (AUZ) in Anlehnung an das in Cölfen, H. et al. ("The Open AUC Project", Eur. Biophys. J. 39(3), 2010, Seiten 347 bis 359, DOI: 10.1007/s00249-009-0438-9) beschriebene Verfahren.

Die Sedimentationseigenschaften der Naproxen-Partikel wurden über eine Analytische Ultrazentrifuge mit Multiwellenlängendektor (MWL-AUZ) spektroskopisch als Funktion von Zeit und Ort erfasst. Aus den daraus gewonnenen Sedimentationskoeffizientenverteilungen wurden sedimentationsäquivalente Partikelgrößenverteilungen berechnet.

Geeignete Ultrazentrifugen mit Multiwellenlängendektor auf Basis der Entwicklungen des Open AUC Projekts werden ebenfalls in Cölfen, H. et al. beschrieben.

Ein Vergleich der mittels analytischer Ultrazentrifugation ("AUZ") bzw. unter Verwendung der dynamischen Lichtstreuung mit einem Malvern Zetasizers Nano ZS ("Zetasizer") ermittelten Partikelgrößenverteilung ist in Abb. 7 dargestellt, wobei zur Herstellung der Suspension eine Mischung aus 3 mL der in Tabelle 6a angegebenen ZrCl₄-Lösung mit einer ZrCl₄-Konzentration von 0,075 mol/L und 3 mL der oben angegebenen Naproxen-Stammlösung verwendet wurden.

Die entsprechenden Lösungen wurden manuell durch Pipettieren gemischt. Anschließend wurde die entstandene Suspension geteilt und die Partikelgrößen x10,3, x50,3 und x90,3 der jeweils enthaltenen ZrCl₄-stabilisierten Naproxen-Partikel entweder mit Hilfe einer analytischen Ultrazentrifuge (Abb. 7 "AUZ") oder unter Verwendung der dynamischen Lichtstreuung (Abb. 7 "Zetasizer") bestimmt.

Die mit beiden Methoden ermittelten Partikelgrößen stimmen im Rahmen der Vergleichbarkeit der beiden Messmethoden gut überein.

### Beispiel 5: Herstellung von stabilisierten Ibuprofen-Suspensionen mit ZrCl₄.

Als Ausgangssubstanz wurde kommerziell erhältliches Ibuprofen [(±)-α-Methyl-4-(isobutyl)phenylessigsäure, CAS-Nr.: 15687-27-1] der Firma TCI Deutschland GmbH (Eschborn, DE) (Molare Masse: 206,28 g/mol, Gehalt gemäß Herstellerangabe: ≥ 98%) ohne weitere Reinigung verwendet. Ibuprofen weist die in Formel (2) dargestellte Struktur auf:

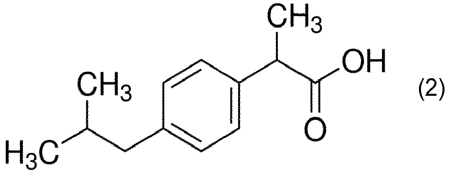

Weiterhin wurde wasserfreies Zirkoniumtetrachlorid, NaOH sowie Reinstwasser aus den in Beispiel 1 angegebenen Bezugsquellen ohne weitere Reinigung verwendet.

Die Herstellung der stabilisierten Ibuprofen-Suspensionen erfolgte durch Verwendung von pH-Verschiebung oder Anti-Solvent-Ausfällung.

Für die Herstellung von stabilisierten Ibuprofen-Suspensionen durch pH-Verschiebung wurde zunächst eine Ibuprofen-Stammlösung mit einem Ibuprofen-Gehalt von 4,417 mg/mL durch lösen von 4,417 g Ibuprofen in 1 L einer wässrigen NaOH-Lösung (Konzentration: 0,04 mol/L) hergestellt.

Weiterhin wurden eine ZrCl₄-Stammlösung mit eine Konzentration von 1 mol/L durch Lösen von 233,04 g ZrCl₄ in 1 L Reinstwasser hergestellt.

Verschiedene ZrCl₄-Lösungen mit den in Tabelle 7a angegebenen Konzentrationen wurden durch Verdünnen der entsprechenden Mengen an ZrCl₄-Stammlösung auf ein Endvolumen von 10 mL hergestellt.

**Tabelle 7a: Verwendete ZrCl₄-Lösungen**

| Konzentration ZrCl₄ | Herstellung durch |
|---|---|
| 0,025 mol/L | Verdünnen von 250 µL ZrCl₄-Stammlösung |
| 0,05 mol/L | Verdünnen von 500 µL ZrCl₄-Stammlösung |
| 0,075 mol/L | Verdünnen von 750 µL ZrCl₄-Stammlösung |
| 0,1 mol/L | Verdünnen von 1000 µL ZrCl₄-Stammlösung |
| 0,125 mol/L | Verdünnen von 1250 µL ZrCl₄-Stammlösung |
| 0,15 mol/L | Verdünnen von 1500 µL ZrCl₄-Stammlösung |
| 0,175 mol/L | Verdünnen von 1750 µL ZrCl₄-Stammlösung |

Gleiche Volumenanteile der verschiedenen ZrCl₄-Lösungen (je 10 mL) und der Ibuprofen-Stammlösung (je 10 mL) wurden jeweils miteinander gemischt. Das Mischen erfolgte manuell durch Pipettieren der Lösungen oder durch Verwendung eines statischen Mischers, wie in Beispiel 4 beschrieben.

Durch den Wechsel des pH-Wertes von basisch zu sauer während des Mischens bildeten sich nanoskalige Ibuprofen-Partikel, an deren Oberfläche zumindest Zirkonium-Ionen und/oder Hydroxid-Komplexe davon gebunden waren.

Für die Herstellung von stabilisierten Ibuprofen-Suspensionen durch Anti-Solvent-Ausfällung wurde zunächst eine ethanolische Ibuprofen-Stammlösung mit einem Ibuprofen-Gehalt von 20 mg/mL durch Lösen von 20 g Ibuprofen in 1 L Ethanol (absolut, Reag. Ph Eur.), bezogen von der Firma TCI Deutschland GmbH, bereitgestellt.

Verschiedene ZrCl₄-Lösungen mit den in Tabelle 4 angegebenen Konzentrationen wurden durch Verdünnen der entsprechenden Mengen der oben angegebenen ZrCl₄-Stammlösung auf ein Endvolumen von 35 mL hergestellt.

**Tabelle 7b: Verwendete ZrCl₄-Lösungen**

| Konzentration ZrCl₄ | Herstellung durch |
|---|---|
| 0 mol/L | Reinstwasser |
| 0,01 mol/L | Verdünnen von 0,35 mL ZrCl₄-Stammlösung |
| 0,02 mol/L | Verdünnen von 0,7 mL ZrCl₄-Stammlösung |
| 0,03 mol/L | Verdünnen von 1,05 mL ZrCl₄-Stammlösung |
| 0,045 mol/L | Verdünnen von 1,575 mL ZrCl₄-Stammlösung |
| 0,06 mol/L | Verdünnen von 2,1 mL ZrCl₄-Stammlösung |
| 0,08 mol/L | Verdünnen von 2,8 mL ZrCl₄-Stammlösung |
| 0,1 mol/L | Verdünnen von 3,5 mL ZrCl₄-Stammlösung |

Die Volumenanteile der ethanolischen Ibuprofen-Stammlösung (je 7 mL) und der verschiedenen ZrCl₄-Lösungen (je 35 mL) wurden im Verhältnis von 1 : 5 gemischt. Das Mischen erfolgte durch Verwendung eines statischen Mischers, wie in Beispiel 4 beschrieben. Der verwendete Gesamtvolumenstrom war 3 ml s⁻¹.

Die Messung der Partikelgröße sowie deren Verteilung sowie des Zeta-Potentials der in durch beide Methoden hergestellten Ibuprofen-Suspensionen enthaltenen ZrCl₄-stabilisierten Ibuprofen-Partikel erfolgte mittels eines Malvern Zetasizers Nano ZS bei einer Temperatur von 25°C wie oben in Beispiel 3 und 4 beschrieben nach einer Standzeit von 120 s.

Die Ergebnisse sind in Tabelle 7c sowie in den Abb. 8 und 9b dargestellt.

**Tabelle 7c: Ergebnis der Partikelgrößenbestimmung**

| c(ZrCl₄) [mol l⁻¹] | X10,3 [nm] | x_{50,3} [mol l⁻¹] | x_{90,3} [nm] | Spanne Δx - |
|---|---|---|---|---|
| 0,02 | 41 | 65 | 134 | 1,44 |
| 0,05 | 33 | 52 | 109 | 1,44 |
| 0,075 | 36 | 58 | 116 | 1,37 |
| 0,1 | 47 | 69 | 113 | 0,95 |
| 0,125 | 56 | 80 | 124 | 0,86 |
| 0,15 | 72 | 116 | 220 | 1,28 |
| 0,175 | 97 | 160 | 277 | 1,13 |

Tabelle 7c und Abb. 8 zeigt jeweils die gemessenen Parameter x10,3, x50,3 und x90,3 der volumenbezogenen Partikelgrößenverteilung der jeweils in den hergestellten Suspensionen enthaltenen ZrCl₄-stabilisierten Ibuprofen-Partikel nach manuellem Mischen mit Hilfe einer Pipette. In Tabelle 7c ist darüber hinaus die aus den gemessenen Parametern x10,3, x50,3 und x90,3 berechnete Spanne Δx der Partikelgrößenverteilung angegeben.

Wie aus Abb. 8 entnommen werden kann, wiesen die mit einer ZrCl₄-Konzentration aus einem Bereich von 0,05 mol/L bis 0,125 mol/L hergestellten Ibuprofen-Partikel eine Partikelgrößenveteilung auf, bei der 90 % aller Partikel kleiner oder gleich 124 nm waren.

Abb. 9a zeigt die einzelnen Summenverteilungen (Q₃) der volumenbezogenen Äquivalentdurchmesser von 3 unabhängigen Messungen an ZrCl₄-stabilisierten Ibuprofen-Partikeln einer Suspension, die durch Vermischen einer 0,04 mol/L NaOH-Lösung mit darin gelösten 4,47 mg ml⁻¹ Ibuprofen und einer ZrCl₄-Lösung (ZrCl4-Konzentration: 0,04 mol/L) in einem statische Mischer hergestellt wurde.

Wie in Abb. 9a zu sehen ist, war die gesamte Partikelverteilung sehr eng und es kam nicht, z.B. ab Q₃ (x)= 0,95, zu einem ausgeprägten Tailing durch unstabiliserte Ibuprofen-Partikel.

Um den Einfluss der ZrCl₄-Konzentration zu untersuchen, wurde die oben hergestellte ethanolische Ibuprofen-Stammlösung in einem statischen Mischer jeweils wie oben angegeben im Verhältnis 1 : 5 mit wässrigen Lösungen, die eine unterschiedliche ZrCl₄-Konzentration aufwies, gemischt.

Die Messung der Partikelgröße sowie deren Verteilung der in den hergestellten Ibuprofen-Suspensionen enthaltenen ZrCl₄-stabilisierten Ibuprofen-Partikel erfolgte mittels eines Malvern Zetasizers Nano ZS bei einer Temperatur von 25°C, wie oben in Beispiel 3 und 4 beschrieben.

Die Ergebnisse sind in Abb. 9b dargestellt, wobei jeweils die gemessenen Größen x10,3, x50,3 und x90,3 der Partikelgrößenverteilung in Abhängigkeit der jeweils verwendeten ZrCl₄-Konzentration angegeben ist.

Darüber hinaus wurden mit dem verwendeten Ibuprofen (Abb. 10 "Bulk") sowie mit hergestellten ZrCl₄-stabilisierten Ibuprofen-Partikeln Fourier-Transform-Infrarotspektren (FTIR-Spektren) vermessen. Dazu wurden ZrCl₄-stabilisierte Ibuprofen-Partikel wie in Beispiel 1 beschrieben durch Zentrifugation isoliert und in einem FTIR-Spektrometer vermessen.

Sowohl durch Anti-Solvent-Ausfällung (Abb. 10 "Anti-Solvent") als auch durch pH-Verschiebung (Abb. 10 "pH-Shift") hergestellte ZrCl₄-stabilisierte Ibuprofen-Partikel wurden durch Zentrifugation isoliert und in einem Trockenschrank bei 50°C bis zur Gewichtskonstanz getrocknet.

Für die Anti-Solvent-Ausfällung wurde die oben beschriebene ethanolische Ibuprofen-Lösung (Ibuprofen-Konzentration: 20 mg/mL in Ethanol) im Verhältnis 1:5 mit einer ZrCl₄-Lösung (Konzentration 0,02 mol/L ZrCl₄ in Reinstwasser) vermischt.

Für die pH-Verschiebung wurde die oben beschriebene Ibuprofen-Lösung (Ibuprofen-Konzentration: 4,417 mg/mL in 0,04 mol/L NaOH-Lösung) im Verhältnis 1:5 mit einer ZrCl₄-Lösung (Konzentration 0,04 mol/L ZrCl₄ in Reinstwasser) vermischt.

Die jeweils gemessenen IR-Spektren nach der Fourier-Transformation des Interferogramms sind in Abb. 10 dargestellt. Eine Zuordnung der Banden zeigt Tabelle 7d.

**Tabelle 7d: Zuordnung der identifizierten FTIR-Banden**

| Wellenzahl / cm⁻¹ | Zuordnung |
|---|---|
| 1720 | Streckschwingung des Carbonyls der Carboxylgruppe [3] |
| 1560 | Asymetrische Schwingung des Carboxylats [4] |

Wie in Abb. 10 zu sehen ist, liegt Ibuprofen in den ZrCl₄-stabilisierten Partikeln, die sowohl durch Anti-Solvent-Ausfällung ("Anti-Solvent") als auch durch pH-Verschiebung ("pH-Shift") hergestellt wurden, im Wesentlichen als Carboxylat vor.
[3] Jubert, A. et al.: "Vibrational and theoretical studies of non-steroidal anti-inflammatory drugs Ibuprofen [2-(4-isobutylphenyl)propionic acid]; Naproxen [6-methoxy-α-methyl-2-naphthalene acetic acid] and Tolmetin acids [1-methyl-5-(4-methylbenzoyl)-1H-pyrrole-2-acetic acid]", J. Mol. Struc. 783 (1-3), 2006, Seiten 34 bis 51, DOI: 10.1016/j.molstruc.2005.08.018
[4] Zordok, W.A. et al.:"Spectroscopic, thermal analysis, and antimicrobial evaluation of new Y(III), Zr(IV), and U(VI) ibuprofen complexes", J. Coord. Chem. 65 (2), 2012, Seiten 353 bis 369, DOI: 10.1080/00958972.2011.654203

Um den Einfluss des verwendeten organischen Lösungsmittels bei Anti-Solvent-Ausfällung zu überprüfen, wurden Ibuprofen-Stammlösung mit einem Ibuprofen-Gehalt von 30 mg/mL durch Lösen von 30 g Ibuprofen in 1 L Methanol, Ethanol oder 2-Propanol (jeweils absolut, Reag. Ph Eur.), bezogen von der Firma TCI Deutschland GmbH, hergestellt.

Eine ZrCl₄-Lösung mit einer Konzentration von 0,015 wurde durch Verdünnen der entsprechenden Mengen der oben angegebenen ZrCl₄-Stammlösung auf ein Endvolumen von 35 mL verdünnt.

Die Volumenanteile der Ibuprofen-Stammlösung (je 3,5 mL) und der verwendeten ZrCl₄-Lösungen (je 35 mL) wurden im Verhältnis von 1 : 10 gemischt. Das Mischen erfolgte durch Verwendung eines statischen Mischers, wie in Beispiel 4 beschrieben. Der verwendete Gesamtvolumenstrom war 3,03 ml s⁻¹.

Die Messung der Partikelgröße sowie deren Verteilung sowie des Zeta-Potentials der in durch beide Methoden hergestellten Ibuprofen-Suspensionen enthaltenen ZrCl₄-stabilisierten Ibuprofen-Partikel erfolgte mittels eines Malvern Zetasizers Nano ZS bei einer Temperatur von 25°C wie oben in Beispiel 3 und 4 beschrieben nach einer Standzeit von 120 s.

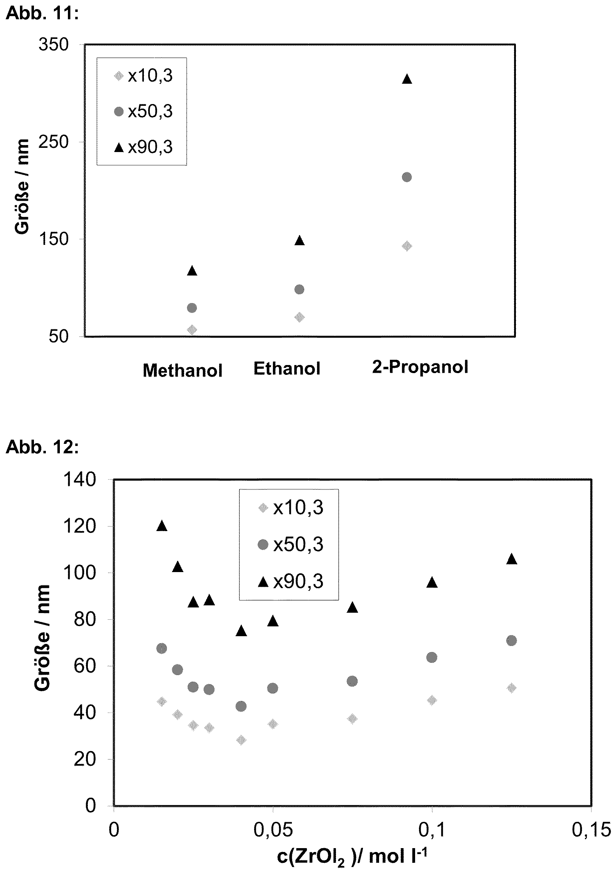

Die Ergebnisse sind in Abb. 11 dargestellt.

Während die Verwendung von Ethanol und Methanol, die bei einer Temperatur von 25°C ähnliche Diffusionskoeffizienten in Wasser aufweisen, in vergleichbaren Partikelgrößenverteilungen resultieren, weisen die Partikel bei der Nutzung von 2-Propanol, das in Wasser bei einer Temperatur von 25°C einen geringeren Diffusionskoeffizienten als Methanol und Ethanol aufweist, eine breitere Verteilung der Partikelgröße auf.

Die Löslichlichkeit von Ibuprofen ist bei 20°C in den verwendeten Lösungsmitteln Methanol, Ethanol und 2-Propanol vergleichbar hoch, siehe Gracin, S. und. Rasmuson, Å. C. ("Solubility of Phenylacetic Acid, p-Hydroxyphenylacetic Acid, p-Aminophenylacetic Acid, p-Hydroxybenzoic Acid, and Ibuprofen in Pure Solvents", J. Chem. Eng. Data 47 (6), 2002, Seiten 1379 bis 1383, DOI: 10.1021/je0255170).

Dichte und Viskosität von binären Mischungen von 2-Propanol und Wasser können beispielsweise aus Pang, F.M. et al. ("Densities and viscosities of aqueous solutions of 1-propanol and 2-propanol at temperatures from 293.15 Kto 333.15 K", J. Mol. Liq. 136 (1-2), 2007, Seiten 71 bis 78, DOI: 10.1016/j.molliq.2007.01.003) entnommen werden. Dichte und Viskosität von binären Mischungen von Methanol und Wasser bzw. Ethanol und Wasser können beispielsweise Dizechi, M. und Marschall, E. ("Viscosity of some binary and ternary liquid mixtures", J. Chem. Eng. Data27 (3), 1982, Seiten 358 bis 363, DOI: 10.1021/je00029a039) entnommen werden.

### Beispiel 6: Herstellung von stabilisierten Ibuprofen-Suspensionen mit Zirkonylchlorid

Als Ausgangssubstanz wurde wie in Beispiel 5 kommerziell erhältliches Ibuprofen der Firma Sigma-Aldrich Chemie GmbH ohne weitere Reinigung verwendet.

Für die Herstellung von stabilisierten Ibuprofen-Suspensionen durch Anti-Solvent-Ausfällung wurde zunächst eine ethanolische Ibuprofen-Stammlösung mit einem Ibuprofen-Gehalt von 40 mg/mL durch Lösen von 40 g Ibuprofen in 1 L Ethanol, das von der in Beispiel 5 angegebenen Quelle bezogen wurde, gelöst.

Weiterhin wurde Zirkon(IV)oxidchlorid Octahydrat [Zirkonylchlorid, ZrOCl₂ * 8 H₂O, CAS Nr.: 13520-92-8] der Merck Chemicals GmbH (Molare Masse: 322,28 g/mol, Gehalt gemäß Herstellerangabe: ≥ 99%) und Reinstwasser aus den in Beispiel 1 angegebenen Bezugsquellen ohne weitere Reinigung verwendet.

Weiterhin wurden eine ZrOCl₂-Stammlösung mit eine Konzentration von 0,5 mol/L durch Lösen von 161,14 g ZrOCl₂ * 8 H₂O in 1 L Reinstwasser hergestellt.

Verschiedene ZrOCl₂-Lösungen mit den in Tabelle 8a angegebenen Konzentrationen wurden durch Verdünnen der entsprechenden Mengen der oben angegebenen ZrOCl₂-Stammlösung auf ein Endvolumen von 40 mL hergestellt.

**Tabelle 8a: Verwendete ZrOCl₂-Lösungen**

| Konzentration ZrOCl₂ | Herstellung durch |
|---|---|
| 0,015 mol/L | Verdünnen von 1,2 mL ZrOCl₂-Stammlösung |
| 0,02 mol/L | Verdünnen von 1,6 mL ZrOCl₂-Stammlösung |
| 0,025 mol/L | Verdünnen von 2 mL ZrOCl₂-Stammlösung |
| 0,03 mol/L | Verdünnen von 2,4 mL ZrOCl₂-Stammlösung |
| 0,04 mol/L | Verdünnen von 3,2 mL ZrOCl₂-Stammlösung |
| 0,05 mol/L | Verdünnen von 4 mL ZrOCl₂-Stammlösung |
| 0,075 mol/L | Verdünnen von 6 mL ZrOCl₂-Stammlösung |
| 0,1 mol/L | Verdünnen von 8 mL ZrOCl₂-Stammlösung |
| 0,125 mol/L | Verdünnen von 10 mL ZrOCl₂-Stammlösung |

Die Volumenanteile der ethanolischen Ibuprofen-Stammlösung (je 1 mL) und der verschiedenen ZrOCl₂-Lösungen (je 10 mL) wurden im Verhältnis von 1 : 10 gemischt. Das Mischen erfolgte durch Verwendung eines statischen Mischers, wie in Beispiel 4 beschrieben.

Die Messung der Partikelgröße und deren Verteilung der in den hergestellten Ibuprofen-Suspensionen enthaltenen ZrOCl₂-stabilisierten Ibuprofen-Partikel erfolgte mittels eines Malvern Zetasizers Nano ZS bei einer Temperatur von 25°C wie oben in Beispiel 3 und 4 beschrieben nach einer Standzeit von 120 s.

Die Ergebnisse sind in Tabelle 8b sowie Abb. 12 dargestellt, wobei jeweils die gemessenen Größen x10,3, x50,3 und x90,3 der Partikelgrößenverteilung nach Mischen mit Hilfe einem statischen Mischer in Abhängigkeit von der verwendeten ZrOCl₂-Konzentration angegeben sind.

**Tabelle 8b: Ergebnis der Partikelgrößenbestimmung**

| c(ZrOCl₂) [mol l⁻¹] | X_{10,3} [nm] | x_{50,3} [nm] | x_{90,3} [nm] | Spanne Δx [-] | pH-Wert [-] |
|---|---|---|---|---|---|
| 0,015 | 45 | 67 | 120 | 1,12 | 1,96 |
| 0,02 | 39 | 58 | 103 | 1,09 | 1,85 |
| 0,025 | 34 | 51 | 88 | 1,05 | 1,81 |
| 0,03 | 33 | 50 | 88 | 1,10 | 1,77 |
| 0,04 | 28 | 43 | 75 | 1,11 | 1,71 |
| 0,05 | 35 | 50 | 79 | 0,88 | 1,65 |
| 0,075 | 37 | 53 | 85 | 0,90 | 1,54 |
| 0,1 | 45 | 64 | 96 | 0,80 | 1,45 |
| 0,125 | 51 | 71 | 106 | 0,78 | 1,39 |

Tabelle 8b zeigt die gemessenen Parameter x10,3, x50,3 und x90,3 der volumenbezogenen Partikelgrößenverteilung sowie die daraus berechnete Spanne Δx der jeweils in den hergestellten Suspensionen enthaltenen ZrOCl₂-stabilisierten Ibuprofen-Partikel sowie den gemessenen pH-Wert der Suspension nach Mischen mit Hilfe eines statischen Mischers.

Wie in Abb. 12 zu sehen ist, weisen die mit ZrOCl₂ stabilisierten Suspensionen Ibuprofen-Partikel mit einer Größe auf, die 150 nm nicht überschreitet. Weiterhin weisen die Partikel eine enge Partikelgrößenverteilung auf.

### Beispiel 7: Herstellung von stabilisierten Ibuprofen-Suspensionen mit unterschiedlichem Ibuprofen-Gehalt.

Als Ausgangssubstanz wurde wie in Beispiel 5 kommerziell erhältliches Ibuprofen der Firma TCI Deutschland GmbH ohne weitere Reinigung verwendet.

Für die Herstellung von stabilisierten Ibuprofen-Suspensionen durch Anti-Solvent-Ausfällung wurden verschiedene ethanolische Ibuprofen-Stammlösung mit einem Ibuprofen-Gehalten von 5 mg/mL, 10 mg/mL, 20 mg/mL, 30 mg/mL, 40 mg/mL und 50 mg/mL durch Lösen der entsprechenden Menge Ibuprofen in Ethanol, das von der in Beispiel 5 angegebenen Quelle bezogen wurde, gelöst.

Verschiedene ZrCl₄-Lösungen mit den in Tabelle 9 angegebenen Konzentrationen wurden durch Verdünnen der entsprechenden Mengen der oben angegebenen ZrCl₄-Stammlösung auf ein Endvolumen von 35 mL hergestellt.

Die Volumenanteile der ethanolischen Ibuprofen-Stammlösungen (je 3,5 mL) und der verschiedenen ZrCl₄-Lösungen (je 35 mL) wurden im Verhältnis von 1 : 10 gemischt. Das Mischen erfolgte durch Verwendung eines statischen Mischers, wie in Beispiel 3 beschrieben.

**Tabelle 9: Verwendete ZrCl₄-Lösungen**

| Konzentration ZrCl₄ | Herstellung durch |
|---|---|
| 0,0025 mol/L | Verdünnen von 87,5 µl ZrCl₄ - Stammlösung |
| 0,005 mol/L | Verdünnen von 175 µl ZrCl₄ - Stammlösung |
| 0,01 mol/L | Verdünnen von 350 µl ZrCl₄ - Stammlösung |
| 0,0125 mol/L | Verdünnen von 437,5 µl ZrCl₄ - Stammlösung |
| 0,015 mol/L | Verdünnen von 525 µl ZrCl₄ - Stammlösung |
| 0,0175 mol/L | Verdünnen von 612,5 µl ZrCl₄ - Stammlösung |
| 0,02 mol/L | Verdünnen von 700 µl ZrCl₄ - Stammlösung |
| 0,025 mol/L | Verdünnen von 875 µl ZrCl₄ - Stammlösung |
| 0,03 mol/L | Verdünnen von 1,05 ml ZrCl₄ - Stammlösung |
| 0,035 mol/L | Verdünnen von 1,225 ml ZrCl₄ - Stammlösung |
| 0,04 mol/L | Verdünnen von 1,4 ml ZrCl₄ - Stammlösung |
| 0,05 mol/L | Verdünnen von 1,75 ml ZrCl₄ - Stammlösung |

Die Messung der Partikelgröße und deren Verteilung der in den hergestellten Ibuprofen-Suspensionen enthaltenen ZrCl₄-stabilisierten Ibuprofen-Partikel erfolgte mittels eines Malvern Zetasizers Nano ZS bei einer Temperatur von 25°C wie oben in Beispiel 3 und 4 beschrieben nach einer Standzeit von 120 s. Die Bestimmung der Parameter x_{10,3}, x_{50,3} und x_{90,3} der Partikelgrößenverteilung erfolgte jeweils drei mal, wobei jede hergestellte Suspension jeweils ebenfalls dreimal vermessen wurde.

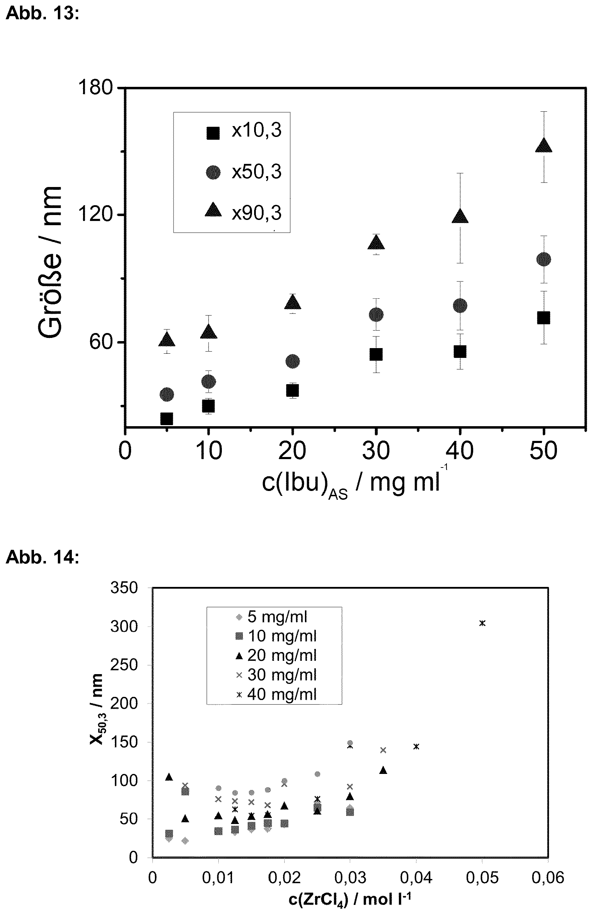

Die Ergebnisse sind in Tabelle 10 sowie in Abb. 13 und 14 dargestellt.

Abb. 13 zeigt das arithmetische Mittel der gemessenen Parameter x_{10,3}, x_{50,3} und x_{90,3} der Partikelgrößenverteilung von ZrCl₄-stabilisierten Ibuprofen-Partikeln, die in mit einer ZrCl₄-Konzentration von 0,0125 mol/L hergestellten Ibuprofen-Suspensionen enthalten waren, in Abhängigkeit der verwendeten Ibuprofen-Konzentration.

Wie aus Abb. 13 entnommen werden kann, wiesen die mit einer Ibuprofen-Konzentration aus einem Bereich von 5 mg/mL bis 50 mg/mL hergestellten Ibuprofen-Partikel eine enge Partikelgröße von deutlich unter 180 nm auf.

**Tabelle 10: Ergebnis der Partikelgrößenbestimmung**

| | x_{50,3} [nm] | | | | | |
|---|---|---|---|---|---|---|
| c (ZrCl₄) [mol/l] | 5 mg/ml Ibuprofen | 10 mg/ml Ibuprofen | 20 mg/ml Ibuprofen | 30 mg/ml Ibuprofen | 40 mg/ml Ibuprofen | 50 mg/ml Ibuprofen |
| 0,0025 | 25 nm | 31 nm | 105 nm | | | |
| 0,005 | 21 nm | 86 nm | 51 nm | 94 nm | | |
| 0,01 | 35 nm | 34 nm | 55 nm | 76 nm | | 90 nm |
| 0,0125 | 33 nm | 36 nm | 48 nm | 73 nm | 63 nm | 84 nm |
| 0,015 | 36 nm | 41 nm | 54 nm | 72 nm | 54 nm | 85 nm |
| 0,0175 | 37 nm | 45 nm | 56 nm | 68 nm | 55 nm | 88 nm |
| 0,02 | 43 nm | 44 nm | 68 nm | 96 nm | | 100 nm |
| 0,025 | 72 nm | 65 nm | 61 nm | 67 nm | 76 nm | 109 nm |
| 0,03 | 64 nm | 59 nm | 80 nm | 92 nm | 146 nm | 149 nm |
| 0,035 | | | 114 nm | 140 nm | | |
| 0,04 | | | | | 144 nm | |
| 0,05 | | | | | 305 nm | |

Tabelle 10 und Abb. 14 zeigen jeweils den gemessenen Parameter x50,3 der Partikelgrößenverteilung von ZrCl₄-stabilisierten Ibuprofen-Partikel, die in mit unterschiedlichen ZrCl₄-Konzentrationen hergestellten Ibuprofen-Suspensionen enthalten waren, in Abhängigkeit der verwendeten ZrCl₄-Konzentration.

Wie aus Abb. 13 sowie aus Tabelle 10 und Abb. 14 ersichtlich ist, steigt mit zunehmender Ibuprofen-Konzentration der zur Herstellung verwendeten Ibuprofen-Lösung die Partikelgröße der in der Suspension enthaltenen Ibuprofen-Partikel an.

### Beispiel 8: Stabilitätsuntersuchungen an in Beispiel 7 hergestellten Ibuprofen-Suspensionen.

Die in Beispiel 7 aus einer ethanolischen Ibuprofen-Lösung (Ibuprofen-Konzentration: 40 mg/ml) durch Anti-Solvent-Fällung mit einer wässrigen ZrCl₄-Lösung (ZrCl₄-Konzentration. 0,025 mol/L) hergestellte Ibuprofen-Suspension wurde für einen Zeitraum von 2 Monaten in einem verschlossenen Gefäß bei Raumtemperatur gelagert.

Die Messung der Partikelgröße und deren Verteilung der in der hergestellten Ibuprofen-Suspension enthaltenen ZrCl₄-stabilisierten Ibuprofen-Partikel direkt nach der Herstellung sowie nach 2 monatiger Lagerung erfolgte mittels eines Malvern Zetasizers Nano ZS bei einer Temperatur von 25°C wie oben in Beispiel 3 und 4 beschrieben nach einer Standzeit von 120 s.

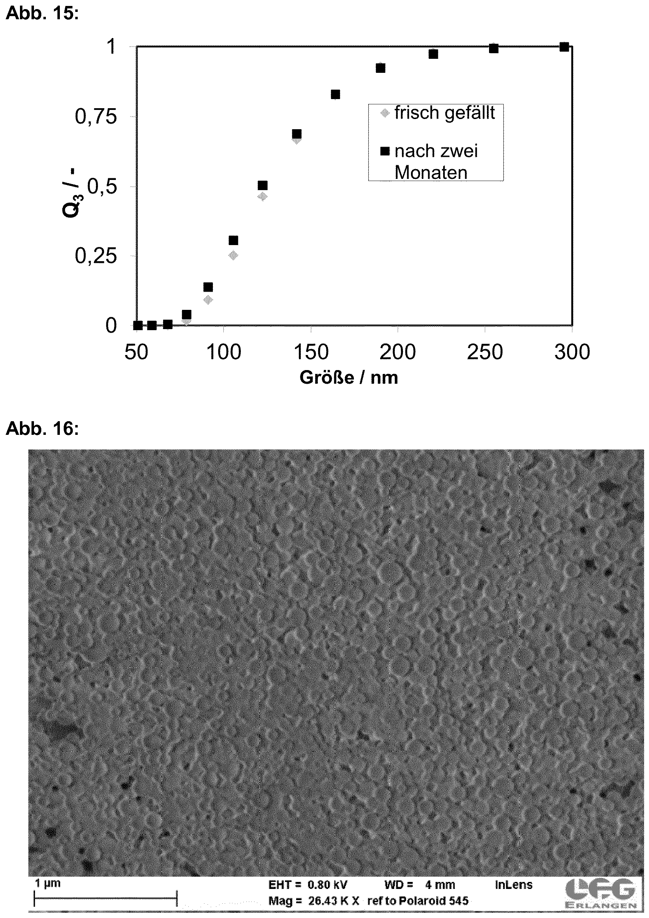

Die Ergebnisse sind in Abb. 15 dargestellt, wobei Abb. 15 jeweils die Summenverteilung (Q₃) der volumenbezogenen Äquivalentdurchmesser der in der hergestellten Suspensionen enthaltenen Ibuprofen-Partikel zeigt, die direkt nach der Herstellung bzw. nach 2 Monaten Lagerung bestimmt wurde.

Nach 2 Monaten Lagerung wurde die hergestellte Suspension mittels Rotationsbeschichtung auf Silicium-Substrate aufgebracht und getrocknet. Die entsprechenden Substrate wurden nachfolgend mittels Rasterelektronenmikroskopie untersucht.

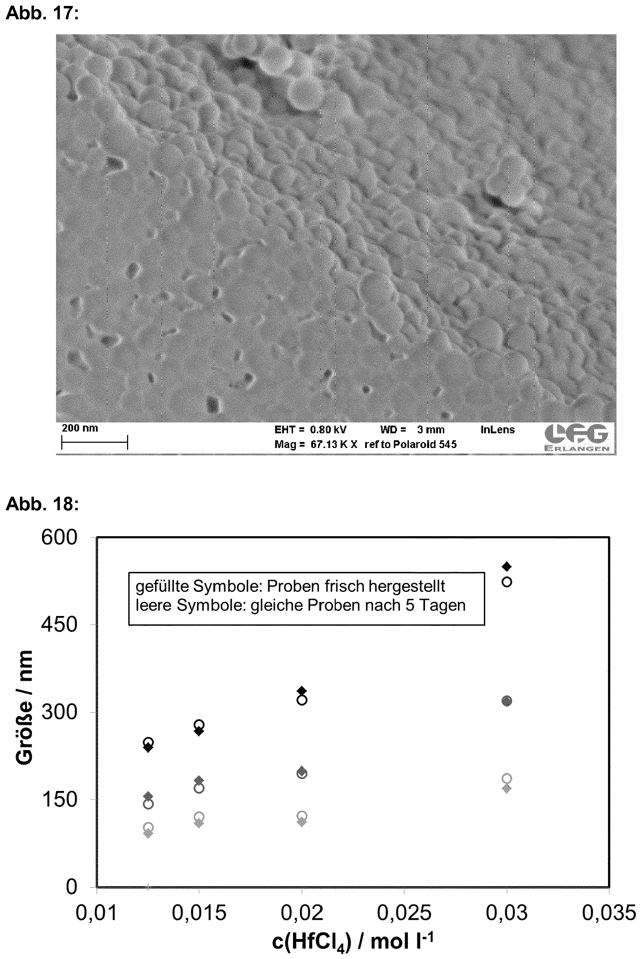

Abb. 16 und 17 zeigen SEM-Aufnahmen der entsprechenden Ibuprofen-Partikel nach 2 monatiger Lagerung mit einer 26430-fachen Vergrößerung (Mag = 26.43 K X) bzw. 67130-fachen Vergrößerung (Mag =67.13 K X).

Die SEM-Aufnahmen zeigen im Wesentlichen rundliche Einzelpartikel, die auch nach 2 monatiger Lagerung nicht zerfallen und nicht gewachsen bzw. auskristallisiert waren.

### Beispiel 9: Herstellung von stabilisierten Ibuprofen-Suspensionen mit Hafniumchlorid

Als Ausgangssubstanz wurde wie in Beispiel 5 kommerziell erhältliches Ibuprofen der Firma TCI Deutschland GmbH ohne weitere Reinigung verwendet.

Für die Herstellung von stabilisierten Ibuprofen-Suspensionen durch Anti-Solvent-Ausfällung wurde zunächst eine ethanolische Ibuprofen-Stammlösung mit einem Ibuprofen-Gehalt von 40 mg/mL durch Lösen von 40 g Ibuprofen in 1 L Ethanol, das von der in Beispiel 5 angegebenen Quelle bezogen wurde, gelöst.

Weiterhin wurde Hafnium(IV) chlorid [HfCl₄, CAS Nr.: 13499-05-3] der Thermo Fisher GmbH (Karlsruhe, DE) (Molare Masse: 320,30 g/mol, Gehalt gemäß Herstellerangabe: 99,9%, Gehalt an Zr < 0,5%) und Reinstwasser aus den in Beispiel 1 angegebenen Bezugsquellen ohne weitere Reinigung verwendet.

Weiterhin wurden eine HfCl₄-Stammlösung mit eine Konzentration von 1,0 mol/L durch Lösen von 320,30 g HfCl₄ in 1 L Reinstwasser hergestellt.

Verschiedene HfCl₄-Lösungen mit den in Tabelle 11a angegebenen Konzentrationen wurden durch Verdünnen der entsprechenden Mengen der oben angegebenen HfCl₄-Stammlösung auf ein Endvolumen von 35 mL hergestellt.

**Tabelle11a: Verwendete HfCl₄-Lösungen**

| Konzentration HfCl₄ | Herstellung durch |
|---|---|
| 0,0125 mol/L | Verdünnen von 437,5 µL HfCl₄-Stammlösung |
| 0,015 mol/L | Verdünnen von 525 µL HfCl₄-Stammlösung |
| 0,02 mol/L | Verdünnen von 700 µL HfCl₄-Stammlösung |
| 0,03 mol/L | Verdünnen von 1050 µL HfCl₄-Stammlösung |

Die Volumenanteile der ethanolischen Ibuprofen-Stammlösung (je 3,5 mL) und der verschiedenen HfCl₄-Lösungen (je 35 mL) wurden im Verhältnis von 1 : 10 gemischt. Das Mischen erfolgte durch Verwendung eines statischen Mischers, wie in Beispiel 4 beschrieben. Der verwendete Gesamtvolumenstrom war 3,3 ml s⁻¹.

Die Messung der Partikelgröße sowie deren Verteilung der in den hergestellten Ibuprofen-Suspensionen enthaltenen HfCl₄-stabilisierten Ibuprofen-Partikel erfolgte am Herstellungstag sowie 5 Tage nach der Herstellung mittels eines Malvern Zetasizers Nano ZS bei einer Temperatur von 25°C wie oben in Beispiel 3 und 4 beschrieben nach einer Standzeit von 120 s.

Die hergestellten Ibuprofen-Suspensionen wurden für einen Zeitraum von 5 Tage in einem verschlossenen Gefäß bei Raumtemperatur gelagert. Die Ergebnisse sind in Tabelle 11b sowie Abb. 18 dargestellt.

**Tabelle 11b: Ergebnis der Partikelgrößenbestimmung**

| Herstellungstag | | | |
|---|---|---|---|
| c(HfCl₄) [mol l⁻¹] | x_{10,3} [nm] | x_{50,3} [nm] | x_{90,3} [nm] |
| 0,0125 | 91 | 142 | 239 |
| 0,015 | 109 | 169 | 267 |
| 0,02 | 111 | 195 | 336 |
| 0,03 | 169 | 319 | 549 |

| Nach 5 Tagen | | | |
|---|---|---|---|
| c(HfCl₄) [mol l⁻¹] | x_{10,3} [nm] | x_{50,3} [nm] | x_{90,3} [nm] |
| 0,0125 | 101 | 155 | 248 |
| 0,015 | 120 | 183 | 278 |
| 0,02 | 121 | 199 | 320 |
| 0,03 | 186 | 318 | 523 |

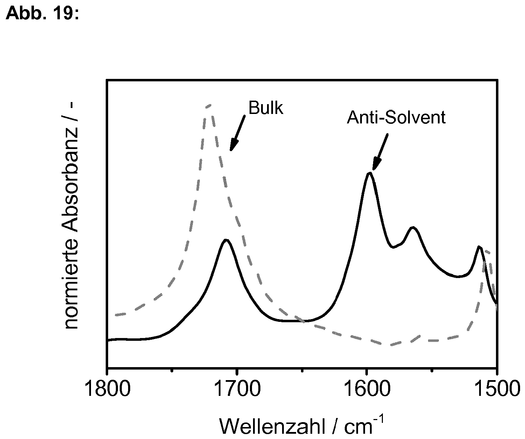

Darüber hinaus wurden mit dem verwendeten Ibuprofen (Abb. 19 "Bulk") sowie mit hergestellten HfCl₄-stabilisierten Ibuprofen-Partikeln Fourier-Transform-Infrarotspektren (FTIR-Spektren) vermessen. Dazu wurden HfCl₄-stabilisierte Ibuprofen-Partikel wie in Beispiel 1 beschrieben durch Zentrifugation isoliert und in einem FTIR-Spektrometer vermessen.

Dazu wurden HfCl₄-stabilisierte Ibuprofen-Partikel, die, wie oben beschrieben, durch Anti-Solvent-Ausfällung (Abb. 19 "Anti-Solvent") aus einer HfCl₄-Lösung mit einer HfCl₄-Konzentration von 0,0125 mol/L hergestellt wurden, durch Zentrifugation isoliert und in einem Trockenschrank bei 50°C bis zur Gewichtskonstanz getrocknet.

Die jeweils gemessenen IR-Spektren nach der Fourier-Transformation des Interferogramms sind in Abb. 19 dargestellt. Eine Zuordnung der Banden zeigt Tabelle7d in Beispiel 5.

Wie in Abb. 19 zu sehen ist, liegt Ibuprofen in den HfCl₄-stabilisierten Partikeln ebenfalls im Wesentlichen als Carboxylat vor.

### Beispiel 10: Herstellung einer trockenen Ibuprofen-Zusammensetzung.

Zur Herstellung einer trockenen Zusammensetzung wurde als Hilfsstoff Lactose monohydrat [4-O-(β-D-Galactopyranosyl)-D-glucopyranose, CAS-Nr.: 63-42-3] der Firma Sigma-Aldrich Chemie GmbH (Molare Masse: 206,28 g/mol, Gehalt gemäß Herstellerangabe: ≥ 98%) ohne weitere Reinigung verwendet.

Die in Beispiel 7 aus einer ethanolischen Ibuprofen-Lösung (Ibuprofen-Konzentration: 40 mg/ml) durch Anti-Solvent-Fällung mit einer wässrigen ZrCl₄-Lösung (ZrCl₄-Konzentration. 0,0125 mol/L) hergestellte Ibuprofen-Suspension wurde mit 200 Gew.-% Lactose, bezogen auf den Gehalt an Ibuprofen, gemischt.

Die Mischung wurde gefriergetrocknet, um vorhandene flüssige Bestandteile zu entfernen. Die hergestellte trockene Zusammensetzung wurde nachfolgend mittels Rasterelektronenmikroskopie untersucht.

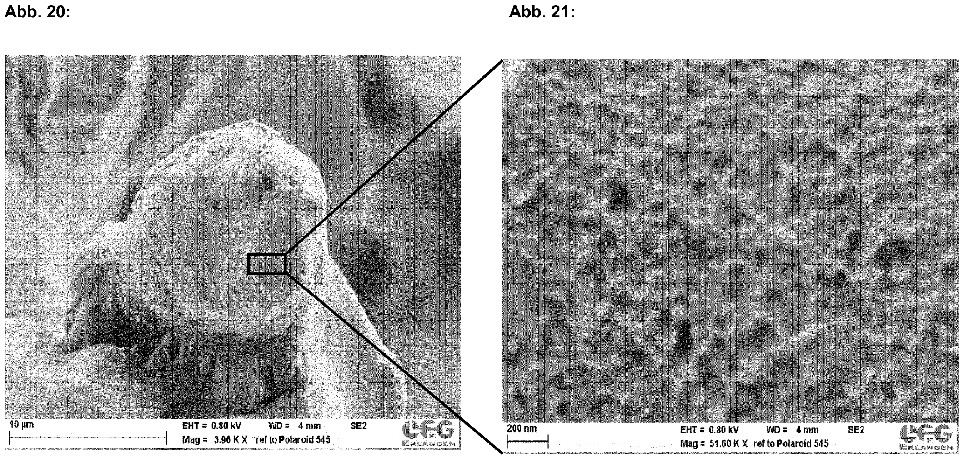

Abb. 20 und 21 zeigen SEM-Aufnahmen der entsprechenden trockenen Zusammensetzung mit einer 3960-fachen Vergrößerung (Mag = 3.96 K X) bzw. mit einer 51600-fachen Vergrößerung (Mag = 51.60 K X), wobei Abb. 21 eine Vergrößerung des in Abb. 20 markierten Ausschnittes zeigt.

In Abb. 21 sind einzelne Ibuprofen-Partikel erkennbar, die in der Lactose-Matrix verteilt sind.

### Vergleichsbeispiel 11: Herstellung von Ibuprofen-Partikeln nach dem in Zordok. A.W. et al. [4] beschriebenen Verfahren

Die Ibuprofen-Partikel wurden nach der in Zordok. A.W. et al. beschriebenen Methode hergestellt.

Dazu wurde zunächst eine methanolische Ibuprofen-Lösung (Ibuprofen-Konzentration: 2 mmol) und 1mmol NaOH-Lösung zu gleichen Teilen mit einer methanolischen ZrOCl₂ - Lösung (ZrOCl₂-Konzentration: 1mmol) vermischt und für 15 h bei 25°C gerührt. Anschließend wurde das Reaktionsgemisch stehen gelassen, bis das enthaltene Methanol vollständig verdampft war. Der übrig gebliebene Niederschlag wurde mit Reinstwasser gewaschen und eine Probe aus dieser Suspension auf ein Siliziumsubstrat aufgebracht.

Die entsprechenden Substrate wurden nachfolgend mittels Rasterelektronenmikroskopie untersucht.

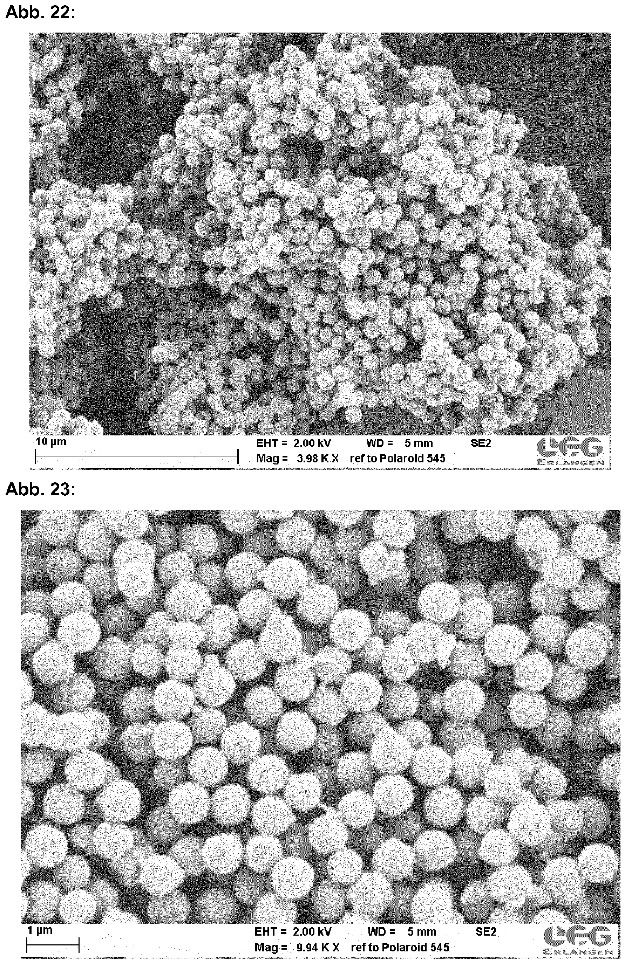

Abb. 22 und 23 zeigen SEM-Aufnahmen der entsprechenden Ibuprofen-Partikel mit einer 3980-fachen Vergrößerung (Mag = 3.98 KX) bzw. 9940-fachen Vergrößerung (Mag =9.94 K X). Die SEM-Aufnahmen zeigen im Wesentlichen sphärische Einzelpartikel.

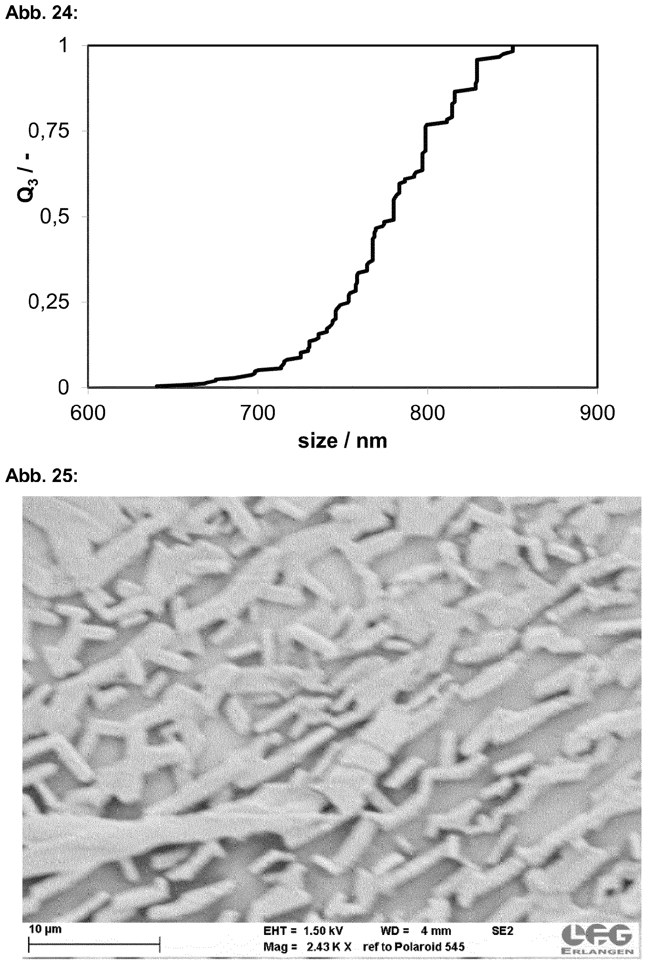

Abb. 24 zeigt die Summenverteilung (Q₃) der volumenbezogenen Äquivalentdurchmesser für die in der hergestellten Suspension enthaltenen Ibuprofen-Partikel.

Die Auswertung der Partikelgröße erfolgte per Bildauswertung der in Abb. 22 und 23 gezeigten SEM-Bilder mithilfe des Programms ImageJ2 (Schindelin, J. et al., "The ImageJ ecosystem: An open platform for biomedical image analysis", Mol. Reprod. Dev. 82(7-8), 2015, Seiten 518 bis 529, DOI: 10.1002/mrd.22489).

Die Auswertung der Partikelgröße zeigte, dass durch das in Zordok. A.W. et al. [4] beschriebenen Verfahren deutlich größere Partikel als durch das erfindungsgemäße Verfahren erhalten wurden.

### Vergleichsbeispiel 12: Präzipitation von Ibuprofen durch Anti-Solvent-Fällung mit Wasser in Abwesenheit von ZrCl₄

Als Ausgangssubstanz wurde wie in Beispiel 5 kommerziell erhältliches Ibuprofen der Firma Sigma-Aldrich Chemie GmbH ohne weitere Reinigung verwendet.

Für die Herstellung des Präzipitates durch Anti-Solvent-Ausfällung wurde eine ethanolische Ibuprofen-Stammlösung mit einem Ibuprofen-Gehalten von 30 mg/mL durch Lösen der entsprechenden Menge Ibuprofen in Ethanol, das von der in Beispiel 5 angegebenen Quelle bezogen wurde, zubereitet.

Die Anti-Solvent-Fällung wurde durch Zugabe von Reinstwasser durchgeführt, wobei die Volumenanteile der ethanolischen Ibuprofen-Stammlösung (1 mL) und des Wassers (10 mL) im Verhältnis von 1 : 10 gemischt wurden. Das Mischen erfolgte durch Verwendung eines statischen Mischers, wie in Beispiel 3 beschrieben.

Das hergestellte Präzipitat wurde nach einer Standzeit von 5 Minuten mittels Rotationsbeschichtung auf Silicium-Substrate aufgebracht und getrocknet. Die entsprechenden Substrate wurden nachfolgend mittels Rasterelektronenmikroskopie untersucht.

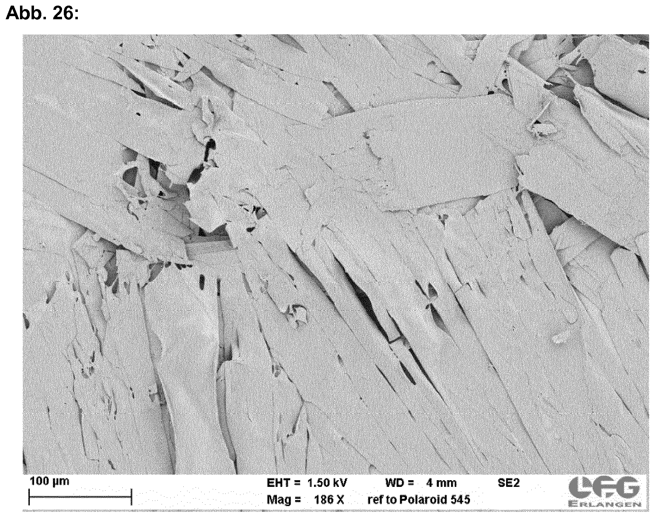

Abb. 25 und 26 zeigen SEM-Aufnahmen der entsprechenden Ibuprofen-Partikel mit einer 2430-fachen Vergrößerung (Mag = 2.43 K X) bzw. 186000-fachen Vergrößerung (Mag =186 K).

Die SEM-Aufnahmen zeigen deutliche Unterschiede zu den mit dem erfindungsgemäßen Verfahren hergestellten Partikeln. Die Partikel waren unregelmäßig geformt.

### Beispiel 13: Stabilitätsuntersuchungen von verschiedenen Wirkstoff-Suspensionen, die mit Zirkonylchlorid hergestellt wurden.

Kommerziell erhältliche Mefenaminsäure [2-(2,3-Dimethylphenylamino)-benzoesäure, CAS-Nummer: 61-68-7] der Firma TCI Deutschland GmbH (Molare Masse: 241,29 g/mol, Gehalt gemäß Herstellerangabe: > 98%) wurde ohne weitere Reinigung verwendet. Mefenaminsäure weist die in Formel (3) dargestellte Struktur auf:

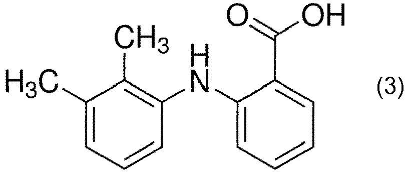

Kommerziell erhältliches Indomethazin [2-[1-(4-Chlorbenzoyl)-5-methoxy-2-methyl-1H-indol-3-yl]essigsäure CAS-Nummer: 53-86-1] der Firma TCI Deutschland GmbH (Molare Masse: 357,79 g/mol, Gehalt gemäß Herstellerangabe: > 98%) wurde ohne weitere Reinigung verwendet. Mefenaminsäure weist die in Formel (4) dargestellte Struktur auf:

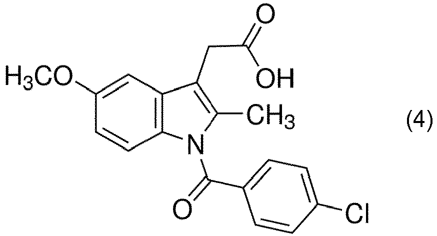

Weiterhin wurde ZrOCl₂ * 8 H₂O aus der in Beispiel 6 angegebenen Bezugsquelle sowie Naproxen und Reinstwasser aus den in Beispiel 1 angegebenen Bezugsquellen jeweils ohne weitere Reinigung verwendet.

Partikel aus Mefenaminsäure, Naproxen und Indomethazin wurden über Antisolvent-Fällung im bereits in Beispiel 4 beschriebenen T-Mischer bei einem Gesamtvolumenstrom von 6 ml s⁻¹ hergestellt. Das Antisolvent bestand in allen Versuchen aus einer wässrigen 0,025 M ZrOCl₂ - Lösung, die wie in Beispiel 6 beschrieben aus einer Stammlösung hergestellt wurde.

Als Solvent wurde Ethanol verwendet. Die Konzentrationen der jeweiligen Wirkstoffe im Solvent waren: Naproxen: 5 mg ml⁻¹; Mefenaminsäure: 1 mg ml⁻¹; Indomethacin: 5 mg ml⁻¹.

Die Messung der Partikelgröße erfolgte an 1000 µl Aliquots nach 1:40 Verdünnung mit 50 Vol.-% Ethanol in einem Zetasizer Nano ZS bei einer Temperatur von 25°C wie oben in Beispiel 3 und 4 beschrieben nach einer Standzeit von 120 s.

Anschließend wurden die Suspensionen jeweils in verschlossenen Gefäßen bei 25°C bis zur nächsten Probenentnahme gelagert. Die Probenentnahme erfolgte nach den in Tabelle 12 angegeben Zeitpunkten.

**Tabelle 12: Ergebnis der volumenbezogenen mittleren Partikelgröße x50,3**

| Indomethacin | | Naproxen | | Mefenaminsäure | |
|---|---|---|---|---|---|
| Zeit / d | x50,3 | Zeit / d | x50,3 | Zeit / d | x50,3 |
| 0,1 | 52,0 | 0,2 | 81,8 | 0,2 | 37,2 |
| 0,9 | 68,5 | 1,1 | 87,0 | 1,3 | 40,6 |
| 1,9 | 69,3 | 2,1 | 108,5 | | |
| 21,0 | 71,5 | 21,0 | 110,1 | 21,0 | 41,9 |

Wie aus Tabelle 12 ersichtlich ist kommt es aufgrund des hohen Lösungsmittelgehalts zu einer Reifung, d.h. zu einer Zunahme der volumenbezogenen mittleren Partikelgröße.

Dennoch konnten in allen Fällen nach einer Standzeit in der jeweiligen Suspension bei Standardbedingungen (Druck: 1013 mbar, Temperatur: 25°C) von wenigstens 48 h, d.h. 2 Tagen (d), nach der Herstellung Wirkstoff-Partikel erhalten werden, die eine stabile volumenbezogene mittlere Partikelgröße von unter 350 nm aufwiesen, sogar nach einer Messung der volumenbezogene mittlere Partikelgröße nach 21 Tagen (d).

## Patentansprüche

1. Suspension,
**dadurch gekennzeichnet,**
**dass** die Suspension Wasser, nanoskalige, organische Partikel mit einer volumenbezogenen mittleren Partikelgröße von weniger als 350 nm und wenigstens ein wasserlösliches Salz eines Übergangsmetalls umfasst,
wobei an der Oberfläche der organischen Partikel wenigstens teilweise Übergangsmetall-Kationen und/oder Hydroxid-Komplexe davon angeordnet sind, und
wobei die nanoskaligen, organischen Partikel wenigstens eine organische Verbindung mit wenigstens 6 C-Atomen und wenigstens einem Säuregruppen-haltigen Rest umfassen.

2. Suspension nach Anspruch 1,
**dadurch gekennzeichnet,**
**dass** das Übergangsmetall aus der Gruppe, die aus Titan, Zirkonium, Hafnium und Kombinationen davon, weiter bevorzugt Zirkonium, Hafnium und Kombinationen davon, besteht, ausgewählt wird.

3. Suspension nach einem der Ansprüche 1 oder 2,
**dadurch gekennzeichnet,**
**dass** das wenigstens ein wasserlösliches Salz eines Übergangsmetalls Anionen umfasst, die aus der Gruppe, die aus Halogeniden, Nitraten, Sulfaten, Hydrogensulfaten, Carbonaten, Hydrogencarbonaten, und Mischungen davon, vorzugsweise Nitraten, Halogeniden, und Mischungen davon, besteht, ausgewählt werden.

4. Suspension nach einem der Ansprüche 1 bis 3,
**dadurch gekennzeichnet,**
**dass** das wenigstens eine wasserlösliche Salz eines Übergangsmetalls Übergangsmetall-Kationen umfasst, die eine formale Ladung aus einem Bereich von +1 bis +6, vorzugsweise von +3 bis +5, weiter bevorzugt von +4, aufweisen.

5. Suspension nach einem der Ansprüche 1 bis 4,
**dadurch gekennzeichnet,**
**dass** der wenigstens eine Säuregruppen-haltige Rest aus der Gruppe, die aus Carboxygruppe, vinyloge Carboxygruppe, Enolgruppe, Sulfonsäuregruppe, Sulfatgruppe, Phosphonsäuregruppe, Phosphatgruppe, acider N-H Gruppe, acider C-H Gruppe, acider O-H Gruppe und Kombinationen davon besteht, ausgewählt wird.

6. Suspension nach einem der Ansprüche 1 bis 5,
**dadurch gekennzeichnet,**
**dass** die wenigstens eine organische Verbindung einen pKs-Wert, gemessen in Wasser bei 25°C und einem Druck von 1013 mbar, von 12,5 oder kleiner aufweist.

7. Suspension nach einem der Ansprüche 1 bis 6,
**dadurch gekennzeichnet,**
**dass** die wenigstens eine organische Verbindung ein saures antiphlogistisches antipyretisches Analgetikum ist, das vorzugsweise aus der Gruppe, die aus Anthracen-Derivaten, Salicylsäure-Derivaten, Anthranilsäure-Derivaten, Arylessigsäure-Derivaten, Arylpropionsäure-Derivaten, Enolsäure-Derivaten und Kombinationen davon, weiter bevorzugt aus Rhein, Diacerein, Salicylsäure, 3,5-Diiodsalicylsäure, 5-Hydroxysalicylsäure, Acetylsalicylsäure, 3,5-Diiodacetylsalicylsäure, Mesalazin, Balsalazid, Olsalazin, Sulfasalazin, Salsalat, Dipyrocetyl, Diflunisal, Triflusal, Mefenaminsäure, Flufenaminsäure, Tolfenaminsäure, Meclofenaminsäure, Nifluminsäure, Flutiazin, Flunixin, Clonixin, Clometazin, Clobuzarit, Cinmetacin, Diclofenac, Indometacin, Aceclofenac, Acemetacin, Lonazolac, Tolmetin, Ketorolac, Lumiracoxib, Felbinac, Fenclozinsäure, Alclofenac, Amfenac, Bromfenac, Eltenac, Etodolac, Mofezolac, Sulindac, Bendazac, Bumadizon, Fentiazac, Isoxepac, Metiazinsäure, Zomepirac, Tiopinac, Robenacoxib, Benoxaprofen, Carprofen, Indoprofen, Ibuprofen, Ketoprofen, Naproxen, Dexketoprofen, Flurbiprofen, Tarenflurbil, Dexibuprofen, Tiaprofensäure, Alminoprofen, Fenbufen, Fenoprofen, Flunoxaprofen, Loxoprofen, Pirprofen, Pranoprofen, Protizinsäure, Suprofen, Vedaprofen, Zaltoprofen, Miroprofen, Oxaprozin, Piroxicam, Meloxicam, Tenoxicam, Lornoxicam, Isoxicam und Mischungen davon, besteht, ausgewählt wird.

8. Suspension nach einem der Ansprüche 1 bis 7,
**dadurch gekennzeichnet,**
**dass** das wenigstens eine wasserlösliche Salz eines Übergangsmetalls und die wenigstens eine organische Verbindung in einem molaren Verhältnis aus einem Bereich von 100:1 bis 1:100 vorliegen.

9. Suspension nach einem der Ansprüche 1 bis 8,
**dadurch gekennzeichnet,**
**dass** der Anteil der nanoskaligen, organischen Partikel in der Suspension bis einschließlich 60 Gew.-%, vorzugsweise aus einem Bereich von 0,01 Gew.-% bis 55 Gew.-%, jeweils bezogen auf das Gesamtgewicht der Suspension, beträgt.

10. Verfahren zur Herstellung einer Suspension nach einem der Ansprüche 1 bis 9, **dadurch gekennzeichnet,**
**dass** das Verfahren folgenden Schritt umfasst:
a) In Kontakt bringen einer Lösung, die wenigstens eine organische Verbindung mit wenigstens 6 C-Atomen und wenigstens einen Säuregruppen-haltigen Rest aufweist, mit einer wässrigen Lösung, die wenigstens ein wasserlösliches Salz eines Übergangsmetalls umfasst.

11. Verfahren nach Anspruch 10,
**dadurch gekennzeichnet,**
**dass** die Lösung, die wenigstens eine organische Verbindung mit wenigstens 6 C-Atomen und wenigstens einen Säuregruppen-haltigen Rest aufweist, durch ein Verfahren bereitgestellt wird, das folgenden Schritt umfasst:
a1) Lösen der wenigstens einen organischen Verbindung in einem wässrigen Lösungsmittel mit einem pH-Wert von größer als 6,0 gemessen bei 25°C, oder
a2) Lösen der wenigstens einen organischen Verbindung in wenigstens einem organischen Lösungsmittel.

12. Verfahren nach einem der Ansprüche 10 oder 11,
**dadurch gekennzeichnet,**
**dass** das wenigstens ein wasserlösliches Salz eines Übergangsmetalls und die wenigstens eine organische Verbindung in einem molaren Verhältnis aus einem Bereich von 100:1 bis 1:100, vorzugsweise aus einem Bereich von 10:1 bis 1:10, weiter bevorzugt in einem Verhältnis von 5:1 bis 1:5, umgesetzt werden.

13. Suspension nach einem der Ansprüche 1 bis 9 zur Verwendung als Arzneimittel.

14. Verwendung eines wasserlöslichen Übergangsmetallsalzes, umfassend Übergangsmetall-Kationen mit einer formalen Oxidationszahl aus einem Bereich von +1 bis +4 zur Stabilisierung einer Suspension umfassend Wasser und nanoskalige, organische Partikel mit einer volumenbezogenen mittleren Primärpartikelgröße von weniger als 350 nm, wobei die organischen Partikel wenigstens eine organische Verbindung mit wenigstens 6 C-Atomen und wenigstens einem Säuregruppen-haltigen Rest umfassen.

15. Verwendung einer Suspension nach einem der Ansprüche 1 bis 9 als Additiv in Kosmetika, als Pflanzenschutzmittel oder bei der Beschichtung oder Imprägnierung von anorganischen oder organischen Oberflächen und/oder Gegenständen.

16. Trockene Zusammensetzung
**dadurch gekennzeichnet,**
**dass** die Zusammensetzung nanoskalige, organische Partikel mit einer volumenbezogenen mittleren Partikelgröße von weniger als 350 nm und wenigstens einen Hilfstoff umfasst, wobei an der Oberfläche der nanoskaligen, organischen Partikel wenigstens teilweise Übergangsmetall-Kationen und/oder Hydroxid-Komplexe davon angeordnet sind, und wobei die nanoskaligen, organischen Partikel wenigstens eine organische Verbindung mit wenigstens 6 C-Atomen und wenigstens 1 Säuregruppen-haltigen Rest umfassen.

17. Amorphe, nanoskalige, organische Partikel,
**dadurch gekennzeichnet,**
**dass** die nanoskaligen, organische Partikel eine volumenbezogene mittlere Partikelgröße von weniger als 350 nm aufweisen, wobei zumindest an der Oberfläche der organischen Partikel wenigstens teilweise, vorzugsweise vollständig, Übergangsmetall-Kationen und/oder, vorzugsweise positiv geladene, Hydroxid-Komplexe davon angeordnet, vorzugsweise gebunden, sind, und wobei die nanoskaligen, organischen Partikel wenigstens eine organische Verbindung mit wenigstens 6 C-Atomen und wenigstens einem Säuregruppen-haltigen Rest und Übergangsmetall-Kationen und/oder, vorzugsweise positiv geladene, Hydroxid-Komplexe davon umfassen oder daraus bestehen.

## Claims

1. Suspension,
**characterized**
**in that** the suspension comprises water, nanoscale organic particles having a volume-based mean particle size of less than 350 nm and at least one water-soluble salt of a transition metal,
wherein transition metal cations and/or hydroxide complexes thereof are disposed at least to some extent on the surface of the organic particles, and
wherein the nanoscale, organic particles comprise at least one organic compound having at least 6 carbon atoms and at least one acid group-containing radical.

2. Suspension according to Claim 1,
**characterized**
**in that** the transition metal is selected from the group consisting of titanium, zirconium, hafnium and combinations thereof, more preferably zirconium, hafnium and combinations thereof.

3. Suspension according to either of Claims 1 and 2,
**characterized**
**in that** the at least one water-soluble salt of a transition metal comprises anions selected from the group consisting of halides, nitrates, sulfates, hydrogensulfates, carbonates, hydrogencarbonates and mixtures thereof, preferably nitrates, halides and mixtures thereof.

4. Suspension according to any of Claims 1 to 3,
**characterized**
**in that** the at least one water-soluble salt of a transition metal comprises transition metal cations having a formal charge from a range from +1 to +6, preferably from +3 to +5, more preferably of +4.

5. Suspension according to any of Claims 1 to 4,
**characterized**
**in that** the at least one acid group-containing radical is selected from the group consisting of carboxy group, vinylogous carboxy group, enol group, sulfonic acid group, sulfate group, phosphonic acid group, phosphate group, acidic N-H group, acidic C-H group, acidic O-H group and combinations thereof.

6. Suspension according to any of Claims 1 to 5,
**characterized**
**in that** the at least one organic compound has a pKₐ, measured in water at 25°C and a pressure of 1013 mbar, of 12.5 or less.

7. Suspension according to any of Claims 1 to 6,
**characterized**
**in that** the at least one organic compound is an acidic antiphlogistic, antipyretic analgesic preferably selected from the group consisting of anthracene derivatives, salicylic acid derivatives, anthranilic acid derivatives, arylacetic acid derivatives, arylpropionic acid derivatives, enolic acid derivatives and combinations thereof, more preferably from rhein, diacerein, salicylic acid, 3,5-diiodosalicylic acid, 5-hydroxysalicylic acid, acetylsalicylic acid, 3,5-diiodoacetylsalicylic acid, mesalazine, balsalazide, olsalazine, sulfasalazine, salsalate, dipyrocetyl, diflunisal, triflusal, mefenamic acid, flufenamic acid, tolfenamic acid, meclofenamic acid, niflumic acid, flutiazin, flunixin, clonixin, clometacin, clobuzarit, cinmetacin, diclofenac, indometacin, aceclofenac, acemetacin, lonazolac, tolmetin, ketorolac, lumiracoxib, felbinac, fenclozic acid, aclofenac, amfenac, bromfenac, eltenac, etodolac, mofezolac, sulindac, bendazac, bumadizone, fentiazac, isoxepac, metiazinic acid, zomepirac, tiopinac, robenacoxib, benoxaprofen, carprofen, indoprofen, ibuprofen, ketoprofen, naproxen, dexketoprofen, flurbiprofen, tarenflurbil, dexibuprofen, tiaprofenic acid, alminoprofen, fenbufen, fenoprofen, flunoxaprofen, loxoprofen, pirprofen, pranoprofen, protizinic acid, suprofen, vedaprofen, zaltoprofen, miroprofen, oxaprozin, piroxicam, meloxicam, tenoxicam, lornoxicam, isoxicam and mixtures thereof.

8. Suspension according to any of Claims 1 to 7,
**characterized**
**in that** the at least one water-soluble salt of a transition metal and the at least one organic compound are present in a molar ratio from a range from 100:1 to 1:100.

9. Suspension according to any of Claims 1 to 8,
**characterized**
**in that** the proportion of the nanoscale organic particles in the suspension is up to and including 60% by weight, preferably from a range from 0.01% by weight to 55% by weight, based in each case on the total weight of the suspension.

10. Process for producing a suspension according to any of Claims 1 to 9,
**characterized**
**in that** the process comprises the following step:
a) bringing a solution that includes at least one organic compound having at least 6 carbon atoms and at least one acid group-containing radical into contact with an aqueous solution comprising at least one water-soluble salt of a transition metal.

11. Process according to Claim 10,
**characterized**
**in that** the solution that includes at least one organic compound having at least 6 carbon atoms and at least one acid group-containing radical is provided by a process comprising the following step:
a1) dissolving the at least one organic compound in an aqueous solvent having a pH of greater than 6.0 measured at 25°C, or
a2) dissolving the at least one organic compound in at least one organic solvent.

12. Process according to either of Claims 10 and 11,
**characterized**
**in that** the at least one water-soluble salt of a transition metal and the at least one organic compound are reacted in a molar ratio from a range from 100:1 to 1:100, preferably from a range from 10:1 to 1:10, more preferably in a ratio from 5:1 to 1:5.

13. Suspension according to any of Claims 1 to 9 for use as medicament.

14. Use of a water-soluble transition metal salt comprising transition metal cations having a formal oxidation number from a range from +1 to +4 for stabilizing a suspension comprising water and nanoscale organic particles having a volume-based mean primary particle size of less than 350 nm, wherein the organic particles comprise at least one organic compound having at least 6 carbon atoms and at least one acid group-containing radical.

15. Use of a suspension according to any of Claims 1 to 9 as additive in cosmetics, as crop protection composition or in the coating or impregnation of inorganic or organic surfaces and/or articles.

16. Dry composition,
**characterized**
**in that** the composition comprises nanoscale organic particles having a volume-based mean particle size of less than 350 nm and at least one auxiliary, wherein transition metal cations and/or hydroxide complexes thereof are disposed at least to some extent on the surface of the nanoscale organic particles, and wherein the nanoscale, organic particles comprise at least one organic compound having at least 6 carbon atoms and at least one acid group-containing radical.

17. Amorphous, nanoscale organic particles,
**characterized**
**in that** the nanoscale organic particles have a volume-based mean particle size of less than 350 nm, wherein transition metal cations and/or, preferably positively charged, hydroxide complexes thereof are disposed, preferably bonded, at least to some extent, preferably completely, at least on the surface of the organic particles, and wherein the nanoscale organic particles comprise or consist of at least one organic compound having at least 6 carbon atoms and at least one acid group-containing radical and transition metal cations and/or, preferably positively charged, hydroxide complexes thereof.

## Revendications

1. Suspension
**caractérisée en ce que**
la suspension contient de l'eau, des particules organiques nanométriques, avec une taille de particules moyenne, par rapport au volume, inférieure à 350 nm et au moins un sel soluble dans l'eau d'un métal de transition,
dans laquelle, sur la surface des particules organiques, sont disposés au moins partiellement des cations du métal de transition et/ou des complexes hydroxydes de celui-ci et
dans laquelle les particules organiques nanométriques comprennent au moins un composé organique avec au moins 6 atomes C et au moins un résidu contenant des groupes acides.

2. Suspension selon la revendication 1,
**caractérisée en ce que**
le métal de transition est sélectionné dans le groupe constitué de titane, de zirconium, de hafnium et de combinaisons de ceux-ci, de préférence de zirconium, de hafnium et de combinaisons de ceux-ci.

3. Suspension selon l'une des revendications 1 ou 2,
**caractérisée en ce que**
l'au moins un sel soluble dans l'eau d'un métal de transition contient des anions, sélectionnés dans le groupe constitué d'halogénures, de nitrates, de sulfates, d'hydrogénosulfates, de carbonates, d'hydrogénocarbonates et de mélanges de ceux-ci, de préférence de nitrates, d'halogénures et de mélanges de ceux-ci.

4. Suspension selon l'une des revendications 1 à 3,
**caractérisée en ce que**
l'au moins un sel soluble dans l'eau d'un métal de transition contient des cations de métal de transition, qui présentent une charge formelle de l'ordre de +1 à +6, de préférence de +3 à +5, de préférence de +4.

5. Suspension selon l'une des revendications 1 à 4,
**caractérisée en ce que**
l'au moins un résidu contenant des groupes acides est sélectionné dans le groupe constitué d'un groupe carboxyle, d'un groupe carboxyle vinylogue, d'un groupe énol, d'un groupe acide sulfonique, d'un groupe sulfate, d'un groupe acide phosphonique, d'un groupe N-H acide, d'un groupe C-H acide, d'un groupe O-H acide et de combinaisons de ceux-ci.

6. Suspension selon l'une des revendications 1 à 5,
**caractérisée en ce que**
l'au moins un composé organique présente une valeur pKₛ, mesurée dans l'eau à 25 °C et sous une pression de 1 013 mbar, de 12,5 ou moins.

7. Suspension selon l'une des revendications 1 à 6,
**caractérisée en ce que**
l'au moins un composé organique est un analgésique antipyrétique antiphlogistique acide qui est sélectionné de préférence dans le groupe constitué de dérivés d'anthracène, de dérivés de l'acide salicylique, de dérivés de l'acide arylacétique, de dérivés de l'acide arylpropionique, de dérivés de l'acide énolique et des combinaisons de ceux-ci, de préférence de la rhéine, de la diacéréine, de l'acide salicylique, de l'acide 3,5-diiodo-salicylique, de l'acide 5-hydroxysalicylique, de l'acide acétylsalicylique, de l'acide 3,5-diiodo-acétylsalicylique, de la mésalazine, de la balsalazine, de l'olsalazine, de la sulfalazine, du salsalate, du dipyrocétayle, du diflusinal, du triflusal, de l'acide nifluminique, de la flutizine, de la clonixine, de la clométazine, du clobuzarite, de la cinmétacine, du diclofenac, de l'indometacine, de l'aceclofenac, de l'acemetacine, du lonazolac, de la tolmétine, du ketorolac, du lumiracoxib, du felbinac, de l'acide fenclonizinique, de l'aclclofenac, de l'amfenac, du bromfénac, de l'eltenac, de l'etodolac, du mofezolac, du sulindac, du benzadac, du bumadizon, du fentiazac, de l'isoxepac, de l'acide métiazinique, du zomepirac, du tiopinac, du robenacoxib, du benoxaprofen, du carprofen, de l'indoprofen, de l'ibuprofen, du ketoprofen, du naproxen, du dexketoprofen, du flurbiprofen, du tarenflurbil, du dexibuprofen, de l'acide tiaprofénique, de l'alminoprofen, du fenbufen, du fenoprofen, du flunoxaprofen, du loxoprofen, du pirprofen, du pranoprofen, de l'acide protizinique, du suprofen, du vedaprofen, du zaltprofen, du microprofen, de l'oxaprozine, du piroxicam, du meloxicam, du tenoxicam, du lornoxicam, de l'isoxicam et de mélanges de ceux-ci.

8. Suspension selon l'une des revendications 1 à 7,
**caractérisée en ce que**
l'au moins un sel soluble dans l'eau d'un métal de transition et l'au moins un composé organique se présentent avec un rapport molaire de l'ordre de 100:1 à 1:100.

9. Suspension selon l'une des revendications 1 à 8,
**caractérisée en ce que**
la part des particules organiques nanométriques dans la suspension représente jusqu'à 60 % en poids inclus, de préférence de l'ordre de 0,01 % en poids à 55 % en poids, respectivement par rapport au poids total de la suspension.

10. Procédé de réalisation d'une suspension selon l'une des revendications 1 à 9,
**caractérisé en ce que**
le procédé comprend l'étape suivante :
a) mise en contact d'une solution, qui contient au moins un composé organique avec au moins 6 atomes C et au moins un résidu contenant des groupes acides, avec une solution aqueuse qui contient au moins un sel soluble dans l'eau d'un métal de transition.

11. Procédé selon la revendication 10,
**caractérisé en ce que**
la solution, qui contient au moins un composé organique avec au moins 6 atomes C et au moins un résidu contenant des groupes acides, est mise à disposition à l'aide d'un procédé qui comprend l'étape suivante :
a1) la dissolution de l'au moins un composé organique dans un solvant aqueux avec une valeur de pH supérieure à 6,0, mesurée à 25 °C ou
a2) la dissolution de l'au moins un composé organique dans au moins un solvant organique.

12. Procédé selon l'une des revendications 10 ou 11,
**caractérisé en ce que**
l'au moins un sel soluble dans l'eau d'un métal de transition et l'au moins un composé organique sont mis en oeuvre dans un rapport molaire de l'ordre de 100:1 à 1:100, de préférence de l'ordre de 10:1 à 1:10, de préférence dans un rapport de 5:1 à 1:5.

13. Suspension selon l'une des revendications 1 à 9, destinée à être utilisée en tant que médicament.

14. Utilisation d'un sel soluble dans l'eau d'un métal de transition, comprenant des cations de métal de transition avec un nombre d'oxydation formel de l'ordre de +1 à +4 pour la stabilisation d'une suspension contenant de l'eau et des particules organiques nanométriques avec une taille de particules moyenne, rapportée au volume, inférieure à 350 nm, dans laquelle les particules organiques comprennent au moins un composé organique avec au moins 6 atomes C et au moins un résidu contenant des groupes acides.

15. Utilisation d'une suspension selon l'une des revendications 1 à 6 en tant qu'additif dans des cosmétiques, en tant que produit phytosanitaire ou pour le revêtement ou l'imprégnation de surfaces et/ou d'objets inorganiques ou organiques.

16. Composition sèche,
**caractérisée en ce que**
la composition contient des particules organiques nanométriques avec une taille de particules moyenne, rapportée au volume, inférieure à 350 nm et au moins une substance auxiliaire, dans laquelle, sur la surface des particules organiques nanométriques, sont disposés au moins partiellement des cations du métal de transition et/ou des complexes hydroxydes de ceux-ci, et dans laquelle les particules organiques nanométriques comprennent au moins un composé organique avec au moins 6 atomes C et au moins 1 résidu contenant des groupes acides.

17. Particules organiques nanométriques amorphes,
**caractérisées en ce que**
les particules organiques nanométriques présentent une taille de particules moyenne, rapportée au volume, inférieure à 350 nm, dans laquelle, au moins sur la surface des particules organiques, sont disposés, de préférence liés, au moins partiellement, de préférence entièrement, des cations d'un métal de transition et/ou des complexes hydroxydes, de préférence chargés positivement, de ceux-ci et dans laquelle les particules organiques nanométriques comprennent ou sont constitués d'au moins un composé organique avec au moins 6 atomes C et au moins un résidu contenant des groupes acides et des cations d'un métal de transition et/ou des complexes hydroxydes, de préférence chargés positivement, de ceux-ci.
